# EUROPEAN PATENT APPLICATION

(11) **EP 4 624 572 A1**
(43) Date of publication of application: **01.10.2025**
(21) Application number: 24166835.9
(22) Date of filing: 27.03.2024
(51) Int. Cl.: C12N 9/54, C11D 3/386

(54) **POLYPEPTIDES HAVING PROTEASE ACTIVITY FOR USE IN DETERGENT COMPOSITIONS**

(71) Applicant: BASF SE, 67056 Ludwigshafen am Rhein (DE)
(72) Inventor: POWERS, Lisa, San Diego, CA 92121 (US); LOGUE, Amanda Rae, San Diego, CA 92121 (US); HOANG, Cindy, San Diego, CA 92121 (US); POP, Christina, San Diego, CA 92121 (US); LZON, Jonathan D, San Diego, CA 92121 (US); KLINE, Katie, San Diego, CA 92121 (US); RISOER, Michael Wulff, San Diego, CA 92121 (US); LOWE, Gina Ning, San Diego, CA 92121 (US); LISZKA, Michael, San Diego, CA 92121 (US); JENEWEIN, Stefan, 67056 Ludwigshafen am Rhein (DE); DYER, Rebekah, San Diego, CA 92130 (US)
(74) Representative: Maiwald GmbH

(57) **Abstract**

In the present invention new protease enzymes are provided. More specifically, genetically engineered protease enzymes, compositions comprising the enzymes, and methods of making and using the enzymes or compositions comprising the enzymes are provided.

## Description

### Field of the invention

In the present invention new protease enzymes are provided. More specifically, genetically engineered protease enzymes, compositions comprising the enzymes, and methods of making and using the enzymes or compositions comprising the enzymes are provided.

### Background of the invention

Enzymes are increasingly used in various applications as sustainable alternatives to petrochemistry. Enzymes are biodegradable and can be catalytically active already at lower temperatures, which results in reduction of energy consumption. In particular, in the detergent industry enzymes are implemented in washing formulations to improve cleaning efficiency and/or reduce energy consumption in a washing step.

Proteases are enzymes capable of hydrolyzing proteins. Thus, proteases have been employed in the removal of protein stains and have been added to detergent compositions for this purpose. In detergent applications the proteases shall be stable at elevated temperatures and/or within the denaturing conditions of the detergents and the wash liquor.

WO 2016/096711 and WO 2016/096714 describe a subtilase variant having improved stability and/or improved wash performance in liquid detergents compared to the parent subtilase and detergents containing the variant. WO 2016/001450 and WO 2020/002255 discloses subtilase variants with increased stability. WO 2010/056640 also describes subtilisin variants. US 6,376,450 discloses multiple-substituted protease variants which provide improved and enhanced cleaning ability. US 2020/172890 A1 discloses performance-enhanced and storage-stable protease variants. WO 99/20770 A2, WO 03/062381 A2, WO 2011/140364 A1, WO 2011/072117 A1, WO 2011/072099 A2, WO 2012/151534 A1, WO 2015/144932 A1, WO 2017/207762 A1, WO 2017/192692A1, WO 2018/069158 A1 and WO 2022/225696 A2 also disclose protease variants.

Nevertheless, there is still a need for new protease enzymes, which are stable in harsh detergent compositions and show a good washing performance.

### Brief summary of the invention

The present invention is directed to a polypeptide having protease activity or a fragment of said polypeptide having protease activity, wherein the polypeptide or fragment thereof has an amino acid sequence which is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57.

The present invention further relates to a polynucleotide encoding said variant polypeptide and a composition comprising said variant polypeptide.

### Detailed description of the invention

The present invention may be understood more readily by reference to the following detailed description of the embodiments of the invention and the examples included herein.

Although the present invention will be described with respect to particular embodiments, this description is not to be construed in a limiting sense.

### Definitions

Unless otherwise noted, the terms used herein are to be understood according to conventional usage by those of ordinary skill in the relevant art.

Before describing in detail exemplary embodiments of the present invention, definitions important for understanding the present invention are given. Unless stated otherwise or apparent from the nature of the definition, the definitions apply to all compounds, methods and uses described herein.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

Throughout this application, various publications are referenced. The disclosure of all of these publications and those references cited within those publications in their entireties are hereby incorporated by reference into this application in order to more fully describe the state of the art to which this invention pertains.

It is to be understood that the term "comprising" is not limiting. For the purposes of the present invention the term "consisting of" is considered to be a preferred embodiment of the term "comprising". If hereinafter a group is defined to comprise at least a certain number of members, this is meant to also encompass a group which consists of these members only.

"Amino acid substitutions" are described by providing the original amino acid followed by the number of the position within the amino acid sequence, followed by the substituted amino acid. For example, the substitution of histidine at position 120 with alanine is designated as "His120Ala" or "H120A". Substitutions can also be described by merely naming the resulting amino acid in the variant without specifying the amino acid of the parent at this position, e.g., by using "X120A" or "120A" or "Xaa120Ala" or "120Ala".

Variants comprising multiple substitutions are separated by "+", e.g., "Arg170Tyr+Gly195Glu", "R170Y+G195E" or "X170Y+X195E" representing a substitution of arginine and glycine at positions 170 and 195 with tyrosine and glutamic acid, respectively. Alternatively, multiple substitutions may be separated by space or a comma, e.g., "R170Y G195E" or "R170Y, G195E" respectively. Where different alternative substitutions can be introduced at a position, the different substitutions are separated by a comma, e.g., "Arg170Tyr,Glu" and "R170T,E", respectively, represents a substitution of arginine at position 170 with tyrosine or glutamic acid. Alternative substitutions at a particular position can also be indicated as "X120A,G,H", "120A,G,H", "X120A/G/H", or "120A/G/H". Alternatively, different substitutions may be indicated in brackets, e.g., "Arg170[Tyr,Gly]" or "Arg170{Tyr,Gly}" or in short "R170 [Y,G]" or "R170 {Y,G}".

The numbering of the amino acid residues of the proteases described herein is as commonly used for proteases in the field (cf. P.N. Bryan, Biochimica et Biophysica Acta 1543 (2000), 203-222, cf. p. 204, left col., 3^{rd} para.) according to the numbering of the BPN' subtilisin protease from *Bacillus amyloliquefaciens* the sequence of which is shown in SEQ ID NO: 42 (i.e., according to the numbering of SEQ ID NO: 42 or according to "BPN' numbering").

In an alternative way, one can describe the amino acid positions with reference to the numbering of SEQ ID NO: 41 or SEQ ID NO: 43, i.e., according to the numbering of SEQ ID NO: 41 or according to the numbering of SEQ ID NO: 43. Table 1 below shows the amino acid numbering according to SEQ ID NO: 42 and the numbering of the corresponding amino acids in the sequences according to SEQ ID NO: 41 or 43:

**Table 1**

| **According to SEQ ID NO: 41/43** | **According to SEQ ID NO: 42** | **According to SEQ ID NO: 41/43** | **According to SEQ ID NO: 42** | **According to SEQ ID NO: 41/43** | **According to SEQ ID NO: 42** |
|---|---|---|---|---|---|
| 18 | 18 | 107 | 109 | 204 | 210 |
| 21 | 21 | 142 | 144 | 212 | 218 |
| 24 | 24 | 158 | 160 | 222 | 228 |
| 37 | 38 | 176 | 182 | 242 | 248 |
| 55 | 56 | 177 | 183 | | |
| 76 | 78 | 198 | 204 | | |

The term "native" (or naturally or wild-type or endogenous) cell or organism or polynucleotide or polypeptide refers to the cell or organism or polynucleotide or polypeptide as found in nature (i.e., without there being any human intervention).

The term "heterologous" (or exogenous or foreign or recombinant or non-native or non-natural) polypeptide is defined herein as a polypeptide that is not native to the host cell, a polypeptide native to the host cell in which structural modifications, e.g., deletions, substitutions, and/or insertions, have been made by recombinant DNA techniques to alter the native polypeptide, or a polypeptide native to the host cell whose expression is quantitatively altered or whose expression is directed from a genomic location different from the native host cell as a result of manipulation of the DNA of the host cell by recombinant DNA techniques, e.g., a stronger promoter. Similarly, the term "heterologous" (or exogenous or foreign or recombinant or non-native or non-natural) polynucleotide refers to a polynucleotide that is not native to the host cell, a polynucleotide native to the host cell in which structural modifications, e.g., deletions, substitutions, and/or insertions, have been made by recombinant DNA techniques to alter the native polynucleotide, or a polynucleotide native to the host cell whose expression is quantitatively altered as a result of manipulation of the regulatory elements of the polynucleotide by recombinant DNA techniques, e.g., a stronger promoter, or a polynucleotide native to the host cell, but integrated not within its natural genetic environment as a result of genetic manipulation by recombinant DNA techniques. With respect to the relation between two or more polynucleotide sequences or the relation between two or more amino acid sequences, the term "heterologous" is used to characterize that the two or more polynucleotide sequences or two or more amino acid sequences are naturally not occurring in the specific combination with each other.

For the purpose of the invention, "recombinant" (or transgenic) with regard to a cell or an organism means that the cell or organism contains a heterologous polynucleotide, which is introduced by man using gene technology. With regard to a polynucleotide "recombinant" includes all constructs produced by using gene technology / recombinant DNA techniques in which either
(a) the sequence of the polynucleotide or a part thereof, or
(b) one or more genetic control sequences, which are operably linked to the polynucleotide, including but not limited to a promoter, or
(c) both a) and b)
are not located in their wild-type genetic environment or have been modified by man.

A "synthetic" compound is obtained by *in vitro* chemical and/or enzymatic synthesis.

Variant polynucleotide and variant polypeptide sequences may be defined by their sequence identity when compared to a parent sequence. Sequence identity usually is provided as "% sequence identity" or "% identity". For calculation of sequence identities, in a first step a sequence alignment is produced. According to this invention, a pairwise global alignment is produced, meaning that two sequences are aligned over their complete length, which is usually produced by using a mathematical approach, called alignment algorithm.

According to the invention, the alignment is generated by using the algorithm of Needleman and Wunsch (J. Mol. Biol. (1970) 48, p. 443-453). Preferably, the program "NEEDLE" (The European Molecular Biology Open Software Suite (EMBOSS)) is used for the purposes of the current invention, with using the programs default parameter (polynucleotides: gap open=10.0, gap extend=0.5 and matrix=EDNAFULL; polypeptides: gap open=10.0, gap extend=0.5 and matrix=EBLOSUM62). After aligning two sequences, in a second step, an identity value is determined from the alignment produced. For this purpose, the %-identity is calculated by dividing the number of identical residues by the length of the alignment region which is showing the respective sequence of the present invention over its complete length multiplied with 100: %-identity = (identical residues / length of the alignment region which is showing the respective sequence of the present invention over its complete length) *100. A special aspect concerning amino acid substitutions are conservative mutations which often appear to have a minimal effect on protein folding resulting in substantially maintained enzyme properties of the respective enzyme variant compared to the enzyme properties of the parent enzyme. Conservative mutations are those where one amino acid is exchanged with a similar amino acid. Such an exchange most probably does not change enzyme properties. Herein the following conservative exchanges are considered:
Amino acid A is similar to amino acids S
Amino acid D is similar to amino acids E; N
Amino acid E is similar to amino acids D; K; Q
Amino acid F is similar to amino acids W; Y
Amino acid H is similar to amino acids N; Y
Amino acid I is similar to amino acids L; M; V
Amino acid K is similar to amino acids E; Q; R
Amino acid L is similar to amino acids I; M; V
Amino acid M is similar to amino acids I; L; V
Amino acid N is similar to amino acids D; H; S
Amino acid Q is similar to amino acids E; K; R
Amino acid R is similar to amino acids K; Q
Amino acid S is similar to amino acids A; N; T
Amino acid T is similar to amino acids S
Amino acid V is similar to amino acids I; L; M
Amino acid W is similar to amino acids F; Y
Amino acid Y is similar to amino acids F; H; W

Conservative amino acid substitutions may occur over the full length of the sequence of a polypeptide sequence of a functional protein such as an enzyme. Preferably, such mutations are not pertaining the functional domains of an enzyme, more preferably conservative mutations are not pertaining the catalytic centers of an enzyme.

A "fragment" or "subsequence" as used herein refers to a portion of an amino acid sequence. Polypeptides comprising a deletion of one or more amino acids at the N terminus and/or the C terminus of the polypeptide and essentially retain protease activity are herein designated as "functional fragments".Preferably, the functional fragment has at least 50%, at least 55%, at least 60%, at least 65%, at least 70%, at least 75%, at least 76%, at least 77%, at least 78%, at least 79%, at least 80% identical, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 98.5 %, at least 99%, or at least 99.5% of the length of the original full length amino acid sequence. Preferably, the functional fragment comprises 100 to 259 consecutive amino acids of the full-length variant polypeptide. Also preferably, the functional fragment retains at least 75%, at least 76%, at least 77%, at least 78%, at least 79%, at least 80% identical, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 98.5 %, at least 99%, at least 99.5% or at least 100% of the enzyme activity of the original full length amino acid sequence. The functional fragment comprises consecutive amino acids compared to the original full length amino acid sequence, respectively. In the context of the present invention, the "original full length amino acid sequence" is an amino acid sequence which is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and which has the same length as the amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39,SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57.

In one embodiment, the polypeptide of the present invention does not comprise any internal deletions within the amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57. However, as discussed above, the polypeptide of the present invention may comprise a deletion of one or more amino acids at the N terminus and/or the C terminus of the polypeptide.

"Genetic construct" or "expression cassette" as used herein, is a nucleic acid molecule composed of at least one sequence of interest to be expressed, operably linked to one or more control sequences (at least to a promoter) as described herein.

The term "vector" as used herein comprises any kind of construct suitable to carry foreign polynucleotide sequences for transfer to another cell, or for stable or transient expression within a given cell. The term "vector" as used herein encompasses any kind of cloning vehicles, such as but not limited to plasmids, phagemids, viral vectors (e.g., phages), bacteriophage, baculoviruses, cosmids, fosmids, artificial chromosomes, and any other vectors specific for specific hosts of interest. Foreign polynucleotide sequences usually comprise a coding sequence which may be referred to herein as "gene of interest". The gene of interest may comprise introns and exons, depending on the kind of origin or destination of host cell.

The term "introduction of a polynucleotide" or "transformation of a polynucleotide" as referred to herein encompasses the transfer of an exogenous polynucleotide into a host cell, irrespective of the method used for transfer. That is, the term "transformation of a polynucleotide" as used herein is independent from vector, shuttle system, or host cell, and it not only relates to the polynucleotide transfer method of transformation as known in the art (cf., for example, Sam-brook, J. et al. (1989) Molecular Cloning: A Laboratory Manual, 2nd Ed., Cold Spring Harbor Laboratory Press, Cold Spring Harbor, NY), but it encompasses any further kind of polynucleotide transfer methods such as, but not limited to, transduction or transfection.

A polynucleotide encoding a polypeptide may be "expressed". The term "expression" or "gene expression" means the transcription of a specific gene or specific genes or specific nucleic acid construct. The term "expression" or "gene expression" means the transcription of a gene or genes or genetic construct into structural RNA (e.g., rRNA, tRNA) or mRNA with or without subsequent translation of the latter into a protein. The process includes transcription of DNA and processing of the resulting mRNA product.

The term "purification" or "purifying" refers to a process in which at least one component, e.g., a protein of interest, is separated from at least another component, e.g., a particulate matter of a fermentation broth, and transferred into a different compartment or phase, wherein the different compartments or phases do not necessarily need to be separated by a physical barrier. Examples of such different compartments are two compartments separated by a filtration membrane or cloth, i.e., filtrate and retentate; examples of such different phases are pellet and supernatant or cake and filtrate, respectively. The resulting solution after purifying the enzyme of interest from the fermentation broth is called herein "purified enzyme solution".

"Protein formulation" (or "enzyme preparation" or "polypeptide formulation"), e.g., "protease formulation", means any non-complex formulation comprising a small number of ingredients, preferably, 2-8 components, wherein the ingredients serve the purpose of stabilizing the proteins/polypeptides comprised in the protein/polypeptide formulation and/or the stabilization of the protein/polypeptide formulation itself. Preferably, the non-complex protein/polypeptide formulation comprises the protein/polypeptide in higher concentrations than the complex formulation, e.g., than a detergent formulation. Thus, preferably the non-complex protein formulation is a concentrated protein/polypeptide formulation. Preferably, non-complex protein/polypeptide formulations comprise 20 to 120 mg/g active enzyme, whereas complex formulations, like detergent compositions, comprise 0.002 to 10 mg/g active enzyme. In contrast to a non-complex formulation, a complex formulation means herein a formulation comprising a higher number of ingredients, preferably, 9-30 components, wherein the ingredients serve the purpose of stabilizing the proteins comprised in the protein formulation and/or the stabilization of the protein formulation itself, but additionally the complex formulation comprises components that serve the purpose of the complex formulation, e.g., a detergent composition. An example for a non-complex protein formulation is a concentrated enzyme composition that is used as a stock-solution to prepare a complex formulation, e.g., a detergent composition, wherein in the detergent compositions other compounds are present that serve the cleaning purpose of the detergent composition, e.g., surfactants and/or chelating agents.

"Enzyme properties" include, but are not limited to, catalytic activity, substrate/cofactor specificity, product specificity, stability in the course of time, thermostability, pH stability, and chemical stability. "Enzymatic activity" or "catalytic activity" means the catalytic effect exerted by an enzyme, expressed as units per milligram of enzyme (specific activity) or molecules of substrate transformed per minute per molecule of enzyme (molecular activity). Enzymatic activity can be specified by the enzyme's actual function, e.g., proteases exerting proteolytic activity by catalyzing hydrolytic cleav-age of peptide bonds, lipases exerting lipolytic activity by hydrolytic cleavage of ester bonds, amylases activity involves hydrolysis of glycosidic linkages in polysaccharides, etc. According to the invention, the enzymatic activity is proteolytic activity which can be determined by using Succinyl-Ala-Ala-Pro-Phe-p-nitroanilide (Suc-AAPF-pNA; see e.g. DelMar et al. (1979), Analytical Biochem 99, 316-320) or Suc-AAPF-AMC (7-amido-4-methylcoumarin) as substrate. pNA or AMC is cleaved from the substrate molecule by proteolytic cleavage, resulting in release of yellow color of free pNA or change of fluorescence properties which can be quantified either by measuring emission at 460 nm after excitation at 380 nm. Other methods using for example casein as a proteinaceous substrate are known to those skilled in the art. The term "enzyme stability" according to the current invention relates to the retention of enzymatic activity as a function of time during storage or operation. Retention of enzymatic activity as a function of time during storage is called "storage stability" and is preferred within the context of the invention.

To determine and quantify changes in catalytic activity of enzymes stored or used under certain conditions over time, the enzymatic activity is measured under defined conditions at time zero (100%) and at a certain point in time later (x%). By comparison of the values measured, the residual activity of the enzyme can be determined in its extent. The extent of the residual activity of the enzyme determines an enzyme's stability or non-stability.

"Enzyme inhibitors" as used herein are compounds that slow down or halt enzymatic activity. Enzyme inhibitors frequently also stabilize the enzyme in its three-dimensional structure. Hence, enzyme inhibitors usually also act as "enzyme stabilizers".

"pH stability" refers to the ability of an enzyme to exert enzymatic activity after exposure to a certain pH value.

The term "detergent stability" (herein also called "residual activity in a detergent" or "storage stability in a detergent composition") refers to the ability of an enzyme to exert catalytic activity or wash performance after storage in a detergent composition, preferably, after storage at a temperature of 37 °C, 45 °C, or 50 °C for up to 14 days in a detergent composition (preferably, in Model B detergent as described herein). Most preferably, "detergent stability" is determined by measuring residual activity of the protease after storage at a temperature of 45 °C for 18 hours and/or 3 days in a detergent composition (preferably, in Model B detergent as described herein).

As used herein, "wash performance" (also called herein "cleaning performance") of an enzyme refers to the contribution of the enzyme to the cleaning performance of a detergent composition, i.e. the cleaning performance added to the detergent composition by the performance of the enzyme. The term "wash performance" is used herein similarly for laundry and hard surface cleaning. Wash performance is compared under relevant washing conditions. The term "relevant washing conditions" is used herein to indicate the conditions, particularly washing temperature, time, washing mechanics, sud concentration, type of detergent and water hardness, actually used in households in a detergent market segment. The term "improved wash performance" is used to indicate that a better end result is obtained in stain removal under relevant washing conditions, or that less enzyme, on weight basis, is needed to obtain the same end result relative to the corresponding control conditions.

As used herein, the term "specific performance" refers to the cleaning and removal of specific stains or soils per unit of active enzyme. In some embodiments, the specific performance is determined using stains or soils such as egg, egg yolk, milk, grass, minced meat blood, chocolate sauce, baby food, sebum, etc.

"Detergent composition" or "detergent" means compositions designated for cleaning soiled material. Detergent compositions are complex formulations as further defined herein. Detergent compositions according to the invention include detergent compositions for different applications such as laundry and hard surface cleaning. Detergent compositions according to the invention also include biodegradable detergent compositions. The term "detergent component" is defined herein to mean a type of chemical, which can be used in detergent compositions. A typical detergent component is a surfactant. "Surfactant" (synonymously used herein with "surface active agent") means an organic chemical that, when added to a liquid, changes the properties of that liquid at an interface. According to its ionic charge, a surfactant is called non-ionic, anionic, cationic, or amphoteric. The term "effective amount of a detergent component" includes amounts of certain components to provide effective stain removal and/or effective cleaning conditions (e.g. pH, temperature, water hardness, quantity of foaming), amounts of certain components to effectively provide optical benefits (e.g. optical brightening, dye transfer inhibition, color care), and amounts of certain components to effectively aid the processing (maintain physical characteristics during processing, storage and use; e.g. rheology modifiers, hydrotropes, desiccants). Detergent compositions typically have a protease concentration of 0.002 to 10 mg/g active enzyme.

The term "laundry" or "laundering" relates to both household laundering and industrial laundering and means the process of treating textiles and/or fabrics with a solution containing a detergent composition of the present invention. The laundering process may be carried out by using technical devices such as a household or an industrial washing machine. Alternatively, the laundering process may be done by hand.

The term "textile" means any textile material including yarns (thread made of natural or synthetic fibers used for knitting or weaving), yarn intermediates, fibers, non-woven materials, natural materials, synthetic materials, as well as fabrics made of these materials such as garments, cloths and other articles. The terms "fabric" (a textile made by weaving, knitting orfelting fibers) or "garment" (any article of clothing made of textile) as used herein, are intended to include the broader term textile as well.

The term "fibers" includes natural fibers, synthetic fibers, and mixtures thereof. Examples of natural fibers are of plant (such as flax, jute and cotton) or animal origin, comprising proteins like collagen, keratin and fibroin (e.g. silk, sheep's wool, angora, mohair, cashmere). Examples for fibers of synthetic origin are polyurethane fibers such as Spandex^{®} or Lycra^{®}, polyester fibers, polyolefins such as elastofin, or polyamide fibers such as nylon. Fibers may be single fibers or parts of textiles such as knitwear, woven or non-woven fabrics.

The term "hard surface cleaning" relates to both household hard surface cleaning and industrial hard surface cleaning and means the process of treating hard surfaces with a solution containing a detergent composition of the present invention. Hard surfaces may include any hard surfaces in the household or industry, such as floors, furnishing, walls, sanitary ceramics, glass, metallic surfaces including cutlery or dishes and medical devices such as diagnostic instruments, trays, pans, holders, racks, forceps, scissors, shears, saws (e.g. bone saws and their blades), hemostats, knives, chisels, rongeurs, files, nippers, drills, drill bits, rasps, burrs, spreaders, breakers, elevators, clamps, needle holders, carriers, clips, hooks, gouges, curettes, retractors, straightener, punches, extractors, scoops, keratomes, spatulas, expressors, trocars, dilators, cages, glassware, tubing, catheters, cannulas, plugs, stents, endoscopes, arthoscopes and related equipment. A particular form of hard surface cleaning is dishwashing, particularly automatic dishwashing (ADW).

The term "dish wash" refers to all forms of washing dishes, e.g. by hand or automatic dish wash. Washing dishes includes, but is not limited to, the cleaning of all forms of crockery such as plates, cups, glasses, bowls, all forms of cutlery such as spoons, knives, forks and serving utensils as well as ceramics, plastics such as melamine, metals, china, glass and acrylics.

Cleaning performance is evaluated under relevant cleaning conditions. The term "relevant cleaning conditions" herein refers to the conditions, particularly cleaning temperature, time, cleaning mechanics, suds concentration, type of detergent and water hardness, actually used in laundry machines, automatic dish washers or in manual cleaning processes.

The term "medical device cleaning" refers to the cleaning step in reprocessing reusable medical devices. Medical device cleaning methods can be divided into two categories, manual and mechanical/automated cleaning methods. Manual cleaning is used when mechanical units are not available or medical devices to be cleaned are too fragile or difficult to clean with a mechanical unit. Mechanical/automated cleaning methods remove soiling and microorganisms through an automated cleaning and rinsing process, this includes ultrasonic cleaning and washing.

In the field of detergency, usually the term "stains" is used with reference to laundry, e.g., cleaning of textiles, fabric, or fibers, whereas the term "soils" is usually used with reference to hard surface cleaning, e.g., cleaning of dishes and cutlery. However, herein the terms "stain" and "soil" shall be used interchangeably.

A "sequestering builder" as used herein is different from a precipitating builder in that no significant amount of precipitate is formed when the builder is used in an amount sufficient to combine with all of the calcium ions in an aqueous solution with 7 °dH hardness (German hardness) initially at neutral pH. A "strong builder" is classified as high efficiency chelators that can bind the divalent cations such as Ca2+ strongly with a logarithmic stability constant (Log K_{Ca} ) of the cation/chelator complex of above 4, particular above 5, above 6 or above 7. The stability constants are determined at an ionic strength of 0.1 M and at a temperature of 25°C. A "strong sequestering builder" combines both of the above-mentioned properties. Strong sequestering builders include, but are not limited to, Ethylenediaminetetraacetic acid (EDTA), Ethylene diamine tetra(methylene phosphonic acid (EDTMP), Nitrilo trimethylene phosphonic acid (NTMP), Diethylenetriamine Penta(Methylene Phosphonic acid) (DTPMP), methylglycinediacetic acid (MGDA), Nitrilotriacetic acid (NTA), 1-Hydroxy Ethylidene-1,1-Diphosphonic acid (HEDP), sodium tripolyphosphate (STPP), iminodisuccinic acid (IDS), N,N-diacetic acid tetra sodium salt (GLDA), pyrophosphate and ethylenediaminedisuccinic acid (EDDS), preferably MGDA and/or EDDS.

An "antimicrobial agent" is a chemical compound that kills microorganisms or inhibits their growth or reproduction. Microorganisms can be bacteria, yeasts or molds. A "preservative" is an antimicrobial agent which may be added to aqueous products and compositions to maintain the original performance, characteristics and integrity of the products and compositions by killing contaminating microorganisms or inhibiting their growth.

A composition "essentially devoid" of a compound shall mean herein that the respective compound is not added to the composition on purpose, meaning that at most non-effective amounts are present, most preferably 0% of the compound are contained in the composition.

### Detailed description

In the present invention new protease enzymes are provided. More specifically, variants of a parent protease, methods of making the variant proteases, compositions comprising the protease variants, and methods of using the variant proteases or compositions comprising the variant proteases are provided.

### Protease Variant

The present invention is directed to a polypeptide having protease activity or a functional fragment of said polypeptide having protease activity, wherein the polypeptide or fragment thereof has an amino acid sequence which is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, wherein the functional fragment comprises 100 to 259 consecutive amino acids of the full-length polypeptide.

The polypeptide having protease activity of the present invention is a non-naturally occurring protease. Preferably, the polypeptide having protease activity of the present invention is a purified, isolated, synthetic, and/or recombinant protease. Preferably, the polypeptide having protease activity of the present invention is a purified and recombinant protease.

Proteases according to the invention have "proteolytic activity" or "protease activity". "Proteolytic activity" or "protease activity" describes the capability for the hydrolysis of peptide bonds in polypeptides. Protease activity may be determined by assays for measurement of protease activity which are known to those skilled in the art. The methods for analyzing proteolytic activity are well-known in the literature (see e.g. Gupta et al. (2002), Appl. Microbiol. Biotechnol. 60: 381-395). For instance, proteolytic activity can be determined by using Succinyl-Ala-Ala-Pro-Phe-p-nitroanilide (Suc-AAPF-pNA; see e.g. DelMar et al. (1979), Analytical Biochem 99, 316-320) or Suc-AAPF-AMC (7-amido-4-methylcoumarin) as substrate. pNA or AMC is cleaved from the substrate molecule by proteolytic cleavage, resulting in release of yellow color of free pNA or change of fluorescence properties which can be quantified either by measuring emission at 460 nm after excitation at 380 nm.

The polypeptide of the present invention having protease activity preferably has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1%, at least 96.2%, at least 96.3%, at least 96.4%, at least 96.5%, at least 96.6%, at least 96.7%, at least 96.8%, at least 96.9%, at least 97%, at least 97.1 %, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57.

In one embodiment, the polypeptide having protease activity of the present invention comprises at least one amino acid substitution at a position selected from the group consisting of: 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the polypeptide having protease activity of the present invention comprises amino acid substitutions at positions 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the polypeptide having protease activity of the present invention comprises amino acid residue D or E at position 101, preferably E at position 101, referring to the numbering of SEQ ID NO: 42.

In one embodiment, the polypeptide having protease activity of the present invention comprises amino acid residue D or E at position 101, preferably E at position 101, referring to the numbering of SEQ ID NO: 42 and comprises at least one amino acid substitution at a position selected from the group consisting of: 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the polypeptide having protease activity of the present invention comprises amino acid residue D or E at position 101, preferably E at position 101, referring to the numbering of SEQ ID NO: 42 and comprises amino acid substitutions at positions 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution at a position selected from the group consisting of: 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution at a position selected from the group consisting of: 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises an amino acid substitution at positions 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39,SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises an amino acid substitution at positions 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution at a position selected from the group consisting of: 18, 38, 78, 183, 204 and 218 compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution at a position selected from the group consisting of: 18, 38, 78, 183, 204 and 218 compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises an amino acid substitution at positions 18, 38, 78, 183, 204 and 218 compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises an amino acid substitution at positions 18, 38, 78, 183, 204 and 218 compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: X18Q/E/D, X21I/V/F/W/Y, X24K, X38R/K/H/W, X56D, X78N/D/R/W/F/H/K/E/L/Y/M/C/Q, X109K,/A X144N/R, X160H, X182K/R/E, X183D/E/C/Q/A/M, X204D/E/C/G, X210I/V, X218T/S/D, X228S and X248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: X18Q/E/D, X21I/V/F/W/Y, X24K, X38R/K/H/W, X56D, X78N/D/R/W/F/H/K/E/L/Y/M/C/Q, X109K/A, X144N/R, X160H, X182K/R/E, X183D/E/C/Q/A/M, X204D/E/C/G, X210I/V, X218T/S/D, X228S and X248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions X18Q/E/D, X21I/V/F/W/Y, X24K, X38R/K/H/W, X56D, X78N/D/R/W/F/H/K/E/L/Y/M/C/Q, X109K/A, X144N/R, X160H, X182K/R/E, X183D/E/C/Q/A/M, X204D/E/C/G, X210I/V, X218T/S/D, X228S and X248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions X18Q/E/D, X21I/V/F/W/Y, X24K, X38R/K/H/W, X56D, X78N/D/R/W/F/H/K/E/L/Y/M/C/Q, X109K/A, X144N/R, X160H, X182K/R/E, X183D/E/C/Q/A/M, X204D/E/C/G, X210I/V, X218T/S/D, X228S and X248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: X18Q/E/D, X38R/K/H/W, X78N/D/R/W/F/H/K/E/L/Y/M/C/Q, X183D/E/C/Q/A/M, X204D/E/C/G and X218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: X18Q/E/D, X38R/K/H/W, X78N/D/R/W/F/H/K/E/L/Y/M/C/Q, X183D/E/C/Q/A/M, X204D/E/C/G and X218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions X18Q/E/D, X38R/K/H/W, X78N/D/R/W/F/H/K/E/L/Y/M/C/Q, X183D/E/C/Q/A/M, X204D/E/C/G and X218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 comprises a D or an E residue, preferably an E residue, at position 101 and comprises the amino acid substitutions X18Q/E/D, X38R/K/H/W, X78N/D/R/W/F/H/K/E/L/Y/M/C/Q, X183D/E/C/Q/A/M, X204D/E/C/G and X218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: X18Q, X21V, X24K, X38K/W, X56D, X78N, X109K, X144N, X160H, X182K, X183D, X204D, X210I, X218S, X228S and X248Q compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: X18Q, X21V, X24K, X38K/W, X56D, X78N, X109K, X144N, X160H, X182K, X183D, X204D, X210I, X218S, X228S and X248Q compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions X18Q, X21V, X24K, X38K/W, X56D, X78N, X109K, X144N, X160H, X182K, X183D, X204D, X210I, X218S, X228S and X248Q compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions X18Q, X21V, X24K, X38K/W, X56D, X78N, X109K, X144N, X160H, X182K, X183D, X204D, X210I, X218S, X228S and X248Q compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: X18Q, X38K/W, X78N, X183D, X204D and X218S compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: X18Q, X38K/W, X78N, X183D, X204D and X218S compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions X18Q, X38K/W, X78N, X183D, X204D and X218S compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions X18Q, X38K/W, X78N, X183D, X204D and X218S compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: N18Q/E/D, L21I/V/F/W/Y, S24K, T38R/K/H/W, S56D, S78N/D/R/W/F/H/K/E/L/Y/M/C/Q, Q109K/A, S144N/R, S160H, Q182K/R/E, N183D/E/C/Q/A/M, N204D/E/C/G, P210I/V, N218T/S/D, A228S and N248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: N18Q/E/D, L21I/V/F/W/Y, S24K, T38R/K/H/W, S56D, S78N/D/R/W/F/H/K/E/L/Y/M/C/Q, Q109K/A, S144N/R, S160H, Q182K/R/E, N183D/E/C/Q/A/M, N204D/E/C/G, P210I/V, N218T/S/D, A228S and N248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions N18Q/E/D, L21I/V/F/W/Y, S24K, T38R/K/H/W, S56D, S78N/D/R/W/F/H/K/E/L/Y/M/C/Q, Q109K/A, S144N/R, S160H, Q182K/R/E, N183D/E/C/Q/A/M, N204D/E/C/G, P210I/V, N218T/S/D, A228S and N248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions N18Q/E/D, L21I/V/F/W/Y, S24K, T38R/K/H/W, S56D, S78N/D/R/W/F/H/K/E/L/Y/M/C/Q, Q109K/A, S144N/R, S160H, Q182K/R/E, N183D/E/C/Q/A/M, N204D/E/C/G, P210I/V, N218T/S/D, A228S and N248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: N18Q/E/D, T38R/K/H/W, S78N/D/R/W/F/H/K/E/L/Y/M/C/Q, N183D/E/C/Q/A/M, N204D/E/C/G and N218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: N18Q/E/D, T38R/K/H/W, S78N/D/R/W/F/H/K/E/L/Y/M/C/Q, N183D/E/C/Q/A/M, N204D/E/C/G and N218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions N18Q/E/D, T38R/K/H/W, S78N/D/R/W/F/H/K/E/L/Y/M/C/Q, N183D/E/C/Q/A/M, N204D/E/C/G and N218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions N18Q/E/D, T38R/K/H/W, S78N/D/R/W/F/H/K/E/L/Y/M/C/Q, N183D/E/C/Q/A/M, N204D/E/C/G and N218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: N18Q, L21V, S24K, T38K/W, S56D, S78N, Q109K, S144N, S160H, Q182K, N183D, N204D, P210I, N218S, A228S and N248Q compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: N18Q, L21V, S24K, T38K/W, S56D, S78N, Q109K, S144N, S160H, Q182K, N183D, N204D, P210I, N218S, A228S and N248Q compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions N18Q, L21V, S24K, T38K/W, S56D, S78N, Q109K, S144N, S160H, Q182K, N183D, N204D, P210I, N218S, A228S and N248Q compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions N18Q, L21V, S24K, T38K/W, S56D, S78N, Q109K, S144N, S160H, Q182K, N183D, N204D, P210I, N218S, A228S and N248Q compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: N18Q, T38K/W, S78N, N183D, N204D and N218S compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: N18Q, T38K/W, S78N, N183D, N204D and N218S compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions N18Q, T38K/W, S78N, N183D, N204D and N218S compared to the amino acid sequence as set forth in SEQ ID NO: 41 and referring to the numbering of SEQ ID NO: 42.

In one embodiment, the amino acid sequence of the polypeptide having protease activity of the present invention is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions N18Q, T38K/W, S78N, N183D, N204D and N218S compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, and comprises at least one amino acid substitution at a position selected from the group consisting of: 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution at a position selected from the group consisting of: 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises an amino acid substitution at positions 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises an amino acid substitution at positions 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution at a position selected from the group consisting of: 18, 38, 78, 183, 204 and 218 compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution at a position selected from the group consisting of: 18, 38, 78, 183, 204 and 218 compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises an amino acid substitution at positions 18, 38, 78, 183, 204 and 218 compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises an amino acid substitution at positions 18, 38, 78, 183, 204 and 218 compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: X18Q/E/D, X21I/V/F/W/Y, X24K, X38R/K/H/W, X56D, X78N/D/R/W/F/H/K/E/L/Y/M/C/Q, X109K/A, X144N/R, X160H, X182K/R/E, X183D/E/C/Q/A/M, X204D/E/C/G, X210I/V, X218T/S/D, X228S and X248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: X18Q/E/D, X21I/V/F/W/Y, X24K, X38R/K/H/W, X56D, X78N/D/R/W/F/H/K/E/L/Y/M/C/Q, X109K/A, X144N/R, X160H, X182K/R/E, X183D/E/C/Q/A/M, X204D/E/C/G, X210I/V, X218T/S/D, X228S and X248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions X18Q/E/D, X21I/V/F/W/Y, X24K, X38R/K/H/W, X56D, X78N/D/R/W/F/H/K/E/L/Y/M/C/Q, X109K/A, X144N/R, X160H, X182K/R/E, X183D/E/C/Q/A/M, X204D/E/C/G, X210I/V, X218T/S/D, X228S and X248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions X18Q/E/D, X21I/V/F/W/Y, X24K, X38R/K/H/W, X56D, X78N/D/R/W/F/H/K/E/L/Y/M/C/Q, X109K/A, X144N/R, X160H, X182K/R/E, X183D/E/C/Q/A/M, X204D/E/C/G, X210I/V, X218T/S/D, X228S and X248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: X18Q/E/D, X38R/K/H/W, X78N/D/R/W/F/H/K/E/L/Y/M/C/Q, X183D/E/C/Q/A/M, X204D/E/C/G and X218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: X18Q/E/D, X38R/K/H/W, X78N/D/R/W/F/H/K/E/L/Y/M/C/Q, X183D/E/C/Q/A/M, X204D/E/C/G and X218T/S/D compared to the amino acid sequence as set forth in SEQ ID NO: 41 and referring to the numbering of SEQ ID NO: 42. In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions X18Q/E/D, X38R/K/H/W, X78N/D/R/W/F/H/K/E/L/Y/M/C/Q, X183D/E/C/Q/A/M, X204D/E/C/G and X218T/S/D compared to the amino acid sequence as set forth in SEQ ID NO: 41 and referring to the numbering of SEQ ID NO: 42. In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions X18Q/E/D, X38R/K/H/W, X78N/D/R/W/F/H/K/E/L/Y/M/C/Q, X183D/E/C/Q/A/M, X204D/E/C/G and X218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: X18Q, X21V, X24K, X38K/W, X56D, X78N, X109K, X144N, X160H, X182K, X183D, X204D, X210I, X218S, X228S and X248Q compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: X18Q, X21V, X24K, X38K/W, X56D, X78N, X109K, X144N, X160H, X182K, X183D, X204D, X210I, X218S, X228S and X248Q compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions X18Q, X21V, X24K, X38K/W, X56D, X78N, X109K, X144N, X160H, X182K, X183D, X204D, X210I, X218S, X228S and X248Q compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions X18Q, X21V, X24K, X38K/W, X56D, X78N, X109K, X144N, X160H, X182K, X183D, X204D, X210I, X218S, X228S and X248Q compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: X18Q, X38K/W, X78N, X183D, X204D and X218S compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: X18Q, X38K/W, X78N, X183D, X204D and X218S compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions X18Q, X38K/W, X78N, X183D, X204D and X218S compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions X18Q, X38K/W, X78N, X183D, X204D and X218S compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: N18Q/E/D, L21I/V/F/W/Y, S24K, T38R/K/H/W, S56D, S78N/D/R/W/F/H/K/E/L/Y/M/C/Q, Q109K/A, S144N/R, S160H, Q182K/R/E, N183D/E/C/Q/A/M, N204D/E/C/G, P210I/V, N218T/S/D, A228S and N248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: N18Q/E/D, L21I/V/F/W/Y, S24K, T38R/K/H/W, S56D, S78N/D/R/W/F/H/K/E/L/Y/M/C/Q, Q109K/A, S144N/R, S160H, Q182K/R/E, N183D/E/C/Q/A/M, N204D/E/C/G, P210I/V, N218T/S/D, A228S and N248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions N18Q/E/D, L21I/V/F/W/Y, S24K, T38R/K/H/W, S56D, S78N/D/R/W/F/H/K/E/L/Y/M/C/Q, Q109K/A, S144N/R, S160H, Q182K/R/E, N183D/E/C/Q/A/M, N204D/E/C/G, P210I/V, N218T/S/D, A228S and N248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions N18Q/E/D, L21I/V/F/W/Y, S24K, T38R/K/H/W, S56D, S78N/D/R/W/F/H/K/E/L/Y/M/C/Q, Q109K/A, S144N/R, S160H, Q182K/R/E, N183D/E/C/Q/A/M, N204D/E/C/G, P210I/V, N218T/S/D, A228S and N248Q/R compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: N18Q/E/D, T38R/K/H/W, S78N/D/R/W/F/H/K/E/L/Y/M/C/Q, N183D/E/C/Q/A/M, N204D/E/C/G and N218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: N18Q/E/D, T38R/K/H/W, S78N/D/R/W/F/H/K/E/L/Y/M/C/Q, N183D/E/C/Q/A/M, N204D/E/C/G and N218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions N18Q/E/D, T38R/K/H/W, S78N/D/R/W/F/H/K/E/L/Y/M/C/Q, N183D/E/C/Q/A/M, N204D/E/C/G and N218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions N18Q/E/D, T38R/K/H/W, S78N/D/R/W/F/H/K/E/L/Y/M/C/Q, N183D/E/C/Q/A/M, N204D/E/C/G and N218T/S/D compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: N18Q, L21V, S24K, T38K/W, S56D, S78N, Q109K, S144N, S160H, Q182K, N183D, N204D, P210I, N218S, A228S and N248Q compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: N18Q, L21V, S24K, T38K/W, S56D, S78N, Q109K, S144N, S160H, Q182K, N183D, N204D, P210I, N218S, A228S and N248Q compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions N18Q, L21V, S24K, T38K/W, S56D, S78N, Q109K, S144N, S160H, Q182K, N183D, N204D, P210I, N218S, A228S and N248Q compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions N18Q, L21V, S24K, T38K/W, S56D, S78N, Q109K, S144N, S160H, Q182K, N183D, N204D, P210I, N218S, A228S and N248Q compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises at least one amino acid substitution selected from the group consisting of: N18Q, T38K/W, S78N, N183D, N204D and N218S compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises at least one amino acid substitution selected from the group consisting of: N18Q, T38K/W, S78N, N183D, N204D and N218S compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 and comprises the amino acid substitutions N18Q, T38K/W, S78N, N183D, N204D and N218S compared to the amino acid sequence as set forth in SEQ ID NO: 41 and referring to the numbering of SEQ ID NO: 42.

In one embodiment, the amino acid sequence of the polypeptide having protease activity of the present invention is at least 98% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57, comprises a D or an E residue, preferably an E residue, at position 101 and comprises the amino acid substitutions N18Q, T38K/W, S78N, N183D, N204D and N218S compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 1, comprises a D or an E residue, preferably an E residue, at position 101 and comprises one or more, preferably all, of the amino acid substitutions S9A, N18Q, L21V, T38W, N43K, S78N, S160H, N183D, A194E, N204D, P210I, S212P, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 1, comprises a D or an E residue, preferably an E residue, at position 101 and comprises one or more, preferably all, of the amino acid substitutions S9A, N18Q, L21V, S24K, G25D, T38W, N43K, S56D, S78N, A103S, Q109K, S144N, S160H, Q182K, N183D, A194E, N204D, P210I, S212P, N218S, A228S, N248Q, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 2, comprises a D or an E residue, preferably an E residue, at position 101 and comprises one or more, preferably all, of the amino acid substitutions S9A, N18Q, L21V, T38K, N43K, S78N, S160H, N183D, A194E, N204D, P210I, S212P, N218S, A228S, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 2, comprises a D or an E residue, preferably an E residue, at position 101 and comprises one or more, preferably all, of the amino acid substitutions S9A, N18Q, L21V, S24K, G25D, T38K, N43K, S56D, S78N, A103S, Q109K, S144N, S160H, Q182K, N183D, A194E, N204D, P210I, S212P, N218S, A228S, N248Q, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 3, comprises a D or an E residue, preferably an E residue, at position 101 and comprises one or more, preferably all, of the amino acid substitutions S9A, N18Q, L21V, T38K, N43K, S78N, S160H, N183D, A194E, N204D, P210I, S212P, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 3, comprises a D or an E residue, preferably an E residue, at position 101 and comprises one or more, preferably all, of the amino acid substitutions S9A, N18Q, L21V, S24K, G25D, T38K, N43K, S56D, S78N, A103S, Q109K, S144N, S160H, Q182K, N183D, A194E, N204D, P210I, S212P, N218S, A228S, N248Q, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 4 , comprises a D or an E residue, preferably an E residue, at position 101 and comprises one or more, preferably all, of the amino acid substitutions S9A, N18Q, L21V, T38K, N43K, S78N, S160H, N183D, A194E, N204D, P210I, S212P, N218S, A228S, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 4, comprises a D or an E residue, preferably an E residue, at position 101 and comprises one or more, preferably all, of the amino acid substitutions S9A, N18Q, L21V, S24K, G25D, T38K, N43K, S56D, S78N, A103S, Q109K, S144N, S160H, Q182K, N183D, A194E, N204D, P210I, S212P, N218S, A228S, N248Q, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 5, comprises a D or an E residue, preferably an E residue, at position 101 and comprises one or more, preferably all, of the amino acid substitutions N18Q, L21V, T38K, S78N, S160H, N183D, A194E, N204D, P210I, S212P, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 5, comprises a D or an E residue, preferably an E residue, at position 101 and comprises one or more, preferably all, of the amino acid substitutions N18Q, L21V, S24K, G25D, T38K, S56D, S78N, A103S, Q109K, S144N, S160H, Q182K, N183D, A194E, N204D, P210I, S212P, N218S, A228S, N248Q, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 6, comprises a D or an E residue, preferably an E residue, at position 101 and comprises one or more, preferably all, of the amino acid substitutions N18Q, L21V, T38W, S78N, S160H, N183D, A194E, N204D, P210I, S212P, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 6, comprises a D or an E residue, preferably an E residue, at position 101 and comprises one or more, preferably all, of the amino acid substitutions N18Q, L21V, S24K, T38W, S56D, S78N, A103S, Q109K, S144N, S160H, Q182K, N183D, A194E, N204D, P210I, S212P, N218S, A228S, N248Q, T260D, N261 F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 7, comprises a D or an E residue, preferably an E residue, at position 101 and comprises one or more, preferably all, of the amino acid substitutions S9A, N18Q, L21V, T38K, S78N, S160H, N183D, N204D, P210I, S212P, N218S, A228S, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 7, comprises a D or an E residue, preferably an E residue, at position 101 and comprises one or more, preferably all, of the amino acid substitutions S9A, N18Q, L21V, S24K, G25D, T38K, S56D, S78N, A103S, Q109K, S144N, S160H, Q182K, N183D, N204D, P210I, S212P, N218S, A228S, N248Q, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 8, comprises a D or an E residue, preferably an E residue, at position 101 and comprises one or more, preferably all, of the amino acid substitutions S9A, N18Q, L21V, T38K, S78N, S160H, N183D, N204D, P210I, S212P, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 8, comprises a D or an E residue, preferably an E residue, at position 101 and comprises one or more, preferably all, of the amino acid substitutions S9A, N18Q, L21V, S24K, T38K, S56D, S78N, A103S, Q109K, S144N, S160H, Q182K, N183D, A194E, N204D, P210I, S212P, N218S, A228S, N248Q, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 9, comprises a D or an E residue, preferably an E residue, at position 101 and comprises one or more, preferably all, of the amino acid substitutions S9A, N18Q, L21V, T38K, S78N, S160H, N183D, A194E, N204D, P210I, S212P, N218S, A228S, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 9, comprises a D or an E residue, preferably an E residue, at position 101 and comprises one or more, preferably all, of the amino acid substitutions S9A, N18Q, L21V, S24K, T38K, S56D, S78N, A103S, Q109K, S144N, S160H, Q182K, N183D, A194E, N204D, P210I, S212P, N218S, A228S, N248Q, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 10, comprises a D or an E residue, preferably an E residue, at position 101 and comprises one or more, preferably all, of the amino acid substitutions N18Q, L21V, T38K, S78N, S160H, N183D, N204D, P210I, S212P, N218S, A228S, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1 %, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 10, comprises a D or an E residue, preferably an E residue, at position 101 and comprises one or more, preferably all, of the amino acid substitutions N18Q, L21V, S24K, T38K, S56D, S78N, A103S, Q109K, S144N, S160H, Q182K, N183D, N204D, P210I, S212P, N218S, A228S, N248Q, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1 %, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1 %, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 11, comprises a D or an E residue, preferably an E residue, at position 101 and comprises one or more, preferably all, of the amino acid substitutions N18Q, L21V, T38K, N43K, S78N, S160H, N183D, A194E, N204D, P210I, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1 %, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 11, comprises a D or an E residue, preferably an E residue, at position 101 and comprises one or more, preferably all, of the amino acid substitutions N18Q, L21V, S24K, G25D, T38K, N43K, S56D, S78N, A103S, Q109K, S144N, S160H, Q182K, N183D, A194E, N204D, P210I, N218S, A228S, N248Q, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 12, comprises a D or an E residue, preferably an E residue, at position 101 and comprises one or more, preferably all, of the amino acid substitutions S9A, N18Q, L21V, T38K, N43K, S78N, S160H, N183D, A194E, N204D, P210I, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 12, comprises a D or an E residue, preferably an E residue, at position 101 and comprises one or more, preferably all, of the amino acid substitutions S9A, N18Q, L21V, S24K, G25D, T38K, N43K, S56D, S78N, A103S, Q109K, S144N, S160H, Q182K, N183D, A194E, N204D, P210I, N218S, A228S, N248Q, T260D, N261F and E271D compared to the amino acid sequence as set forth in SEQ iD NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 13, comprises a D or an E residue, preferably an E residue, at position 101 and comprises one or more, preferably all, of the amino acid substitutions S9A, N18Q, L21V, T38K, N43K, S78N, S160H, N183D, N204D, P210I, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 13, comprises a D or an E residue, preferably an E residue, at position 101 and comprises one or more, preferably all, of the amino acid substitutions S9A, N18Q, L21V, S24K, G25D, T38K, N43K, S56D, S78N, A103S, Q109K, S144N, S160H, Q182K, N183D, N204D, P210I, N218S, A228S, N248Q, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 14, comprises a D or an E residue, preferably an E residue, at position 101 and comprises one or more, preferably all, of the amino acid substitutions S9A, N18Q, L21V, T38K, N43K, S78N, S160H, N183D, A194E, N204D, P210I, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 14, comprises a D or an E residue, preferably an E residue, at position 101 and comprises one or more, preferably all, of the amino acid substitutions S9A, N18Q, L21V, S24K, T38K, N43K, S56D, S78N, A103S, Q109K, S144N, S160H, Q182K, N183D, A194E, N204D, P210I, N218S, A228S, N248Q, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 15, comprises a D or an E residue, preferably an E residue, at position 101 and comprises one or more, preferably all, of the amino acid substitutions S9A, N18Q, L21V, T38K, S78N, S160H, N183D, N204D, P210I, N218S, A228S, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 15, comprises a D or an E residue, preferably an E residue, at position 101 and comprises one or more, preferably all, of the amino acid substitutions S9A, N18Q, L21V, S24K, T38K, S56D, S78N, Q109K, S144N, S160H, Q182K, N183D, N204D, P210I, N218S, A228S, N248Q, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 16, comprises a D or an E residue, preferably an E residue, at position 101 and comprises one or more, preferably all, of the amino acid substitutions N18Q, L21V, T38K, N43R, S78N, S160H, N183D, A194E, N204D, P210I, S212P, N218S, A228S and N261F compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1%, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 16, comprises a D or an E residue, preferably an E residue, at position 101 and comprises one or more, preferably all, of the amino acid substitutions N18Q, L21V, S24K, G25D, T38K, N43R, S56D, S78N, A103S, Q109K, S144N, S160H, Q182K, N183D, A194E, N204D, P210I, S212P, N218S, A228S, N248Q and N261F compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 17, comprises a D or an E residue, preferably an E residue, at position 101 and comprises one or more, preferably all, of the amino acid substitutions N18Q, L21V, T38K, N43R, S78N, S160H, N183D, A194E, N204D, P210I, S212P, N218S and A228S compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 17, comprises a D or an E residue, preferably an E residue, at position 101 and comprises one or more, preferably all, of the amino acid substitutions N18Q, L21V, S24K, G25D, T38K, N43R, S56D, S78N, Q109K, S144N, S160H, Q182K, N183D, A194E, N204D, P210I, S212P, N218S, A228S and N248Q compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 18, comprises a D or an E residue, preferably an E residue, at position 101 and comprises one or more, preferably all, of the amino acid substitutions S9A, N18Q, L21V, T38K, N43R, S78N, S160H, N183D, A194E, N204D, P210I, S212P, N218S, A228S, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 18, comprises a D or an E residue, preferably an E residue, at position 101 and comprises one or more, preferably all, of the amino acid substitutions S9A, N18Q, L21V, S24K, G25D, T38K, N43R, S56D, S78N, A103S, Q109K, S144N, S160H, Q182K, N183D, A194E, N204D, P210I, S212P, N218S, A228S, N248Q, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 19, comprises a D or an E residue, preferably an E residue, at position 101 and comprises one or more, preferably all, of the amino acid substitutions N18Q, L21V, T38K, N43K, S78N, S160H, N183D, A194E, N204D, P210I, S212P, N218S, A228S, S259D and N261F compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 19, comprises a D or an E residue, preferably an E residue, at position 101 and comprises one or more, preferably all, of the amino acid substitutions N18Q, L21V, S24K, G25D, T38K, N43K, S56D, S78N, Q109K, S144N, S160H, Q182K, N183D, A194E, N204D, P210I, S212P, N218S, A228S, N248Q, S259D and N261F compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 20, comprises a D or an E residue, preferably an E residue, at position 101 and comprises one or more, preferably all, of the amino acid substitutions S9A, N18Q, L21V, T38W, N43K, S78N, S160H, N183D, N204D, P210I, S212P, N218S, A228S, S259D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 20, comprises a D or an E residue, preferably an E residue, at position 101 and comprises one or more, preferably all, of the amino acid substitutions S9A, N18Q, L21V, S24K, G25D, T38W, N43K, S56D, S78N, A103S, Q109K, S144N, S160H, Q182K, N183D, N204D, P210I, S212P, N218S, A228S, N248Q, S259D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 21, comprises a D or an E residue, preferably an E residue, at position 101 and comprises one or more, preferably all, of the amino acid substitutions S9A, N18Q, L21V, T38K, N43K, S78N, S160H, N183D, A194E, N204D, P210I, S212P, N218S, A228S, S259D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 21, comprises a D or an E residue, preferably an E residue, at position 101 and comprises one or more, preferably all, of the amino acid substitutions S9A, N18Q, L21V, S24K, G25D, T38K, N43K, S56D, S78N, A103S, Q109K, S144N, S160H, Q182K, N183D, A194E, N204D, P210I, S212P, N218S, A228S, N248Q, S259D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 22, comprises a D or an E residue, preferably an E residue, at position 101 and comprises one or more, preferably all, of the amino acid substitutions S9A, N18Q, L21V, T38K, N43K, S78N, S160H, N183D, A194E, N204D, P210I, S212P, N218S, A228S, S259D and N261F compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 22, comprises a D or an E residue, preferably an E residue, at position 101 and comprises one or more, preferably all, of the amino acid substitutions S9A, N18Q, L21V, S24K, G25D, T38K, N43K, S56D, S78N, A103S, Q109K, S144N, S160H, Q182K, N183D, A194E, N204D, P210I, S212P, N218S, A228S, N248Q, S259D and N261F compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 23, comprises a D or an E residue, preferably an E residue, at position 101 and comprises one or more, preferably all, of the amino acid substitutions S9A, N18Q, L21V, T38K, N43K, S78N, S160H, N183D, N204D, P210I, S212P, N218S, A228S, S259D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 23, comprises a D or an E residue, preferably an E residue, at position 101 and comprises one or more, preferably all, of the amino acid substitutions S9A, N18Q, L21V, S24K, G25D, T38K, N43K, S56D, S78N, A103S, Q109K, S144N, S160H, Q182K, N183D, N204D, P210I, S212P, N218S, A228S, N248Q, S259D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 24, comprises a D or an E residue, preferably an E residue, at position 101 and comprises one or more, preferably all, of the amino acid substitutions S9A, N18Q, L21V, T38K, N43K, S78N, S160H, N183D, N204D, P210I, S212P, N218S, A228S, S259D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 24, comprises a D or an E residue, preferably an E residue, at position 101 and comprises one or more, preferably all, of the amino acid substitutions S9A, N18Q, L21V, S24K, G25D, T38K, N43K, S56D, S78N, A103S, Q109K, S144N, S160H, Q182K, N183D, N204D, P210I, S212P, N218S, A228S, N248Q, S259D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 25, comprises a D or an E residue, preferably an E residue, at position 101 and comprises one or more, preferably all, of the amino acid substitutions S9A, N18Q, L21V, T38K, N43K, S78N, S160H, N183D, A194E, N204D, P210I, S212P, N218S, A228S, S259D and N261F compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 25, comprises a D or an E residue, preferably an E residue, at position 101 and comprises one or more, preferably all, of the amino acid substitutions S9A, N18Q, L21V, S24K, T38K, N43K, S56D, S78N, A103S, Q109K, S144N, S160H, Q182K, N183D, A194E, N204D, P210I, S212P, N218S, A228S, N248Q, S259D and N261F compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 26, comprises a D or an E residue, preferably an E residue, at position 101 and comprises one or more, preferably all, of the amino acid substitutions S9A, N18Q, L21V, T38K, N43K, S78N, S160H, N183D, A194E, N204D, P210I, S212P, N218S, A228S, S259D, N261 F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 26, comprises a D or an E residue, preferably an E residue, at position 101 and comprises one or more, preferably all, of the amino acid substitutions S9A, N18Q, L21V, S24K, T38K, N43K, S56D, S78N, A103S, Q109K, S144N, S160H, Q182K, N183D, A194E, N204D, P210I, S212P, N218S, A228S, N248Q, S259D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 27, comprises a D or an E residue, preferably an E residue, at position 101 and comprises one or more, preferably all, of the amino acid substitutions S9A, N18Q, L21V, T38K, N43K, S78N, S160H, N183D, N204D, P210I, S212P, N218S, A228S, S259D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 27, comprises a D or an E residue, preferably an E residue, at position 101 and comprises one or more, preferably all, of the amino acid substitutions S9A, N18Q, L21V, S24K, T38K, N43K, S56D, S78N, A103S, Q109K, S144N, S160H, Q182K, N183D, N204D, P210I, S212P, N218S, A228S, N248Q, S259D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 28, comprises a D or an E residue, preferably an E residue, at position 101 and comprises one or more, preferably all, of the amino acid substitutions N18Q, L21V, T38W, Q59K, S78N, S160H, N183D, A194E, N204D, P210I, S212P, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 28, comprises a D or an E residue, preferably an E residue, at position 101 and comprises one or more, preferably all, of the amino acid substitutions N18Q, L21V, S24K, T38W, S56D, Q59K, S78N, A103S, Q109K, S144N, S160H, Q182K, N183D, A194E, N204D, P210I, S212P, N218S, A228S, N248Q, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 29, comprises a D or an E residue, preferably an E residue, at position 101 and comprises one or more, preferably all, of the amino acid substitutions S9A, N18Q, L21V, T38W, Q59K, S78N, S160H, N183D, A194E, N204D, P210I, S212P, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 29, comprises a D or an E residue, preferably an E residue, at position 101 and comprises one or more, preferably all, of the amino acid substitutions S9A, N18Q, L21V, S24K, G25D, T38W, S56D, Q59K, S78N, A103S, Q109K, S144N, S160H, Q182K, N183D, A194E, N204D, P210I, S212P, N218S, A228S, N248Q, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 30, comprises a D or an E residue, preferably an E residue, at position 101 and comprises one or more, preferably all, of the amino acid substitutions S9A, N18Q, L21V, T38W, Q59K, S78N, S160H, N183D, N204D, P210I, S212P, N218S, A228S, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 30, comprises a D or an E residue, preferably an E residue, at position 101 and comprises one or more, preferably all, of the amino acid substitutions S9A, N18Q, L21V, S24K, G25D, T38W, S56D, Q59K, S78N, A103S, Q109K, S144N, S160H, Q182K, N183D, N204D, P210I, S212P, N218S, A228S, N248Q, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 31, comprises a D or an E residue, preferably an E residue, at position 101 and comprises one or more, preferably all, of the amino acid substitutions S9A, N18Q, L21V, T38W, Q59K, S78N, S160H, N183D, N204D, P210I, S212P, N218S, A228S, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 31, comprises a D or an E residue, preferably an E residue, at position 101 and comprises one or more, preferably all, of the amino acid substitutions S9A, N18Q, L21V, S24K, G25D, T38W, S56D, Q59K, S78N, Q109K, S144N, S160H, Q182K, N183D, N204D, P210I, S212P, N218S, A228S, N248Q, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 32, comprises a D or an E residue, preferably an E residue, at position 101 and comprises one or more, preferably all, of the amino acid substitutions S9A, N18Q, L21V, T38K, Q59K, S78N, S160H, N183D, A194E N204D, P210I, S212P, N218S, A228S, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 32, comprises a D or an E residue, preferably an E residue, at position 101 and comprises one or more, preferably all, of the amino acid substitutions S9A, N18Q, L21V, S24K, G25D, T38K, S56D, Q59K, S78N, A103S, Q109K, S144N, S160H, Q182K, N183D, A194E, N204D, P210I, S212P, N218S, A228S, N248Q, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 33, comprises a D or an E residue, preferably an E residue, at position 101 and comprises one or more, preferably all, of the amino acid substitutions S9A, N18Q, L21V, T38K, Q59K, S78N, S160H, N183D, A194E N204D, P210I, S212P, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 33, comprises a D or an E residue, preferably an E residue, at position 101 and comprises one or more, preferably all, of the amino acid substitutions S9A, N18Q, L21V, S24K, G25D, T38K, S56D, Q59K, S78N, A103S, Q109K, S144N, S160H, Q182K, N183D, A194E, N204D, P210I, S212P, N218S, A228S, N248Q, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 34, comprises a D or an E residue, preferably an E residue, at position 101 and comprises one or more, preferably all, of the amino acid substitutions S9A, N18Q, L21V, T38K, Q59K, S78N, S160H, N183D, A194E N204D, P210I, S212P, N218S, A228S, S259D, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 34, comprises a D or an E residue, preferably an E residue, at position 101 and comprises one or more, preferably all, of the amino acid substitutions S9A, N18Q, L21V, S24K, G25D, T38K, S56D, Q59K, S78N, A103S, Q109K, S144N, S160H, Q182K, N183D, A194E, N204D, P210I, S212P, N218S, A228S, N248Q, S259D, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 35, comprises a D or an E residue, preferably an E residue, at position 101 and comprises one or more, preferably all, of the amino acid substitutions S9A, N18Q, L21V, T38K, Q59K, S78N, S160H, N183D, A194E N204D, P210I, S212P, N218S, A228S, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 35, comprises a D or an E residue, preferably an E residue, at position 101 and comprises one or more, preferably all, of the amino acid substitutions S9A, N18Q, L21V, S24K, G25D, T38K, S56D, Q59K, S78N, A103S, Q109K, S144N, S160H, Q182K, N183D, A194E, N204D, P210I, S212P, N218S, A228S, N248Q, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 36, comprises a D or an E residue, preferably an E residue, at position 101 and comprises one or more, preferably all, of the amino acid substitutions S9A, N18Q, L21V, T38K, Q59K, S78N, S160H, N183D, N204D, P210I, S212P, N218S, A228S, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 36, comprises a D or an E residue, preferably an E residue, at position 101 and comprises one or more, preferably all, of the amino acid substitutions S9A, N18Q, L21V, S24K, G25D, T38K, S56D, Q59K, S78N, A103S, Q109K, S144N, S160H, Q182K, N183D, N204D, P210I, S212P, N218S, A228S, N248Q, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 37, comprises a D or an E residue, preferably an E residue, at position 101 and comprises one or more, preferably all, of the amino acid substitutions S9A, N18Q, L21V, T38K, Q59K, S78N, S160H, N183D, N204D, P210I, S212P, N218S, A228S, S259D, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 37, comprises a D or an E residue, preferably an E residue, at position 101 and comprises one or more, preferably all, of the amino acid substitutions S9A, N18Q, L21V, S24K, G25D, T38K, S56D, Q59K, S78N, Q109K, S144N, S160H, Q182K, N183D, N204D, P210I, S212P, N218S, A228S, N248Q, S259D, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 38, comprises a D or an E residue, preferably an E residue, at position 101 and comprises one or more, preferably all, of the amino acid substitutions S9A, N18Q, L21V, T38W, Q59K, S78N, S160H, N183D, N204D, P210I, S212P, N218S, A228S, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 38, comprises a D or an E residue, preferably an E residue, at position 101 and comprises one or more, preferably all, of the amino acid substitutions S9A, N18Q, L21V, S24K, T38W, S56D, Q59K, S78N, A103S, Q109K, S144N, S160H, Q182K, N183D, N204D, P210I, S212P, N218S, A228S, N248Q, S259D, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 39, comprises a D or an E residue, preferably an E residue, at position 101 and comprises one or more, preferably all, of the amino acid substitutions N18Q, L21V, T38K, Q59K, S78N, S160H, N183D, N204D, P210I, S212P, N218S, A228S, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 39, comprises a D or an E residue, preferably an E residue, at position 101 and comprises one or more, preferably all, of the amino acid substitutions N18Q, L21V, S24K, T38K, S56D, Q59K, S78N, A103S, Q109K, S144N, S160H, Q182K, N183D, A194E, N204D, P210I, S212P, N218S, A228S, N248Q, T260D and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 40, comprises a D or an E residue, preferably an E residue, at position 101 and comprises one or more, preferably all, of the amino acid substitutions N18Q, L21V, T38K, Q59K, S78N, S160H, N183D, N204D, P210I, S212P, N218S, A228S, T260D, N261F and E271D compared to the amino acid sequence as set forth in SEQ ID NO: 41 and referring to the numbering of SEQ ID NO: 42.

In one embodiment, the amino acid sequence of the polypeptide having protease activity of the present invention has a sequence identity of at least 95%, at least 95.1%, at least 95.2%, at least 95.3%, at least 95.4%, at least 95.5%, at least 95.6%, at least 95.7%, at least 95.8%, at least 95.9% at least 96%, at least 96.1 %, at least 96.2%, at least 96.3%, at least 96.4%, at least 96.5%, at least 96.6%, at least 96.7%, at least 96.8%, at least 96.9%, at least 97%, at least 97.1%, at least 97.2%, at least 97.3%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% or 100% sequence identity to the amino acid sequence according to SEQ ID NO: 40, comprises a D or an E residue, preferably an E residue, at position 101 and comprises one or more, preferably all, of the amino acid substitutions N18Q, L21V, S24K, T38K, S56D, Q59K, S78N, A103S, Q109K, S144N, S160H, Q182K, N183D, A194E, N204D, P210I, S212P, N218S, A228S, N248Q, T260D, N261F and E271D compared to the amino acid sequence as set forth in SEQ ID NO: 41 and referring to the numbering of SEQ ID NO: 42.

In a preferred embodiment, the polypeptide having protease activity has an amino acid sequence which is at least 95% identical to an amino acid sequence selected from the group consisting of SEQ ID NO: 1, SEQ ID NO: 3, SEQ ID NO: 5, SEQ ID NO: 15, SEQ ID NO: 32 and SEQ ID NO: 33.

In a preferred embodiment, the polypeptide having protease activity has an amino acid sequence which is at least 95% identical to an amino acid sequence selected from the group consisting of SEQ ID NO: 1, SEQ ID NO: 3, SEQ ID NO: 5, SEQ ID NO: 32 and SEQ ID NO: 33.

In a preferred embodiment, the polypeptide having protease activity has an amino acid sequence which is at least 98% identical to an amino acid sequence selected from the group consisting of SEQ ID NO: 1, SEQ ID NO: 3, SEQ ID NO: 5, SEQ ID NO: 15, SEQ ID NO: 32 and SEQ ID NO: 33.

In a preferred embodiment, the polypeptide having protease activity has an amino acid sequence which is at least 98% identical to an amino acid sequence selected from the group consisting of SEQ ID NO: 1, SEQ ID NO: 3, SEQ ID NO: 5, SEQ ID NO: 32 and SEQ ID NO: 33.

In a preferred embodiment, the polypeptide having protease activity has an amino acid sequence which is selected from the group consisting of SEQ ID NO: 1, SEQ ID NO: 3, SEQ ID NO: 5, SEQ ID NO: 15, SEQ ID NO: 32 and SEQ ID NO: 33.

In a preferred embodiment, the polypeptide having protease activity has an amino acid sequence which is selected from the group consisting of SEQ ID NO: 1, SEQ ID NO: 3, SEQ ID NO: 5, SEQ ID NO: 32 and SEQ ID NO: 33.

In a preferred embodiment, the polypeptide having protease activity has an amino acid sequence which is at least 95% identical to an amino acid sequence selected from the group consisting of SEQ ID NO: 1, SEQ ID NO: 3, SEQ ID NO: 5, SEQ ID NO: 15, SEQ ID NO: 32 and SEQ ID NO: 33 and comprises the amino acid substitutions X18Q, X21V, X24K, X25D, X38W/K, X56D, X78N, X103S, X109K, X144N, X160H, X182K, X183D, X194E, X204D, X210I, X212P, X218S, X228S, X248Q, X260D and X271D compared to the amino acid sequence as set forth in SEQ ID NO: 41 and referring to the numbering of SEQ ID NO: 42.

In a preferred embodiment, the polypeptide having protease activity has an amino acid sequence which is at least 95% identical to an amino acid sequence selected from the group consisting of SEQ ID NO: 1, SEQ ID NO: 3, SEQ ID NO: 5, SEQ ID NO: 32 and SEQ ID NO: 33 and comprises the amino acid substitutions X18Q, X21V, X24K, X25D, X38W/K, X56D, X78N, X103S, X109K, X144N, X160H, X182K, X183D, X194E, X204D, X210I, X212P, X218S, X228S, X248Q, X260D and X271D compared to the amino acid sequence as set forth in SEQ ID NO: 41 and referring to the numbering of SEQ ID NO: 42.

In a preferred embodiment, the polypeptide having protease activity has an amino acid sequence which is at least 98% identical to an amino acid sequence selected from the group consisting of SEQ ID NO: 1, SEQ ID NO: 3, SEQ ID NO: 5, SEQ ID NO: 15, SEQ ID NO: 32 and SEQ ID NO: 33 and comprises the amino acid substitutions X18Q, X21V, X24K, X25D, X38W/K, X56D, X78N, X103S, X109K, X144N, X160H, X182K, X183D, X194E, X204D, X210I, X212P, X218S, X228S, X248Q, X260D and X271D compared to the amino acid sequence as set forth in SEQ ID NO: 41 and referring to the numbering of SEQ ID NO: 42.

In a preferred embodiment, the polypeptide having protease activity has an amino acid sequence which is at least 98% identical to an amino acid sequence selected from the group consisting of SEQ ID NO: 1, SEQ ID NO: 3, SEQ ID NO: 5, SEQ ID NO: 32 and SEQ ID NO: 33 and comprises the amino acid substitutions X18Q, X21V, X24K, X25D, X38W/K, X56D, X78N, X103S, X109K, X144N, X160H, X182K, X183D, X194E, X204D, X210I, X212P, X218S, X228S, X248Q, X260D and X271D compared to the amino acid sequence as set forth in SEQ ID NO: 41 and referring to the numbering of SEQ ID NO: 42.

In a preferred embodiment, the polypeptide having protease activity has an amino acid sequence which is selected from the group consisting of SEQ ID NO: 1, SEQ ID NO: 3, SEQ ID NO: 5, SEQ ID NO: 15, SEQ ID NO: 32 and SEQ ID NO: 33 and comprises the amino acid substitutions X18Q, X21V, X24K, X25D, X38W/K, X56D, X78N, X103S, X109K, X144N, X160H, X182K, X183D, X194E, X204D, X210I, X212P, X218S, X228S, X248Q, X260D and X271D compared to the amino acid sequence as set forth in SEQ ID NO: 41 and referring to the numbering of SEQ ID NO: 42.

In a preferred embodiment, the polypeptide having protease activity has an amino acid sequence which is selected from the group consisting of SEQ ID NO: 1, SEQ ID NO: 3, SEQ ID NO: 5, SEQ ID NO: 32 and SEQ ID NO: 33 and comprises the amino acid substitutions X18Q, X21V, X24K, X25D, X38W/K, X56D, X78N, X103S, X109K, X144N, X160H, X182K, X183D, X194E, X204D, X210I, X212P, X218S, X228S, X248Q, X260D and X271D compared to the amino acid sequence as set forth in SEQ ID NO: 41 and referring to the numbering of SEQ ID NO: 42.

In one embodiment, the protease variant of the present invention exhibits one or more improved properties compared to the parent protease, preferably compared to the protease according to SEQ ID NO: 41 or 43, preferably compared to the protease according to SEQ ID NO: 43.

Preferably, the variant polypeptide having protease activity of the present invention shows an increased stability, in particular storage stability, compared to the parent protease according to SEQ ID NO: 41 or 43, preferably compared to the protease according to SEQ ID NO: 43. The increased stability is preferably indicated as residual activity after stability challenge. Preferably, storage stability is indicated as residual activity after storage under the respective storage conditions, preferably, after storage in a detergent composition (preferably in a laundry or dishwash detergent, preferably laundry detergent). Preferably, the residual activity of the protease variant is increased compared to the residual activity of the parent protease SEQ ID NO: 41 or 43, preferably the protease according to SEQ ID NO: 43, after storage. Preferably, the residual activity of the protease variant after storage is at least 0.5%, at least 1%, at least 2%, at least 3%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, at least 160%, at least 170%, at least 180%, at least 190%, or at least 200% increased compared to the residual activity of the parent protease, preferably compared to the protease SEQ ID NO: 41 or 43, preferably compared to the protease according to SEQ ID NO: 43.

Preferably, the improved property is one or more property selected from the group consisting of an increase in stability, thermostability, performance in a detergent, performance in a laundry detergent, performance in an ADW detergent. Preferably, the improved activity is improved wash performance, wash performance of a laundry detergent, and/or wash performance of an ADW detergent

Preferably, the improved property is improved stability in a detergent composition, preferably a laundry detergent composition, preferably in a detergent composition comprising 1-20%, more preferably 5-10% anionic surfactants, preferably LAS, and/or greater than 50% water, preferably in a Model B type detergent as described herein. In one embodiment, the improved property is improved stability in a detergent composition, preferably a laundry detergent composition, preferably in a detergent composition comprising 0.1-5%, preferably 0.3-3%, of a strong sequestering builder. In this embodiment, the detergent composition preferably comprises 1-20%, more preferably 5-10% anionic surfactants, preferably LAS, and/or greater than 50% water.

### Nucleic acid construct

The present invention also refers to a polynucleotide encoding the polypeptide having protease activity of the present invention. Preferably, the polynucleotide is a codon-optimized polynucleotide for improving expression in a specific host cell, preferably a Bacillus cell.

The present invention also refers to a nucleic acid construct, preferably an expression cassette, comprising the polynucleotide as described herein.

Typically, the expression cassette comprises three elements: a promoter sequence, an open reading frame and a 3' untranslated region that, in eukaryotes, usually contains a polyadenylation site. Additional regulatory elements may include transcriptional as well as translational enhancers. An intron sequence may also be added to the 5' untranslated region (UTR) or in the coding sequence to increase the amount of the mature message that accumulates in the cytosol.

The expression cassette may be part of a vector or may be integrated into the genome of a host cell and replicated together with the genome of its host cell. The expression cassette usually is capable of increasing or decreasing expression.

The present invention also refers to an expression vector comprising the polynucleotide or the nucleic acid construct as described herein. The expression vector can be a low copy number vector or high copy number vector.

A vector as used herein may provide segments for transcription and translation of a foreign polynucleotide upon transformation into a host cell or host cell organelles. Such additional segments may include regulatory nucleotide sequences, one or more origins of replication that is required for its maintenance and/or replication in a specific cell type, one or more selectable markers, a polyadenylation signal, a suitable site for the insertion of foreign coding sequences such as a multiple cloning site etc. One example is when a vector is required to be maintained in a bacterial cell as an episomal genetic element (e.g., plasmid or cosmid molecule). Non-limiting examples of suitable origins of replication include the f1-ori and colE1.

A vector may replicate without integrating into the genome of a host cell, e.g., as a plasmid in a bacterial host cell, or it may integrate part or all of its DNA into the genome of the host cell and thus lead to replication and expression of its DNA.

The polynucleotide encoding the polypeptide having protease activity of the present invention may be introduced into a vector by means of standard recombinant DNA techniques. Once introduced into the vector, the polynucleotide comprising a coding sequence may be suitable to be introduced (transformed, transduced, transfected, etc.) into a host cell or host cell organelles. A cloning vector may be chosen suitable for expression of the polynucleotide sequence in the host cell or host cell organelles.

### Host cell

The present invention also refers to a host cell comprising the polynucleotide encoding the polypeptide having protease activity of the present invention, the nucleic acid construct as described herein, or the expression vector as described herein. In one embodiment of the invention, a vector is used for transformation of a host cell. Preferably, the host cell is capable of expressing the polynucleotide encoding the polypeptide having protease activity of the present invention. The polynucleotide encoding the polypeptide having protease activity of the present invention herein may be transiently or stably introduced into a host cell. The polynucleotide may be maintained non-integrated, for example, as a plasmid. Usually, stable transformation is due to integration of nucleic acid comprising a foreign coding sequence into the host cell chromosomes or as an episome (separate piece of nuclear DNA). Usually, transient transformation is due to nucleic acid comprising a foreign nucleic acid sequence not being integrated into the host cell chromosomes or as an episome. Methods for introducing a nucleic acid into a host cell are well-known in the art.

Various host cells can be used for expressing the nucleic acid construct described herein. The host cell of the present invention does not naturally express the polypeptide having protease activity of the present invention. Thus, the host cell is a recombinant host cell; the nucleic acid construct described herein is heterologous for the host cell.

In one embodiment, the host cell is a prokaryote or a eukaryote. In another embodiment, the host cell is a bacteria, an archaea, a fungal cell, a yeast cell or a eukaryotic cell. In another embodiment, the host cell is a non-human host cell. In one embodiment, the host cell is a bacterial cell. The bacterial host cell may be any gram-positive bacterium or a gram-negative bacterium. Gram-positive bacteria include, but are not limited to, Bacillus, Brevibacterium, Corynebac-terium, Streptococcus, Streptomyces, Staphylococcus, Enterococcus, Lactobacillus, Lactococcus, Clostridium, Geobacillus, and Oceanobacillus. Gram-negative bacteria include, but are not limited to, Escherichia, Pseudomonas, Salmonella, Campylobacter, Helicobacter, Acetobacter, Flavobacterium, Fusobacterium, Gluconobacter. In a specific embodiment, the bacterial host cell is a Echerichia coli cell. In one embodiment, the host cell is a bacterial cell. In a specific embodiment the host cell is of the genus Escherichia or Bacillus.

In the methods of the present invention, the bacterial host cell may be any Bacillus cell. Bacillus cells useful in the practice of the present invention include, but are not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus*, *Bacillus stearothermophilus, Bacillus methylotrophicus, Bacillus cereus Bacillus paralicheniformis, Bacillus subtilis,* and *Bacillus thuringiensis cells.* In one embodiment, the bacterial host cell is a *Bacillus amyloliquefaciens, Bacillus pumilus, Bacillus lentus, Bacillus licheniformis, Bacillus stearothermophilus* or *Bacillus subtilis* cell. In preferred embodiment, the bacterial host cell is a *Bacillus licheniformis cell, a Bacillus pumilus, or a Bacillus subtilis* cell. Preferably, the bacterial host cell is a *Bacillus licheniformis* cell.

### Methods of making

The polypeptide having protease activity of the present invention can be produced in an industrial scale and subsequently purified. Industrial production of enzymes usually is done by cultivating a host cell (also called fermentation) which expresses the enzyme. Suitable host cells are described herein. A nucleic acid sequence encoding the polypeptide having protease activity of the present invention can be transformed into the host cell, which is subsequently cultivated under conditions suitable for the host cell to produce the polypeptide having protease activity of the present invention. In a preferred embodiment, the polypeptide having protease activity of the present invention is purified from the host cell.

Hence, in yet another embodiment, the present invention is directed to a method of producing a polypeptide having protease activity, comprising the steps of
(a) providing a host cell comprising a heterologous nucleic acid construct comprising a polynucleotide encoding the polypeptide having protease activity;
(b) cultivating the recombinant host cell of step (a) under conditions conductive for the expression of the polynucleotide; and
(c) optionally, recovering the polypeptide having protease activity encoded by the polynucleotide.

Cultivation of the host cell normally takes place in a suitable nutrient medium allowing the recombinant cells to grow and express the desired protein. At the end of fermentation, the fermentation broth comprising a liquid fraction and a solid fraction is collected and may be further processed. The polypeptide having protease activity may be further purified from the fermentation broth.

The polypeptide having protease activity described herein may be secreted (into the liquid fraction of the fermentation broth) or may not be secreted from the microbial cells (and therefore is comprised in the cells of the fermentation broth). Depending on this, the polypeptide having protease activity may be recovered from the liquid fraction of the fermentation broth or from cell lysates. Preferably, the polypeptide having protease activity is secreted from the cell into the fermentation broth, preferably by means of a secretion signal peptide added to the N-terminus of the amino acid sequence, forming a propeptide of the polypeptide. The polypeptide is folded upon cleavage of the propeptide and the mature protease is released as active variant. Within the cultivation process measures might be taken to actively protect the protein from autocatalysis such as described in WO 2024/028340 or WO 2024/028338. Recovery of the polypeptide having protease activity can be achieved by methods known to those skilled in the art. Suitable methods for recovery of proteins from fermentation broth include, but are not limited to, collection, centrifugation, filtration, extraction, and precipitation. If the protein of interest precipitates or crystallizes in the fermentation broth or binds at least in part to the particulate matter of the fermentation broth additional treatment steps might be needed to release the protein of interest from the biomass or to solubilize protein of interest crystals and precipitates. WO 00/43502 A1, WO 2008/110498 A1, and WO 2017/097869 A1 describe a method for recovering a protein of interest, which precipitates and/or crystallizes during fermentation, from the fermentation broth. In case the desired protein is comprised in the cells of the fermentation broth release of the protein of interest from the cells might be needed. Release from the cells can be achieved for instance, but not being limited thereto, by cell lysis using techniques well known to the skilled person, e.g., lysozyme treatment, ultrasonic treatment, French press or combinations thereof.

The polypeptide having protease activity may be purified from the fermentation broth by methods known in the art. For example, the polypeptide having protease activity may be isolated from the fermentation broth by conventional procedures including, but not limited to, centrifugation, filtration, extraction, spray-drying, evaporation, or precipitation. The isolated polypeptide may then be further purified by a variety of procedures known in the art including, but not limited to, chromatography (e.g., ion exchange, affinity, hydrophobic, chromatofocussing, and size exclusion), electrophoretic procedures (e.g., preparative isoelectric focusing (IEF), differential solubility (e.g., ammonium sulfate precipitation), or extraction (see, e.g., Protein Purification, J.-C. Janson and Lars Ryden, editors, VCH Publishers, New York, 1989). The purified polypeptide may then be concentrated by procedures known in the art including, but not limited to, ultrafiltration and evaporation, in particular, thin film evaporation.

### Protease Compositions

The purified solution of the polypeptide having protease activity of the present invention may be further processed to form a protease containing composition. Hence, also claimed herein is a composition comprising the polypeptide having protease activity of the present invention and at least one additional component.

Thus, the present invention therefore also refers to a method for making a composition comprising the steps of mixing
i. a polypeptide having protease activity as described herein; and
ii. one or more components described herein.

Further, the present invention also refers to a method for improving protease stability in a composition comprising the steps of mixing
(a) a polypeptide having protease activity as described herein; and
(b) one or more components described herein.

The composition can be a non-complex formulation, e.g., a protease formulation, or a complex formulation, e.g., a detergent composition, as described herein.

In one embodiment of the present invention, the polypeptide having protease activity is formulated as a protease formulation, preferably a concentrated protease formulation. The protease formulation can be eithersolid or liquid. Protein formulations can be obtained by using techniques known in the art. For instance, without being limited thereto, solid enzyme formulations can be obtained by extrusion or granulation. Suitable extrusion and granulation techniques are known in the art and are described for instance in WO 94/19444 A1 and WO 97/43482 A1.

Liquid protease formulations may comprise amounts of protease enzyme in the range of 2% to 40%, 2% to 30%, 2% to 25%, 2% to 12%, or preferably 2-7.5% by weight, all relative to the total weight of the enzyme formulation.

In one embodiment, the protease formulation, in particular the liquid protease formulation, comprises in addition one or more additional compounds selected from the group consisting of solvent, salt, pH regulator, preservative, enzyme stabilizer, and thickening agent. Preferably, the protease formulation is essentially devoid of surfactants, i.e. the protease formulation comprises less than 1% surfactant, preferably less than 0.5% surfactant. The solvent may be water and/or an organic solvent. Aqueous protease formulations of the invention may comprise water in amounts of more than about 30% by weight, more than about 40% by weight, more than about 50% by weight or more than about 60% by weight, all relative to the total weight of the protease formulation. The protease-containing formulations of the invention may comprise an organic solvent in amounts of more than 30%, more than 40%, more than about 50% by weight, more than about 60% by weight, more than about 70% by weight, or more than about 80% by weight, all relative to the total weight of the protease formulation. The organic solvent may be a water-miscible solvent. The organic solvent may be one or more selected from the group consisting of glycerol, propanediol, polypropylene glycol, and polyethylene glycol.

In one embodiment, the protease formulation comprises at least one preservative. Preferably, preservative means substances that are added to a liquid composition for the purpose of preservation, meaning more preferably that compounds known to have preserving features comprised in a liquid composition formed in the production process are excluded from the term preservatives. In one embodiment, the preservative is selected from the group consisting of 2-phenoxyethanol, glutaraldehyde, 2-bromo-2-nitropropane-1 ,3-diol, formic acid in acid form or as its salt, and 4,4'-di-chloro 2-hydroxydiphenylether. Usually, the liquid compositions of the invention comprise at least one preservative in amounts below 10ppm, such as in amounts ranging from 2 ppm to 5% by weight relative to the total weight of the liquid composition. Preferably, the protease formulation is free from preservatives, meaning that preservatives are comprised in amounts less than 1 ppm, preferably 0 ppm.

Preferably, the protease formulation comprises an enzyme stabilizing system. Preferably, the enzyme stabilizing system comprises at least one compound selected from the group consisting of polyols (preferably, 1 ,3-propanediol, ethylene glycol, glycerol, 1,2-propanediol, or sorbitol), inorganic salts (preferably, CaCl₂, MgCl₂, or NaCl), short chain (preferably, C₁-C₃) carboxylic acids or salts thereof (preferably, formic acid, formate (preferably, sodium formate), acetic acid, acetate, or lactate), borate, boric acid, boronic acids (preferably, 4-formyl phenylboronic acid (4-FPBA)), peptide aldehydes (preferably, Benzyloxycarbony-VAL-H (Z-VAL-H or Cbz-VAL-H) or Benzyloxycarbony-GAY-H (Z-GAY-H or Cbz-GAY-H), peptide acetals, and peptide aldehyde hydrosulfite adducts. Preferably, the enzyme stabilizing system comprises a combination of at least two of the compounds selected from the group consisting of salts, polyols, and short chain carboxylic acids and preferably one or more of the compounds selected from the group consisting of borate, boric acid, boronic acids (preferably, 4-formyl phenylboronic acid (4-FPBA)), peptide aldehydes, peptide acetals, and peptide aldehyde hydrosulfite adducts. In a particularly preferred embodiment, the stabilizing system comprises a protease inhibitor, preferably selected from borate, boric acid, boronic acids (preferably, 4-FPBA), peptide aldehydes (preferably, peptide aldehydes like Z-VAL-H or Z-GAY-H), peptide acetals, and peptide aldehyde hydrosulfite adducts, preferably the protease inhibitor is a peptide aldehyde, preferably Z-VAL-H or Z-GAY-H. In one embodiment, the stabilizing system does not comprise a protease inhibitor. Preferably, the composition is boron-free. Preferably, the protease formulation comprises a calcium salt, preferably calcium chloride.

In one embodiment, the protease formulation comprises a protease inhibitor, preferably a peptide aldehyde, in amounts in the range of about 0.04% to 0.8%, 0.04% to 0.7%, 0.04% to 0.6%, 0.04% to 0.5%, 0.04% to 0.4% or 0.04% to 0.3% by weight relative to the total weight of the liquid protease variant formulation. Preferably, the peptide aldehyde is comprised in amounts in the range of about 0.1% to 0.6% by weight, of about 0.1% to 0.5% by weight, of about 0.1% to 0.4%, or of about 0.1% to 0.35% by weight, all relative to the total weight of the liquid protease formulation.

Preferably, the liquid protease formulation comprises or consists of the polypeptide having protease activity of the present invention, a solvent, an enzyme stabilizing system, and optionally a preservative and optionally an additional enzyme different from the protease variant. Preferably, the formulation of the polypeptide having protease activity of the present invention is essentially devoid of surfactants, i.e. the protease formulation comprises less than 1% surfactant, preferably less than 0.5% surfactant.

The present invention therefore also relates to a method for making a protease formulation, preferably a concentrated protease formulation, comprising the steps of mixing
a) a polypeptide having protease activity of the present invention; and
b) one or more components selected from the group consisting of solvent, enzyme stabilizing system, preservative, and an additional enzyme different from the protease.

Further, the present invention relates to a method for improving protease stability in a composition with comprising the steps of mixing
a) a polypeptide having protease activity of the present invention; and
b) one or more components selected from the group consisting of solvent, enzyme stabilizing system, preservative, and an additional enzyme different from the protease.

In one embodiment, the polypeptide having protease activity of the present invention is part of a microorganism (alive, attenuated or inactivated), probiotic, or prebiotic.

### Additional enzymes

In another embodiment, the composition comprising a polypeptide having protease activity of the present invention further comprises one or more additional enzymes different from the protease. Preferably, the additional enzyme is selected from the group consisting of amylases, lipases, proteases other than the polypeptide having protease activity of the present invention, cellulases, mannanases, hemicellulases, phospholipases, esterases, pectinases, lactases, peroxidases, xylanases, cutinases, pectate lyases, keratinases, reductases, oxidases, phenoloxidases, lipoxygenases, ligninases, pullulanases, tannases, pentosanases, malanases, beta-glucanases, arabinosidases, hyaluronidases, chondroitinases, laccases, nucleases, ribonucleases (RNAses), deoxyribonucleases (DNAses), phosphodiesterases, phytases, carbohydrases, galactanases, xanthanases, xyloglucanases, oxidoreductases, perhydrolases, aminopeptidases, asparaginases, carbohydrases, carboxypeptidases, catalases, chitinases, cyclodextrin glycosyltransferases, alpha-galactosidases, beta-galactosidases, glucoamylases, alpha-glucosidases, beta-glucosidases, invertases, transglutaminases, and dispersins, and combinations of at least two of the foregoing types. More preferably, the additional enzyme is selected from the group consisting of amylases, lipases, proteases other than the polypeptide having protease activity of the present invention, cellulases, mannanases, esterases, xylanases, DNAses, dispersins, pectinases, oxidoreductases, and cutinases, and combinations of at least two of the foregoing types. Further preferred, the additional enzyme is selected from the group consisting of amylases, lipases, proteases other than the polypeptide having protease activity of the present invention, cellulases, mannanases, and combinations of at least two of the foregoing types. Most preferably, the additional enzyme is an amylase, preferably, an alpha-amylase.

The composition of the present invention can comprise more than one enzyme of different types, e.g., an amylase and a protease, or more than one enzyme of the same type, e.g., two or more different proteases, or mixtures thereof, e.g., an amylase and two different proteases of which one is the polypeptide having protease activity of the present invention.

### Proteases

Proteases other than the polypeptide having protease activity of the present invention can comprise an amino acid sequence having with increasing preference at least 80%, at least 85%, at least 90%, at least 91%, at least 92%, at least 93%, at least 94%, at least 95%, at least 96%, at least 97%, at least 98%, or at least 99%, but below 100% sequence identity with SEQ ID NO: 45 and comprising amino acid substitutions in one or more of the following positions 3, 4, 9, 15, 27, 33, 36, 57, 68, 77, 87, 95, 96, 97, 98, 99, 100, 101, 102, 103, 104, 106, 118, 120, 123, 128, 129, 130, 131, 154, 160, 167, 170, 194, 199, 205, 206, 217, 218, 222, 224, 232, 235, 236, 245, 252 and 274 (according to the BPN' numbering), which has proteolytic activity. In one embodiment, such a protease is not mutated at positions Asp32, His64 and Ser221 (according to the BPN' numbering). Preferably, the protease used in combination with the polypeptide having protease activity of the present invention comprises an amino acid sequence having with increasing preference at least 80%, at least 85%, at least 90%, at least 91%, at least 92%, at least 93%, at least 94%, at least 95%, at least 96%, at least 97%, at least 98%, or at least 99%, but below 100% sequence identity with SEQ iD NO: 45 and is further characterized by having amino acid glutamic acid (E), or aspartic acid (D), or asparagine (N), or glutamine (Q), or alanine (A), or glycine (G), or serine (S), preferably glutamic acid (E), at position 101 (according to the BPN' numbering) and has proteolytic activity. Mostly preferred is a protease which has at least 80%, but below 100% sequence identity with SEQ ID NO: 45 and is characterized by having amino acid glutamic acid (E) at position 101 (according to the BPN' numbering) and has proteolytic activity. The protease may comprise an amino acid substitution at position 101, such as R101E, alone or in combination with one or more substitutions at positions 3, 4, 9, 15, 27, 33, 36, 57, 68, 77, 87, 95, 96, 97, 98, 99, 100, 102, 103, 104, 106, 118, 120, 123, 128, 129, 130, 131, 154, 160, 167, 170, 194, 199, 205, 206, 217, 218, 222, 224, 232, 235, 236, 245, 252 and/or 274 (according to the BPN' numbering) and has proteolytic activity. In one embodiment, said protease comprises one or more further substitutions: (a) threonine at position 3 (3T), (b) isoleucine at position 4 (4I), (c) alanine, threonine or arginine at position 63 (63A, 63T, or 63R), (d) aspartic acid or glutamic acid at position 156 (156D or 156E), (e) proline at position 194 (194P), (f) methionine at position 199 (199M), (g) isoleucine at position 205 (205I), (h) aspartic acid, glutamic acid or glycine at position 217 (217D, 217E or217G), (i) combinations of two or more amino acids according to (a) to (h), the numbering according to the BPN' numbering. A suitable protease may be at least 80% identical to SEQ ID NO: 45 and is characterized by comprising one amino acid (according to (a)-(h)) or combinations according to (i) together with the amino acid 101E, 101D, 101N, 101Q, 101A, 101G, or 101S (according to the BPN' numbering) and has proteolytic activity. In one embodiment, the protease is at least 80% identical to SEQ ID NO: 45 and is characterized by comprising the mutation (according to the BPN' numbering) R101E, or S3T + V4I + V205I, or S3T + V4I + R101E + V205I or S3T + V4I + V199M + V205I + L217D, and has proteolytic activity. In another embodiment, the protease comprises an amino acid sequence having at least 80% identity to SEQ ID NO: 45 and being further characterized by comprising S3T + V4I + S9R + A 15T + V68A + D99S + R101S + A 1 03S + I104V + N218D (according to the BPN' numbering) and has proteolytic activity. In another embodiment, the protease may have an amino acid sequence being at least 80% identical to SEQ ID NO:45 and being further characterized by comprising R101E, and one or more substitutions selected from the group consisting of S156D, L262E, Q137H, S3T, R45E,D,Q, P55N, T58W,Y,L, Q59D,M,N,T, G61D,R, S87E, G97S, A98D,E,R, S106A,W, N117E, H120V,D,K,N, S125M, P129D, E136Q, S144W, S161T, S163A,G, Y171L, A172S, N185Q, V199M, Y209W, M222Q, N238H, V244T, N261T,D and L262N,Q,D (according to the BPN' numbering), and has proteolytic activity.

In this embodiment, preferably, the protease used in combination with the polypeptide having protease activity comprises an amino acid sequence which is at least 80%, at least 85%, at least 90%, at least 95%, at least 96%, at least 97%, at least 98%, or at least 99%, but less than 100% identical to SEQ ID NO: 45 and the protease comprises compared to SEQ ID NO: 45 the amino acid substitutions R101E and S156D and/or L262E, and optionally at least one further mutation selected from I104T, H120D, Q137H, S141H, R145H and S163G according to the BPN' numbering. In another embodiment, the protease used in combination with the polypeptide having protease activity has at least 60%, preferably at least 80%, at least 85%, at least 90%, at least 95%, at least 96%, at least 97%, at least 98%, or at least 99%, but less than 100% sequence identity to SEQ ID NO: 45 and comprises compared to SEQ ID NO: 45 the amino acid substitution R101D or R101E, preferably R101E, and one or more of the amino acid substitutions selected from the group consisting of S3T, V4I, and V205I, preferably all of the amino acid substitutions S3T, V4I, and V205I, according to the BPN' numbering, and one or more substitutions at positions according to the BPN' numbering selected from the group consisting of 76, 138, 145, 156, 166, 167, 169, 177, 187, 189, 191, 206, 209, 215, 218, and 262, preferably selected from the group consisting of N76D/E/Q, A138Q, R145L/W/Y, S156D/Q, S166G, Y167T, A169G, V177 L, A187 D, F189R, Q191 R, Q206A/L/S/T, Y209K/V/W, A215K/W, N218S/T, and L262D/E/Q

In this embodiment, preferably, the protease used in combination with the polypeptide having protease activity has at least 80%, at least 85%, at least 90%, at least 95%, at least 96%, at least 97%, at least 98%, or at least 99%, but less than 100% sequence identity to SEQ ID NO: 45 and comprises compared to SEQ ID NO: 45 the amino acid substitution R101D or R101E, preferably R101E, and one or more of the amino acid substitutions selected from the group consisting of S3T, V4I, and V205I, preferably all of the amino acid substitutions S3T, V4I, and V205I, according to the BPN' numbering, and one or more substitutions at positions according to the BPN' numbering selected from the group consisting of 156, 166, 187, 189, 191, 206, 209, 215 and 262, preferably selected from the group consisting of S156D, S166G, A187 D, F189R, Q191R, Q206L, Y209W, A215K und L262E.

In this embodiment, particularly preferred, the protease used in combination with the polypeptide having protease activity has at least 80%, at least 85%, at least 90%, at least 95%, at least 96%, at least 97%, at least 98%, or at least 99%, but less than 100% sequence identity to SEQ ID NO: 45 and comprises compared to SEQ ID NO: 45 according to the BPN' numbering the amino acid substitutions
(i) S3T-V4I-R101E-V199I-Q206L-Y209W;
(ii) S3T-V4I-R101E-V199I-N218S;
(iii) S3T-V4I-R101E-V199I-N76D;
(iv) S3T-V4I-R101E-V199I-S156D-L262E;
(v) S3T-V4I-R101E-V199I-Q206L-Y209W-S156D-L262E;
(vi) S3T-V4I-R101E-V199I-N760-Q206L-Y209W;
(vii) S3T-V4I-R101E-V199I-N760-S1560-Q206L-Y209W-L262E;
(viii) S3T-V4I-R101E-V199I-N76D-N218S;
(ix) S3T-V4I-R101E-V199I-N760-S1560-Y209W-L262E;
(x) S3T-V4I-R101E-V199I-N76D-Y209W;
(xi) S3T-V4I-R101E-V199I-N76D-S156D-Q206L-L262E;
(xii) S3T-V4I-R101E-V199I-N76D-Q206L;
(xiii) S3T-V4I-R101E-V199I-S1560-Q206L-Y209W;
(xiv) S3T-V4I-R101E-V199I-Q206L-Y209W-L262E;
(xv) S3T-V4I-R101E-V199I-A138Q-R145W-Y167 T-Q206L;
(xvi) S3T-V4I-R101E-V199I-N76D-R145Y-A215W-N218S-L262E;
(xvii)S3T-V4I-R101E-V199I-A138Q-S156D-V177 L-Q206L;
(xviii) S3T-V4I-R101E-V199I-Q206L-Y209W-A215K-S156D-L262E;
(xix) S3T-V4I-R101E-V199I-S156D-S166G-Q191R-Q206L-Y209W-L262E; or
(xx) S3T-V4I-R101E-V199I-S156D-A187D-F189R-Q206L-Y209W-L262E.
Commercially available protease enzymes include but are not limited to those sold under the trade names Progress^{®} Uno, Alcalase^{®}, Blaze^{®}, Duralase^{™}, Durazym^{™}, Relase^{®}, Relase^{®} Ultra, Savinase^{®}, Savinase^{®} Ultra, Primase^{®}, Polarzyme^{®}, Kannase^{®}, Liquanase^{®}, Liquanase^{®} Ultra, Ovozyme^{®}, Coronase^{®}, Coronase^{®} Ultra, Neutrase^{®}, Everlase^{®} and Esperase^{®} (Novozymes A/S) and those sold under the tradename Maxatase^{®}, Maxacai^{®}, Maxapem^{®}, Purafect^{®}, Purafect^{®} Prime, Purafect MA^{®}, Purafect Ox^{®}, Purafect OxP^{®}, Puramax^{®}, Properase^{®}, FN2^{®}, FN3^{®}, FN4^{®}, Excellase^{®}, Eraser^{®}, Ultimase^{®}, Opticlean^{®}, Effectenz^{®}, Preferenz^{®} (P100, P200, P300 and P400 from IFF) and Optimase^{®} (Danisco/DuPont), Axapem^{™} (Gist-Brocases N.V.), KAP (Bacillus alkalophilus subtilisin) from Kao Corporation and Lavergy Pro (BASF).

### Lipases

"Lipases", "lipolytic enzyme", "lipid esterase", all refer to an enzyme of EC class 3.1.1 ("carboxylic ester hydrolase"). Lipase means active protein having lipase activity (or lipolytic activity; triacylglycerol lipase, EC 3.1.1.3), cutinase activity (EC 3.1.1.74; enzymes having cutinase activity may be called cutinase herein), sterol esterase activity (EC 3.1.1.13) and/or wax-ester hydrolase activity (EC 3.1.1.50). Lipases include those of bacterial or fungal origin.

The methods for determining lipolytic activity are well-known in the literature (see e.g. Gupta et al. (2003), Biotechnol. Appl. Biochem. 37, p. 63-71). For instance, the lipase activity may be measured by ester bond hydrolysis in the substrate para-nitrophenyl palmitate (pNP-Palmitate, C:16) and releases pNP which is yellow and can be detected at 405 nm.

In one aspect of the invention, a suitable lipase (component (b)) is selected from the following: lipases from Humicola (synonym Thermomyces), e.g. from H. lanuginosa (T. lanuginosus) as described in EP 258068, EP 305216, WO 92/05249 and WO 2009/109500 or from H. insolens as described in WO 96/13580; lipases derived from Rhizomucor miehei as described in WO 92/05249; lipase from strains of Pseudomonas (some of these now renamed to Burkholderia), e.g. from P. alcaligenes or P. pseudoalcaligenes (EP 218272, WO 94/25578, WO 95/30744, WO 95/35381, WO 96/00292), P. cepacia (EP 331376), P. stutzeri (GB 1372034), P. fluorescens, Pseudomonas sp. strain SD705 (WO 95/06720 and WO 96/27002), P. wisconsinensis (WO 96/12012), Pseudomonas mendocina (WO 95/14783), P. glumae (WO 95/35381, WO 96/00292); lipase from Streptomyces griseus (WO 2011/150157) and S. pristinaespiralis (WO 2012/137147), GDSL-type Streptomyces lipases (WO 2010/065455); lipase from Thermobifida fusca as disclosed in WO 2011/084412; lipase from Geobacillus stearothermophilus as disclosed in WO 2011/084417; Bacillus lipases, e.g. as disclosed in WO 00/60063, lipases from B. subtilis as disclosed in Dartois et al. (1992), Biochemica et Biophysica Acta, 1131, 253-360 or WO 2011/084599, B. stearothermophilus (WO 2011/084417) or B. pumilus (WO 91/16422); lipase from Candida antarctica as disclosed in WO 94/01541; cutinase from Pseudomonas mendocina (US 5389536, WO 88/09367); cutinase from Magnaporthe grisea (WO 2010/107560); cutinase from Fusarum solani pisi as disclosed in WO 90/09446, WO 00/34450 and WO 01/92502; ester hydrolase from Thermogutta terrifontis (WO2020104157) and cutinase from Humicola lanuginosa as disclosed in WO 00/34450 and WO 01/92502.

Such suitable lipase variants are e.g. those which are developed by methods as disclosed in WO 95/22615, WO 97/04079, WO 97/07202, WO 00/60063, WO 2007/087508, EP 407225 and EP 260105.

Suitable lipases include also those, which are variants of the above-described lipases which have lipolytic activity. In one embodiment lipase variants include variants with at least 40 to 100% identity when compared to the full-length polypeptide sequence of the parent enzyme as disclosed above. In one embodiment lipase variants having lipolytic activity are at least 70%, at least 75%, at least 80%, at least 85%, at least 90%, at least 91%, at least 92%, at least 93%, at least 94%, at least 95%, at least 96%, at least 97%, at least 98% or at least 99% identical to the full-length polypeptide sequence of the parent enzyme as disclosed above.

In another embodiment, the invention relates to lipase variants comprising conservative mutations not pertaining the functional domain of the respective lipase.

In one embodiment, lipase is selected from fungal triacylglycerol lipase (EC class 3.1.1.3). Fungal triacylglycerol lipase may be selected from lipases of Thermomyces lanuginosa. In one embodiment, at least one Thermomyces lanuginosa lipase is selected from triacylglycerol lipase according to amino acids 1-269 of SEQ ID NO: 2 of US5869438 and variants thereof having lipolytic activity.

Thermomyces lanuginosa lipase may be selected from variants having lipolytic activity which are at least 90%, at least 91%, at least 92%, at least 93%, at least 94%, at least 95%, at least 96%, at least 97%, at least 98% at least 99%, or 100% identical when compared to the full-length polypeptide sequence of amino acids 1-269 of SEQ ID NO: 2 of US5869438.

Thermomyces lanuginosa lipase may be selected from variants having lipolytic activity comprising at least the following amino acid substitutions when compared to amino acids 1-269 of SEQ ID NO: 2 of US5869438: T231R and N233R. Said lipase variants may further comprise one or more of the following amino acid exchanges when compared to amino acids 1-269 of SEQ ID NO: 2 of US5869438: Q4V, V60S, A150G, L227G, P256K.

Thermomyces lanuginosa lipase may be selected from variants having lipolytic activity comprising at least the amino acid substitutions T231R, N233R, Q4V, V60S, A150G, L227G, P256K within the polypeptide sequence of amino acids 1-269 of SEQ ID NO: 2 of US5869438 and are at least 95%, at least 96%, or at least 97% identical when compared to the full-length polypeptide sequence of amino acids 1-269 of SEQ ID NO: 2 of US5869438.

Thermomyces lanuginosa lipase may be selected from variants having lipolytic activity comprising the amino acid substitutions T231R and N233R within amino acids 1-269 of SEQ ID NO: 2 of US5869438 and are at least 95%, at least 96%, at least 97%, at least 98%, or at least 99% identical when compared to the full-length polypeptide sequence of amino acids 1-269 of SEQ ID NO: 2 of US5869438.

Thermomyces lanuginosa lipase may be a variant of amino acids 1-269 of SEQ ID NO: 2 of US5869438 having lipolytic activity, wherein the variant of amino acids 1-269 of SEQ ID NO: 2 of US5869438 is characterized in containing the amino acid substitutions T231R and N233R.

Thermomyces lanuginosa lipase may be selected from variants having lipolytic activity preferably comprising at least one, preferably more than one, more preferably all of the following substitutions N11K, A18K, G23K, K24A, V77I, D130A, V154I, V187T, T189Q within the polypeptide sequence of amino acids 1-269 of SEQ ID NO: 1 of WO2015/010009 and are at least 95%, at least 96%, or at least 97% identical when compared to the full length polypeptide sequence of amino acids 1-269 of SEQ ID NO: 1 of WO2015/010009.

Commercially available lipase enzymes include but are not limited to those sold under the trade names Lipolase^{™}, Lipex^{™}, Lipolex^{™} and Lipoclean^{™} (Novozymes A/S), Lumafast (originally from Genencor), Preferenz L (IFF) and Lipomax (Gist-Brocades/ now DSM).

### Amylases

"Amylases" according to the invention (alpha and/or beta) include those of bacterial or fungal origin (EC 3.2.1.1 and 3.2.1.2, respectively). Preferably, amylases are selected from the group of alpha-amylases (EC 3.2.1.1).

Amylases according to the invention have "amylolytic activity" or "amylase activity" involving (endo)hydrolysis of glucosidic linkages in polysaccharides. alpha-amylase activity may be determined by assays for measurement of alpha-amylase activity which are known to those skilled in the art. For example, alpha-amylase activity can be determined by a method employing Phadebas tablets as substrate (Phadebas Amylase Test, supplied by Magle Life Science). Starch is hydrolyzed by the alpha-amylase giving soluble blue fragments. The absorbance of the resulting blue solution, measured spectrophotometrically at 620 nm, is a function of the alpha-amylase activity. The measured absorbance is directly proportional to the specific activity (activity/mg of pure alpha-amylase protein) of the alpha-amylase in question under the given set of conditions.

Alpha-amylase activity can also be determined by a method employing the Ethyliden-4-nitrophenyl-alpha-D-malto-heptaosid (EPS). D-maltoheptaoside is a blocked oligosaccharide which can be cleaved by an endo-amylase. Following the cleavage, the alpha-glucosidase included in the kit to digest the substrate to liberate a free PNP molecule which has a yellow color and thus can be measured by visible spectophotometry at 405nm. Kits containing EPS substrate and alpha-glucosidase is manufactured by Roche Costum Biotech (cat. No. 10880078103). The slope of the time dependent absorption-curve is directly proportional to the specific activity (activity per mg enzyme) of the alpha-amylase in question under the given set of conditions.

Amylolytic activity may be provided in units per gram enzyme. For example, 1 unit alpha-amylase may liberate 1 mg of maltose from starch in 3 min at pH 6.9 at 20°C.

Amylases maybe from Bacillus licheniformis having SEQ ID NO:2 as described in WO 95/10603 and variants with at least 95% sequence identity thereto. Suitable variants are described in WO 95/10603 comprising one or more substitutions in the following positions: 15, 23, 105, 106, 124, 128, 133, 154, 156, 178, 179, 181, 188, 190, 197, 201, 202, 207, 208, 209, 211, 243, 264, 304, 305, 391, 408, and 444 which have amylolytic activity. Variants are described in WO 94/02597, WO 94/018314, WO 97/043424 and SEQ ID NO:4 of WO 99/019467.

Amylases may also be from B. stearothermophilus having SEQ ID NO:6 as disclosed in WO 02/10355 or an amylase with optionally having a C-terminal truncation over the wildtype sequence. Suitable variants of SEQ ID NO:6 include those comprising a deletion in positions 179 and/or 181 and/or 182 and/or a substitution in position 193.

Amylases may also be from Bacillus sp.707 having SEQ ID NO:6 as disclosed in WO 99/19467 and variants at least 95% thereto. Preferred variants of SEQ NO: 6 are those having a substitution, a deletion or an insertion in one or more of the following positions: R181, G182, H183, G184, N195, I206, E212, E216 and K269.

Amylases may also be from Bacillus halmapalus having SEQ ID NO:2 or SEQ ID NO:7 as described in WO 96/23872, also described herein as SP-722. Preferred variants are described in WO 97/3296, WO 99/194671 and WO 2013/001078.

Amylases may also be from Bacillus sp. DSM 12649 having SEQ ID NO:4 as disclosed in WO 00/22103 and variants at least 95% thereto.

Amylases may also be from Bacillus sp. A 7-7 (DSM 12368) having an amino acid sequence at least 95% identical to SEQ ID NO:2, in particular over the region of the amino acids 32 to 516 according to SEQ ID NO:2, as disclosed in WO 02/10356.

Amylases may also be from Bacillus strain TS-23 having SEQ ID NO:2 as disclosed in WO 2009/061380 and variants thereof.

Amylases may also be from Cytophaga sp. having SEQ ID NO:1 as disclosed in WO 2013/184577 and variants at least 95% thereto.

Amylases may also be from Bacillus megaterium DSM 90 having SEQ ID NO:1 as disclosed in WO 2010/104675 and variants at least 95% thereto.

Amylases may also be from Bacillus sp. comprising amino acids 1 to 485 of SEQ ID NO:2 as described in WO 00/60060 and variants at least 95% thereto.

Amylases may also be from Bacillus amyloliquefaciens or variants thereof, preferably selected from amylases according to SEQ ID NO: 3 as described in WO 2016/092009.

Amylases may have SEQ ID NO:12 as described in WO 2006/002643 or amylase variants thereof comprising the substitutions Y295F and M202LITV within said SEQ ID NO:12.

Amylases may have SEQ ID NO:6 as described in WO 2011/098531 or amylase variants comprising a substitution at one or more positions selected from the group consisting of 193 [G,A,S,T or M], 195 [F,W,Y,L,I or V], 197 [F,W,Y,L,I or V], 198 [Q or N], 200 [F,W,Y,L,I or V], 203 [F,W,Y,L,I or V], 206 [F,W,Y,N,L,I,V,H,Q,D or E], 210 [F,W,Y,L,I or V], 212 [F,W,Y,L,I or V], 213 [G,A,S,T or M] and 243 [F,W,Y,L,I or V] within said SEQ ID NO:6.

Amylases may have SEQ ID NO:1 as described in WO 2013/001078 or amylase variants comprising an alteration at two or more (several) positions corresponding to positions G304, W140, W189, D134, E260, F262, W284, W347, W439, W469, G476, and G477 within said SEQ ID NO:1.

Amylases may have SEQ ID NO:2 as described in WO 2013/001087 or amylase variants comprising a deletion of positions 181+182, or 182+183, or 183+184, within said SEQ ID NO:2, optionally comprising one or two or more modifications in any of positions corresponding to W140, W159, W167, Q169, W189, E194, N260, F262, W284, F289, G304, G305, R320, W347, W439, W469, G476 and G477 within said SEQ ID NO:2.

Amylases may be hybrid alpha-amylases from above mentioned amylases as for example as described in WO 2006/066594.

Hybrid amylases may be according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity.

Hybrid amylases may be according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 and having amylolytic activity;

Hybrid amylases may be according to WO 2021/032881 comprising an A and B domain originating from the alpha amylase originating from Bacillus sp. A 7-7 (DSM 12368) and a C domain originating from the alpha-amylase from Bacillus cereus; preferably, the A and B domain are at least 75% identical to the amino acid sequence of SEQ ID NO: 42 and a C domain is at least 75% identical to the amino acid sequence of SEQ ID NO: 44 - both sequences as disclosed in WO 2021/032881; more preferably, the hybrid amylase is at least 80% identical to SEQ ID NO:54 as disclosed in WO 2021/032881.

Amylases might further be variants as disclosed in WO 2022/175435, WO 2024/033136, or WO 2024/033135, preferably as claimed in WO 2024/033136 or WO 2024/033135.

Suitable amylases include also those, which are variants of the above-described amylases which have amylolytic activity. In one embodiment amylase variants include variants with at least 40 to 100% identity when compared to the full-length polypeptide sequence of the parent enzyme as disclosed above. In one embodiment amylase variants having amylolytic activity are at least 70%, at least 75%, at least 80%, at least 85%, at least 90%, at least 91%, at least 92%, at least 93%, at least 94%, at least 95%, at least 96%, at least 97%, at least 98% or at least 99% identical to the full-length polypeptide sequence of the parent enzyme as disclosed above.

In another embodiment, the invention relates to amylase variants comprising conservative mutations not pertaining the functional domain of the respective amylase.

In one embodiment, at least one amylase is selected from commercially available amylases which include but are not limited to products sold under the trade names Duramyl^{™}, Termamyl^{™}, Fungamyl^{™}, Stainzyme^{™}, Stainzyme Plus^{™}, Natalase^{™}, Liquozyme X and BAN^{™}, Amplify^{™}, Amplify Prime^{™} (from Novozymes A/S), and Rapidase^{™}, Purastar^{™}, PoweraseTM, Effectenz^{™} (M100 from DuPont), Preferenz^{™} (S1000, S110 and S210; from DuPont), PrimaGreen^{™} (ALL; DuPont), Optisize^{™} (DuPont).

### Mannanases

The mannanase may be selected from the group of mannan degrading enzyme. At least one mannan degrading enzyme may be selected from β-mannosidase (EC 3.2.1.25), endo-1,4-β-mannosidase (EC 3.2.1.78), and 1,4-β-man-nobiosidase (EC 3.2.1.100). Preferably, at least one mannan degrading enzyme is selected from the group of endo-1,4-β-mannosidase (EC 3.2.1.78), a group of enzymes which may be called endo-β-1,4-D-mannanase, β-mannanase, or mannanase herein.

A polypeptide having mannan degrading activity or mannanase activity may be tested for according to standard test procedures known in the art, such as by applying a solution to be tested to 4 mm diameter holes punched out in agar plates containing 0.2% AZCL galactomannan (carob), i.e. substrate for the assay of endo-1 ,4-beta-D-mannanase available as CatNo. I-AZGMA from the company Megazyme (Megazyme's Internet address: http://www. megazyme. com/Purchase/index. html).

Mannan degrading activity may be tested in a liquid assay using carob galactomannan dyed with Remazol Brilliant Bue as described in McCleary, B. V. (1978). Carbohydrate Research, 67(1), 213-221. Another method for testing mannan degrading activity uses detection of reducing sugars when incubated with substrate such as guar gum or locust bean gut - for reference see Miller, G. L. Use of Dinitrosalicylic Acid Reagent for Determination of Reducing Sugars. Analytical Chemistry 1959; 31: 426-428.

The mannanase may be selected from alkaline mannanase of Family 5 or 26 (i.e. GH5 or GH26). The term "alkaline mannanase" is meant to encompass mannanases having an enzymatic activity of at least 40% of its maximum activity at a given pH ranging from 7 to 12, preferably 7.5 to 10.5.

The mannanase may be selected from mannanases originating from Bacillus organisms, such as described in JP-0304706 [beta-mannanase from Bacillus sp.], JP-63056289 [alkaline, thermostable beta-mannanase], JP-63036774 [Bacillus microorganism FERM P-8856 producing beta-mannanase and beta-mannosidase at an alkaline pH], JP-08051975 [alkaline beta-mannanases from alkalophilic Bacillus sp. AM-001], WO 97/11164 [mannanase from Bacillus amyloliquefaciens], WO 91/18974 [mannanase active at an extreme pH and temperature], WO 97/11164 [mannanase from Bacillus amyloliquefaciens], WO 2014/100018 [endo-(3-mannanase1 cloned from a Bacillus circulans or Bacillus lentus strain CMG1240 (Bleman1; see US 5,476,775)]. Suitable mannanases are described in WO 99/064619].

The mannanase may be selected from mannanases originating from Trichoderma organisms, such as disclosed in WO 93/24622.

Mannanases might further be variants as disclosed in WO 2021160818, in particular mannanase variants having at least 75% identity to SEQ ID NO: 2 or SEQ ID NO 3 or SEQ ID NO: 4 of WO 2021160818, comprising two or more acid substitutions selected from A31V, Q89V, N96D, A119Y/H/T, E264Q/V, W289F/M/H, N312F/Y, T348S/R/N/M/G, E349T/S/D/G, S352N/G, D379V, wherein the numbering is according to SEQ ID NO: 2 of WO 2021160818.

Suitable mannanases include also those, which are variants of the above described mannanases which have mannan degrading activity. In one embodiment mannanase variants include variants with at least 40 to 100% identity when compared to the full-length polypeptide sequence of the parent enzyme as disclosed above. In one embodiment mannanase variants having mannan degrading activity are at least 70%, at least 75%, at least 80%, at least 85%, at least 90%, at least 91%, at least 92%, at least 93%, at least 94%, at least 95%, at least 96%, at least 97%, at least 98% or at least 99% identical to the full-length polypeptide sequence of the parent enzyme as disclosed above. In another embodiment, the invention relates to mannanase variants comprising conservative mutations not pertaining the functional domain of the respective mannanase.

At least one mannanase may be selected from a commercially available mannanase such as Mannaway^{®} (Novozymes A/S), Preferenz^{®} (M100, M200) (DuPont), or Lavergy MAce (BASF).

### Cellulases

At least one enzyme comprised in the composition of the invention may be selected from the group of cellulases. Cellulases according to the invention include those of bacterial or fungal origin.

At least one cellulase comprised in the composition of the invention may be selected from cellobiohydrolase (1,4-P-D-glucan cellobiohydrolase, EC 3.2.1.91), endo-ss-1,4-glucanase (EC 3.2.1.4), ss-glucosidase (EC 3.2.1.21), and licheninase (3.2.1.73). Endoglucanases of EC class 3.2.1.4 may be named endoglucanase, endo-1,4-ss-D-glucan 4-glucano hydrolase, endo-1,4-beta-glucanase, carboxymethyl cellulase, and beta-1,4-glucanase.

Endoglucanases may be classified by amino acid sequence similarities (Henrissat, B. Accessed at UniProt 10/26/2011) under family 5 containing more than 20 endoglucanases of EC 3.2.1.4. Reference is also made to T.-M. Enveri, "Microbial Cellulases" in W.M. Fogarty, Microbial Enzymes and Biotechnology, Applied Science Publishers, p. 183-224 (1983); Methods in Enzymology, (1988) Vol. 160, p. 200-391 (edited by Wood, W.A. and Kellogg, S.T.); Béguin, P., "Molecular Biology of Cellulose Degradation", Annu. Rev. Microbiol. (1990), Vol. 44, pp. 219248; Begun, P. and Aubert, J-P., "The biological degradation of cellulose", FEMS Microbiology Reviews 13 (1994) p.25-58; Henrissat, B., "Cellulases and their interaction with cellulose", Cellulose (1994), Vol. 1, pp. 169-196.

Preferably, at least one cellulase comprised in the composition of the invention is selected of the glycosyl hydrolase family 7 (GH7, pfam00840), preferably selected from endoglucanases (EC 3.2.1.4).

"Cellulases", "cellulase enzymes" or "cellulolytic enzymes" according to the invention are enzymes involved in hydrolysis of cellulose, i.e having "cellulolytic activity" or "cellulase activity". Preferably, an alkaline cellulase is according to the invention, wherein "alkaline cellulase" is meant to encompass cellulases having enzymatic activity at a given pH ranging from 7 to 12, preferably 7.5 to 10.5.

Assays for measurement of "cellulase activity" or "cellulolytic activity" are known to those skilled in the art. For example, cellulolytic activity may be determined by virtue of the fact that cellulase hydrolyses carboxymethyl cellulose to reducing carbohydrates, the reducing ability of which is determined colorimetrically by means of the ferricyanide reaction, according to Hoffman, W. S., J. Biol. Chem. 120, 51 (1937).

Cellulolytic activity may be provided in units per gram enzyme. For example, 1 unit may liberate 1 pmole of glucose from cellulose in one hour at pH 5.0 at 37°C (2 hour incubation time).

In one embodiment, at least one cellulase comprised in the composition of the invention is selected from cellulases comprising a cellulose binding domain. In one embodiment, at least one cellulase is selected from cellulases comprising a catalytic domain only, meaning that the cellulase lacks cellulose binding domain.

In one embodiment, the composition of the invention comprises at least one endoglucanases of EC class 3.2.1.4 is originating from

### Bacillus, such as Bacillus sp. CBS 670.93 and CBS 669.93

### Melanocarpus, such as Melanocarpus albomyces as disclosed in WO 97/14804

### Clostridium, e.g. Clostridium thermocellum

Humicola, such as Humicola insolens (DSM1800) as disclosed in EP 0495257, EP 0531315, EP 0531372, US 4435307, US 5648263, US 5776757, WO 89/09259, WO 91/17244, WO 94/07998 (sequence displayed in figure 1 43kd human variants thereof), WO 95/24471, WO 96/11262 and WO 98/12307.

Fusarium, such as Fusarium oxysporum e.g. strain J79 (DSM2672) as disclosed in EP 0495257, EP 0531315, EP 0531372, US 5648263, US 5776757, WO 89/09259, WO 91/17244, WO 95/24471 and WO 96/11262

Thielavia, such as Thielavia terrestris or Myceliophthora thermophila strain CBS 11765 as disclosed in EP 0531315, US 5648263, US 5776757, WO 89/09259, WO 91/17244, WO 95/24471, WO 96/11262, WO 96/29397 (SEQ ID NO: 9 and variants thereof), and WO 98/12307.

Trichoderma, such as Trichoderma reesei, Trichoderma longibrachiatum or Trichoderma harzianum as disclosed in EP 1305432, EP 1240525, WO 92/06165, WO 94/21801, WO 94/26880, WO 95/02043, WO 95/24471 and WO 02/099091.

### Aspergillus, such as Aspergillus aculeatus as disclosed in WO 93/17244

Erwinia, such as Erwinia chrysanthermi as described by M. H. Boyer et. al. in European Journal of Biochemistry, vol. 162, page 311-316 (1987).

Acremonium such as Acremonium sp., Acremonium persicinum, Acremonium acremonium, Acremonium brachypenium, Acremonium dichromosporum, Acremonium obclavatum, Acremonium pinkertoniae, Acremonium roseogriseum, Acremonium incoloratum, and Acremonium furatum as disclosed in WO 96/11262 and WO 96/29397 (SEQ ID NO: 5 and variants thereof).

Cellvibrio such as Cellvibrio mixtus DSM 11683, Cellvibrio mixtus DSM 11684, Cellvibrio mixtus DSM 11685, Cellvibrio mixtus ACM 2601, Cellvibrio mixtus DSM 1523, and Cellvibrio gilvus DSM 11686, as disclosed in WO 98/08940. Cephalosporium, such as Cephalosporium sp. RYM-202 as disclosed in WO 96/11262.

Suitable cellulases also include those, which are variants of the above described cellulases which have cellulolytic activity. In one embodiment cellulase variants include variants with at least 40 to 100% identity when compared to the full-length polypeptide sequence of the parent enzyme as disclosed above. In one embodiment cellulase variants having cellulolytic activity are at least 70%, at least 75%, at least 80%, at least 85%, at least 90%, at least 91%, at least 92%, at least 93%, at least 94%, at least 95%, at least 96%, at least 97%, at least 98% or at least 99% identical to the full-length polypeptide sequence of the parent enzyme as disclosed above.

In another embodiment, the invention relates to cellulase variants comprising conservative mutations not pertaining the functional domain of the respective cellulase.

In one embodiment, the composition of the invention comprises a Humicola insolens DSM 1800 cellulase complex having endoglucanase, cellobiohydrolase and beta-glucosidase activity.

The cellulase may be a Humicola insolens DSM 1800 endoglucanase (EC 3.2.1.4), preferably having the polypeptide sequence according to position 21 -435 of SEQ ID NO:2 as disclosed in WO 2018/224544 or variants at least 95% identical thereto.

The cellulase may be a Humicola insolens endoglucanase (EC 3.2.1.4) having 43kD, preferably according to the polypeptide sequence as disclosed in Figure 1a of WO 94/07998 ("43kDhum") or variants thereof which are preferably at least 90% identical thereto, preferably those disclosed in WO 94/07998.

The cellulase may be a Bacillus sp. cellulase (EC 3.2.1.4) selected from a polypeptide at least 80% similar and/or identical to the amino acid sequence of position 1 to position 773 of SEQ ID NO: 2 of WO 2004/053039 or a catalytically active fragment thereof. In one embodiment, the cellulase is a mature polypeptide which is at least 95% identical to SEQ ID NO:1 of WO 2018/224544.

The cellulase may be a Thielavia terrestris cellulase (EC 3.2.1.4) having a polypeptide at least 80% similar and/or identical to the amino acid sequence of position 1 to position 299 of SEQ ID NO: 4 of WO 2004/053039 or a catalytically active fragment thereof). In one embodiment, the cellulase is a mature polypeptide which is at least 95% identical to SEQ ID NO:4 of WO 2018/224544.

The cellulase may be a mature Sordaria fimicola cellulase, preferably having a polypeptide sequence according to SEQ iD NO:5 of WO 2018/224544 or variants at least 95% identical thereto.

In one embodiment the beta-glucanase may be selected from licheninases acting on lichenin and cereal beta-D-glucans, but not on beta-D-glucans containing only 1,3- or 1,4-bonds (EC 3.2.1.73). In one embodiment, the composition of the invention comprises at least one licheninase which is originating from Bacillus, such as Bacillus amyloliquefaciens, Bacillus subtilis, Bacillus akibai, Bacillius agaradhaerens, Bacillus mojavensis, Bacillus sp. 62449 as described in WO2017097861.

At least one cellulase may be selected from Renozyme^{®}, Celluzyme^{®}, Celluclean^{®}, Endolase^{®} and Carezyme^{®} (Novozymes A/S), Clazinase^{™}, and Puradax HA^{™} (Genencor Int. Inc.), KAC-500(B)^{™} (Kao Corporation), Revitalenz 200 (IFF), and Lavergy C Bright (BASF).

### DNAses/RNAses

At least one enzyme may be selected from the group of DNA or RNA degrading enzymes. Said enzymes usually catalyzes the hydrolytic cleavage of phosphodiester linkages in DNA or RNA. The DNAses are classified e.g. in E.C. 3.1.11, E.C. 3.1.12, E.C. 3.1.15, E.C. 3.1.16, E.C. 3.1.21, E.C 3.1.22, E.C 3.1.23, E.C 3.1.24 and E.C.3.1.25 as well as EC 3.1.21.X, where X=1, 2, 3, 4, 5, 6, 7, 8 or 9.

DNAse activity may be determined on DNAse Test Agar with Methyl Green (BD, Franklin Lakes, NJ, USA), which should be prepared according to the manual from supplier. Briefly, 21 g of agar is dissolved in 500 ml water and then autoclaved for 15 min at 121°C. Autoclaved agar is temperated 10 to 48°C in water bath, and 20 ml of agar is to be poured into petridishes with and allowed to solidify by incubation o/n at room temperature. On solidified agar plates, 5 µl of enzyme solution is added and DNAse activity is observed as colorless zones around the spotted enzyme solutions.

DNAse activity may be determined by using the DNAseAlert^{™} Kit (11-02-01-04, IDT Intergrated DNA Technologies) according to the supplier's manual. Briefly, 95µl DNase sample is mixed with 5µl substrate in a microtiter plate, and fluorescence is immediately measured using e.g. a Clariostar microtiter reader from BMG Labtech (536 nm excitation, 556 nm emission).

At least one DNAse comprised in the composition of the invention may be selected from DNAses originating from Bacillus such as from Bacillus cibi, Bacillus horikoshii, Bacillus horneckiae, Bacillus idriensis, Bacillus algicola, Bacillus vietnamensis, Bacillus hwajinpoensis, Paenibacillus mucilanginosus, Bacillus indicus, Bacillus luciferensis, Bacillus marisflavi; and variants thereof. In one embodiment, at least one DNAse comprised in the composition of the invention is selected from polypeptides 80% identical to SEQ ID NO: 1 of WO 2019/081724. Said polypeptide may comprise one or more substitutions at positions selected from T1, G4, S7, K8, S9, S13, N16, T22, S25, S27, D32, L33, S39, G41, S42, D45, Q48, S57, S59, N61, T65, S66, V76, F78, P91, S101, S106, Q109, A112, S116, T127, S130, T138, Q140, S144, A147, C148, W154, T157, Y159, G162, S167, Q174, G175, L177, S179, and C180- all as disclosed in WO 2019/081724 and WO 2019/081721.

At least one DNAse comprised in the composition of the invention may be selected from DNAses originating from fungal source such as Aspergillus, for example from Aspergillus oryzae. In one embodiment, at least one DNAse comprised in the composition of the invention is selected from the polypeptide as given in SEQ ID NO: 2 of WO 2015/155350. Said polypeptide may comprise truncations as for example given in SEQ ID NO: 9 of WO 2015/155350. In preferred embodiment a truncation as given in SEQ ID NO: 8 of WO 2015/155350 or polypeptides being 80% to SEQ ID NO: 8 of WO 2015/155350 identical are present.

The composition of the invention may comprise DNAse variants having DNA degrading activity which are at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% identical when compared to the full-length polypeptide sequences of the corresponding parent enzyme as disclosed above.

In another embodiment, the invention relates to DNAse variants comprising conservative mutations not pertaining the functional domain of the respective DNAse.

At least one DNase may be selected from a commercially available DNase such as Pristine 100T^{®} (Novozymes A/S).

### Other enzymes

### Acyltransferases/perhydrolases

At least one enzyme may be selected from acyltransferases (E.C 2.3.1) or perhydrolases. Perhydrolases catalyze perhydrolysis reaction that results in the production of a peracid from a carboxylic acid ester (acyl) substrate in the presence of a source of peroxygen (e.g., hydrogen peroxide). While many enzymes perform this reaction at low levels, perhydrolases exhibit a high perhydrolysis:hydrolysis ratio, often greater than 1. Suitable perhydrolases may be of plant, bacterial or fungal origin.

Examples of useful perhydrolases include acyltransferases with homology to *Candida antarctica* lipase A (WO 2010/111143) and naturally occurring *Mycobacterium* perhydrolase enzymes, or variants thereof- e.g. a variant of *Mycobacterium smegmatis* as described in WO 2005/056782, WO 2008/063400, US 2008145353, and US 2007167344; perhydrolases from the CE7 family (WO 2009/67279), and variants of the *M*. *smegmatis* perhydrolase in particular the S54V variant (WO 2010/100028).

### Peroxidases

In order to supply hydrogen peroxide for bleaching purposes in detergent compositions, oxidoreductase enzymes my be employed. The catalyzed reaction is the transfer of electrons from the organic substrate, for the glucose oxidase, for example, from the glucose, to the oxygen as the electron acceptor with the formation of the desired hydrogen peroxide.

"Peroxidase activity" may be measured by the ABTS method as described in Childs et al. 1975 (Biochemical J, 145, p. 93-103) and commercial kits are available from different suppliers. Other measuring methods are known to those known in the art.

The hydrogen peroxide-producing oxidoreductases herein concern enzymes that produce hydrogen peroxide, using oxygen as an electron acceptor. In this regard, particularly preferred oxidoreductases include those of the EC classes E.C. 1.1.3 (CH-OH as the electron donor), E.C. 1.2.3 (aldehyde or oxo groups as the electron donor), E.C. 1.4.3 (CH-NH₂ as the donor), E.C. 1.7.3 (N-containing groups as the donor) and E.C. 1.8.3 (S-containing groups as the donor) come into consideration, wherein enzymes of the EC class EC 1.1.3.

In a preferred embodiment, the hydrogen peroxide-producing oxidoreductase is one in which a sugar is used as the electron donor. The hydrogen peroxide-producing and sugar-oxidizing oxidoreductase is preferably chosen from glucose oxidase (EC 1.1.3.4), hexose oxidase (EC 1.1.3.5), galactose oxidase (EC 1.1.3.9) and pyranose oxidase (EC 1.1.3.10). According to the invention, glucose oxidase (EC 1.1.3.4) is particularly preferred. In one embodiment, aromatic compounds are added that interact with the enzymes to enhance the activity of the oxidoreductases (Enhancer) or to facilitate electron flow (Mediators) between the oxidizing enzymes and the stains over strongly different redox potentials.

At least one enzyme may be selected from oxidases such as amino acid oxidase and polyol oxidase (e.g., WO 2008/051491). Oxidases and their corresponding substrates may be used as hydrogen peroxide generating enzyme systems, and thus a source of hydrogen peroxide.

Several enzymes, such as peroxidases, haloperoxidases and perhydrolases, require a source of hydrogen peroxide. By studying EC 1.1.3._, EC 1.2.3._, EC 1.4.3._, and EC 1.5.3._ or similar classes (under the International Union of Biochemistry), other examples of such combinations of oxidases and substrates are easily recognized by one skilled in the art.

In one embodiment at least one oxidoreductase is chosen from enzymes that use peroxides as the electron accepter (EC-Classes 1.11 or 1.11.1), in particular, from catalases (EC 1.11.1.6), peroxidases (EC 1.11.1.7), glutathione peroxidases (EC 1.11.1.9), chloride peroxidases (EC 1.11.1.10), manganese peroxidases (EC 1.11.1.13) and/or lignin peroxidases (EC 1.11.1.14), which can also be generally classified under the term peroxidases. Examples of useful peroxidases include peroxidases from *Coprinus,* e.g. from *C*. *cinereus,* and variants thereof as those described in WO 93/24618, WO 95/10602, WO 98/10060 and WO 98/15257.

A peroxidase for use in the invention also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase is a chloroperoxidase. In one embodiment, the haloperoxidase is a vanadium haloperoxidase, i.e., a vanadate-containing haloperoxidase. In one embodiment of the present invention the vanadate-containing haloperoxidase is combined with a source of chloride ion.

Haloperoxidases have been isolated from many different fungi, in particular from the fungus group dematiaceous hyphomycetes, such as *Caldariomyces,* e.g., *C*. *fumago, Alternaria, Curvularia,* e.g., *C*. *verrucu*-
losa and *C*. *inaequalis, Drechslera, Ulocladium* and *Botrytis.* Haloperoxidases have also been isolated from bacteria such as *Pseudomonas,* e.g. *P. pyrrocinia,* and *Streptomyces,* e.g. *S*. *aureofaciens.*

In one embodiment, the haloperoxidase is from *Curvularia sp.,* in particular *Curvularia verruculosa* or *Curvularia inaequalis,* such as *C*. *inaequalis* CBS 102.42 as described in WO 95/27046; or *C*. *verruculosa* CBS 147.63 or *C*. *verruculosa* CBS 444.70 as described in WO 97/04102; or from *Drechslera hartlebii* as described in WO 2001/79459, *Dendryphiella salina* as described in WO 2001/79458, *Phaeotrichoconis crotalarie* as described in WO 2001/79461, or *Geniculosporium sp.* as described in WO 2001/79460.

Commercially available peroxidases include Guardzyme^{™} (Novozymes A/S), PrimaGreen^{™} Oxy (DuPont).

### Laccase

At least one enzyme may be selected from laccases. The term "laccase activity" is defined herein as covered by enzyme classification EC 1.10.3.2, or a similar activity, such as a catechol oxidase activity (EC 1.10.3.1), o-aminophenol oxidase activity (EC 1.10.3.4), or bilirubin oxidase activity (EC 1.3.3.5), that catalyzes the oxidation of a substrate using molecular oxygen.

"Laccase activity" is determined by oxidation of syringaldazin under aerobic conditions. The violet colour produced is measured at 530 nm. The analytical conditions are 19 µM syringaldazin, 23 mM Tris/maleate buffer, pH 7.5, 30°C, and 1 min reaction time.

Preferred laccase enzymes are enzymes of microbial origin. The enzymes may be derived from plants, bacteria or fungi (including filamentous fungi and yeasts; e.g. *Polyporus radiata* (WO 92/01046), *Coriolus hirsutus* (JP 2238885), *Coprinopsis cinerea* (WO 97/08325), *Myceliophthora thermophila* (WO 95/33836)).

In one embodiment, laccase is selected from those as described in SEQ ID NO: 2, 4, 6, and 8 of WO 2009/127702 and variants thereof.

At least one laccase may be selected from commercially available laccase Denilite^{®} 1 and 2 from Novozymes.

### Lyase

In one embodiment, at least one enzyme is selected from lyases. "Lyase" may be a pectate lyase derived from *Bacil*lus, particularly *B*. *licheniformis* or *B*. *agaradhaerens,* or a variant derived of any of these, e.g. as described in US 6,124,127, WO 99/027083, WO 99/027084, WO 2002/006442, WO 2002/092741, WO 2003/095638.

Commercially available pectate lyases are Xpect^{™}, Pectawash^{™} and Pectaway^{™} (Novozymes A/S); Prima-Green^{™}, EcoScour (DuPont).

### Other enzymes

In one embodiment, at least one enzyme is selected from the group of pectinases (EC 3.2.1.15 gycosidase), and/or arabinases (EC 3.2.1.99), and/or galactanases (EC 3.2.1.89 and EC 3.2.1.181), and/or xylanases (EC 3.2.1.8, EC 3.2.1.32, EC 3.2.1.136, and EC 3.2.1.156).

In one embodiment, at least one enzyme is a dispersin, preferably at least one dispersin which is at least 80% identical to SEQ ID NO:10 as disclosed in WO2017/186943.

### Detergent compositions

In one embodiment, the present invention is directed to the use of the polypeptide having protease activity of the present invention in a detergent composition. Thus, the present invention is also directed to a detergent composition comprising the polypeptide having protease activity of the present invention and one or more detergent component. Thus, the present invention also relates to a method for making a detergent composition comprising the steps of mixing
a) a polypeptide having protease activity of the present invention; and
b) one or more detergent components described herein.

The present invention also refers to a method for making a detergent composition with improved protease stability and/or for providing a detergent composition with improved wash performance comprising the steps of mixing
a) a polypeptide having protease activity of the present invention; and
b) one or more detergent components described herein.

The addition of the liquid protease formulation to a detergent composition, preferably liquid detergent composition, usually occurs in a weight ratio liquid protease formulation:detergent composition of about 1:1000, 1:500, 1:100, 1:50, 1:30, 1:25, 1:20, or 1:10.

The one or more detergent components may be selected from the group consisting of additional enzyme different from the protease, enzyme stabilizing system, surfactant, defoamer, builder, polymer, bleaching system (bleach), rheology modifier, hydrotrope, softening agent, desiccant, whitening agent, buffer, preservative, anti-corrosion additive, dyestuff and fragrance.

Preferably, at least one component of the detergent is selected from the group consisting of surfactant, builder, polymer, preservative, and additional enzyme different to the protease. Preferably one or more of the detergent components, preferably the surfactant and/or the builder, is bio-degradable and/or bio-based.

Detergent components may have more than one function in the final application of a detergent composition, therefore any detergent component mentioned in the context of a specific function herein may also have another function in the final application of a detergent composition. The function of a specific detergent component in the final application of a detergent composition usually depends on its amount within the detergent composition, i.e., the effective amount of a detergent component. Detergent components vary in type and/or amount in a detergent composition depending on the desired application such as laundering white textiles, colored textiles, and wool. The component(s) chosen further depend on the physical form of a detergent composition (liquid, solid, gel, provided in pouches or as a tablet, etc.). The component(s) chosen e.g. for laundering formulations further depend on regional conventions which themselves are related to aspects like washing temperatures used, mechanics of laundry machine (vertical vs. horizontal axis machines), water consumption per wash cycle etc. and geographical characteristics like average hardness of water.

In one embodiment, a detergent composition is a formulation of more than two detergent components, wherein at least one component is effective in stain-removal, at least one component is effective in providing the optimal cleaning conditions, and at least one component is effective in maintaining the physical characteristics of the detergent.

The detergent composition can be a liquid or solid detergent composition or a combination of liquid and solid detergent composition. The solid detergent composition can be a soap bar or a powder detergent composition, preferably a powder detergent composition, wherein the powder detergent composition can be pressed to a tablet.

The detergent composition can be a unit dose or multi dose composition. The detergent composition can be in the form of a pouch, including multi-compartment pouches. The detergent composition can be a laundry or dish washing detergent composition, suitable for home care and/or industrial and institutional (|&|) cleaning. Both laundry and dish wash composition can be in the form of a hand wash or automated wash composition. Preferably, the dish wash composition is an Automatic Dish Wash (ADW) composition.

Detergent pouches can be of any form, shape and material which is suitable for holding the composition, e.g., without allowing the release of the composition from the pouch prior to water contact. The pouch is made from water-soluble film, which encloses an inner volume. Said inner volume can be divided into compartments of the pouch. Preferred films are polymeric materials preferably polymers which are formed into a film or sheet, e.g., polyvinyl alcohol copolymers and hydroxypropyl methyl cellulose (HPMC). The pouches can comprise a solid laundry detergent composition or part components and/or a liquid detergent composition or part components separated by the water-soluble film. The compartment for liquid components can be different in composition from compartments containing solids (see e.g. US 2009/0011970).

In one embodiment, the detergent composition has a pH in the range of 5-12, preferably in the range of 6-11, more preferably in a range selected from 6-10, 7-9, and 7.5-8.5. In one embodiment, the formulation is a detergent composition, preferably a liquid detergent composition.

In one embodiment, the detergent compositions according to the invention comprise one or more surfactant(s). According to its ionic charge, a surfactant is called non-ionic, anionic, cationic, or amphoteric.

The detergent composition of the present invention may comprise one or more surfactants, which may be anionic and/or cationic and/or non-ionic and/or semi-polar and/or zwitterionic, or a mixture thereof. In a preferred embodiment, the detergent compositions of the invention comprise at least one surfactant. In a particular embodiment, the detergent composition of the present invention includes a mixture of one or more nonionic surfactants and one or more anionic surfactants. The surfactant(s) is/are typically present at a level of from about 0.1 to 60 wt.-%, such as 1 to 40 wt.-%, 3 to 20 wt.-% or 3 to 10 wt.-%. The surfactant(s) is/are chosen based on the desired cleaning application, and includes any conventional surfactant(s) known in the art. Any surfactant known in the art for use in detergents may be utilized. Non-limiting examples of surfactants are disclosed in McCutcheon's 2016 Detergents and Emulsifiers, and McCutcheon's 2016 Functional Materials, both North American and International Edition, MC Publishing Co, 2016 edition. Further useful examples are disclosed in earlier editions of the same publications which are known to those skilled in the art.

When included therein, the detergent will usually comprise from about 1 to 40 wt.-%, such as 5 to 30 wt.-%, 5 to 15 wt.-% or 20 to 25 wt.-%, of an anionic surfactant. Non-limiting examples of anionic surfactants include sulfates and sulfonates, in particular linear alkyl benzene sulfonates (LAS), isomers of LAS, branched alkyl benzene sulfonates (BABS), phenyl alkane sulfonates, alpha-olefin sulfonates (AOS), olefin sulfonates, alkene sulfonates, alkane-2,3-diylbis(sulfates), hydroxy alkane sulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkane sulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenyl succinic acid, dodecenyl/tetradecenyl succinic acid (DTSA), fatty acid derivatives of amino acids, diesters and monoesters of sulfo succinic acid or soap, and combinations thereof.

When included therein, the detergent will usually comprise from about 0 to 10 wt.-% of a cationic surfactant. Non-limiting examples of cationic surfactants include alkyl dimethyl ethanolamine quat (ADMEAQ), cetyl trimethyl ammonium bromide (CTAB), dimethyl distearyl ammonium chloride (DSDMAC), and alkyl benzyl dimethyl ammonium, alkyl quaternary ammonium compounds, alkoxylated quaternary ammonium (AQA) compounds, and combinations thereof. When included therein, the detergent will usually comprise from about 0.2 to 40 wt.-% of a nonionic surfactant, e.g. 0.5 to 30 wt.-%, in particular 1 to 20 wt.-%, 3 to 10 wt.-%, 3 to 5 wt.-% or 8 to 12 wt.-%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkyl phenol ethoxylates (APE), nonyl phenol ethoxylates (NPE), alkyl polyglycosides (APG), alkoxylated amines, fatty acid monoethanolamides (FAM), fatty acid diethanol amides (FADA), ethoxylated fatty acid monoethanolamides (EFAM), propoxylated fatty acid monoethanolamides (PFAM), polyhydroxy alkyl fatty acid amides, or N-acyl N-alkyl derivatives of glucosamine (glucamides, GA, or fatty acid glucamide, FAGA), as well as products available under the trade names SPAN and TWEEN, and combinations thereof.

When included therein, the detergent will usually comprise from about 0 to 10 wt.-% of a semipolar surfactant. Non-limiting examples of semipolar surfactants include amine oxides (AO) such as alkyl dimethyl amine oxide, N-(coco alkyl)-N,N-dimethyl amine oxide and N-(tallow-alkyl)-N,N-bis-(2-hydroxy ethyl) amine oxide, fatty acid alkanol amides and ethoxylated fatty acid alkanol amides, and combinations thereof.

When included therein, the detergent will usually comprise from about 0 to 10 wt.-% of a zwitterionic surfactant. Non-limiting examples of zwitterionic surfactants include betaine, alkyl dimethyl betaine, sulfo betaine, and combinations thereof.

The detergent compositions according to the invention may comprise one or more compounds selected from complexing agents (chelating agents (chelants), sequestrating agents), precipitating agents, and ion exchange compounds which may form water-soluble complexes with calcium and magnesium. Such compounds may be called "builders" or "building agents" herein, without meaning to limit such compounds to this function in the final application of a detergent composition.

In one embodiment, the detergent composition of the invention comprises at least one builder selected from non-phosphate based builders such as sodium gluconate, citrate(s), silicate(s), carbonate(s), phosphonate(s), amino carboxylate(s), polycarboxylate(s), polysulfonate(s), and polyphosphonate(s). In one embodiment, the detergent composition of the invention comprises a strong sequestering builder. Preferably, detergent compositions of the current invention are free from phosphate, meaning essentially free from phosphate-based builders. Herein, "essentially free from phosphate" is to be understood as meaning that the content of phosphate and polyphosphate is in sum in the range of 10 ppm to 1% by weight, determined by gravimetry and referring to the respective inventive detergent composition. In another preferred embodiment, the detergent composition comprises a phosphonate, wherein the phosphonate is preferably DTPMP and/or HEDP.

In one embodiment, the detergent compositions of the invention comprise at least one "citrate" selected from the mono- and the dialkali metal salts and in particular the mono- and preferably the trisodium salt of citric acid, ammonium or substituted ammonium salts of citric acid as well as citric acid as such. Citrate can be used as the anhydrous compound or as the hydrate, for example as sodium citrate dihydrate. The citrate may be comprised in a total amount in the range of 0% to about 20% by weight, in the range of about 0.5% to about 10% by weight, or in the range of 1-5% by weight, all relative to the total weight of the detergent composition. In one embodiment, the detergent composition of the invention comprises a total amount of citrate in the range of about 1-3% relative to the total weight of the detergent composition.

Detergent compositions of the invention may comprise one or more silicates. "Silicate(s)" in the context of the present invention include in particular sodium disilicate and sodium metasilicate, aluminosilicates such as sodium aluminosilicates like zeolith A (i.e. Na₁₂(AlO₂)₁₂(SiO₂)₁₂*27H₂O), and sheet silicates, in particular those of the formula alpha-Na₂Si₂O₅, beta-Na₂Si₂O₅, and delta-Na₂Si₂O₅.

Detergent compositions of the invention may comprise one or more carbonates. The term "carbonate(s)" includes alkali metal carbonates and alkali metal hydrogen carbonates, preferred are the sodium salts. Particularly suitable is sodium carbonate (Na₂CO₃).

Detergent compositions of the invention may comprise one or more phosphonates. "Phosphonates" include, but are not limited to 2-phosphinobutane-1,2,4-tricarboxylic acid (PBTC); ethylenediaminetetra(methylenephosphonic acid)

(EDTMPA); 1-hydroxyethane-1,1-diphosphonic acid (HEDP), CH₂C(OH)[PO(OH)₂]₂; aminotris(methylenephosphonic acid) (ATMP), N[CH₂PO(OH)₂]₃; aminotris(methylenephosphonate), sodium salt (ATMP), N[CH₂PO(ONa)₂]₃; 2-hydroxyethyliminobis(methylenephosphonic acid), HOCH₂CH₂N[CH₂PO(OH)₂]₂; diethylenetriaminepenta(methylenephosphonic acid) (DTPMP), (HO)₂POCH₂N[CH₂CH₂N[CH₂PO(OH)₂]₂]₂; diethylenetriaminepenta(methylenephosphonate), sodium salt, C₉H₍₂₈₋ₓ₎N₃NaₓO₁₅P₅ (x=7); hexamethylenediamine(tetramethylenephosphonate), potassium salt, C₁₀H₍₂₈₋ₓ₎N₂KₓO₁₂P₄ (x=6); and bis(hexamethylene)triamine(pentamethylenephosphonic acid), (HO₂)POCH₂N[(CH₂)₂N[CH₂PO(OH)₂]₂]₂. Salts thereof may be suitable, too.

Detergent compositions of the invention may comprise one or more aminocarboxylates. Non-limiting examples of suitable "amino carboxylates" include, but are not limited to: diethanol glycine (DEG), dimethylglycine (DMG), nitrilitriacetic acid (NTA), N-hydroxyethylaminodiaoetic acid, ethylenediaminetetraacetic acid (EDTA), N-(2hydroxyethyl)iminodiacetic acid (HEIDA), hydroxyethylenediaminetriacetic acid, N-hydroxyethyl-ethylenediaminetriacetic acid (HEDTA), hydroxyethylenediaminetetraaoetic acid, diethylenetriaminepentaacetic acid (DTPA), and methylglycinediacetic acid (MGDA), glutamic acid-diacetic acid (GLDA), iminodisuccinic acid (IDS), hydroxyiminodisuccinic acid, ethylenediaminedisuccinic acid (EDDS), aspartic acid-diacetic acid, and alkali metal salts or ammonium salts thereof. Further suitable are aspartic acid-N-monoacetic acid (ASMA), aspartic acid-N,N-diacetic acid (ASDA), aspartic acid-N-mono-propionic acid (ASMP), N-(2-sulfomethyl) aspartic acid (SMAS), N-(2-sulfoethyl) aspartic acid (SEAS), N-(2-sulfomethyl) glutamic acid (SMGL), N-(2-sulfoethyl) glutamic acid (SEGL), N-methyliminodiaoetic acid (MIDA), alpha-alanine-N,N-diacetic acid (alpha-ALDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA), taurine-N,N-diacetic acid (TUDA) and sulfomethyl-N,N-diacetic acid (SMDA) and alkali metal salts or ammonium salts thereof. Preferred are MGDA and/or EDDS. The term "ammonium salts" as used in in this context refers to salts with at least one cation that bears a nitrogen atom that is permanently or temporarily quatemized. Examples of cations that bear at least one nitrogen atom that is permanently quaternized include tetramethylammonium, tetraethylammonium, dimethyldiethyl ammonium, and n-C₁₀-C₂₀-alkyl trimethyl ammonium. Examples of cations that bear at least one nitrogen atom that is temporarily quaternized include protonated amines and ammonia, such as monomethyl ammonium, dimethyl ammonium, trimethyl ammonium, monoethyl ammonium, diethyl ammonium, triethyl ammonium, n-C₁₀-C₂₀-alkyl dimethyl ammonium 2-hydroxyethylammonium, bis(2-hydroxyethyl) ammonium, tris(2-hydroxyethyl)ammonium, N-methyl 2-hydroxyethyl ammonium, N,N-dimethyl-2-hydroxyethylammonium, and especially NH₄⁺.

In one embodiment, detergent compositions of the invention comprise more than one builder. Preferably, inventive detergent compositions contain less than 0.2% by weight of nitrilotriacetic acid (NTA), or 0.01 to 0.1% NTA by weight relative to the total weight of the detergent composition.

In one embodiment, the detergent composition of the invention comprises of at least one aminocarboxylate selected from methylglycine diacetate (MGDA), glutamic acid diacetate (GLDA), and the respective salts thereof, e.g., alkali (such as sodium) salts thereof in amounts in the range of 0.1% to 25.0% by weight, in the range of 1.0% to 18.0% by weight, in the range of 3.0% to 15.0% by weight, in the range of 3.0% to 10.0% by weight, or in the range of 5.0% to 8.0% by weight relative to the total weight of the detergent composition.

The detergent compositions of the invention may comprise one or more hydrotropes. One or more hydrotropes may be selected from organic solvents such as ethanol, isopropanol, ethylene glycol, 1 ,2-propylene glycol, and further organic solvents known in the art that are water-miscible under normal conditions without limitation. In one embodiment, the detergent composition of the invention comprises 1,2-propylene glycol in a total amount in the range of 5-10% by weight, preferably of about 6% by weight, all relative to the total weight of the detergent composition. Further non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycol ethers, sodium hydroxy naphthoate, sodium hydroxy naphthalene sulfonate, sodium ethyl hexyl sulfate, and combinations thereof.

In one embodiment, the detergent composition comprises at least one preservative. Preferably, preservative means substances that are added to a liquid composition for the purpose of preservation, meaning more preferably that compounds known to have preserving features comprised in a liquid composition formed in the production process are excluded from the term preservatives. In one embodiment, the preservative is selected from the group consisting of 2-phenoxyethanol, glutaraldehyde, 2-bromo-2-nitropropane-1,3-diol, formic acid in acid form or as its salt, and 4,4'-dichloro 2-hydroxydiphenylether. Usually, the liquid compositions of the invention comprise at least one preservative in amounts below 10ppm, such as in amounts ranging from 2 ppm to 5% by weight relative to the total weight of the liquid composition. Preferably, the liquid composition is free from preservatives, meaning that preservatives are comprised in amounts less than 1 ppm.

In one embodiment, the detergent composition comprising a polypeptide having protease activity of the present invention further comprises one or more additional enzymes different from the protease variant. Preferably, the additional enzyme is selected from the group consisting of amylases, lipases, proteases other than the polypeptide having protease activity of the present invention, cellulases, mannanases, hemicellulases, phospholipases, esterases, pectinases, lactases, peroxidases, xylanases, cutinases, pectate lyases, keratinases, reductases, oxidases, phenoloxidases, lipoxygenases, ligninases, pullulanases, tannases, pentosanases, malanases, beta-glucanases, arabinosidases, hyaluronidases, chondroitinases, laccases, nucleases, ribonucleases (RNAses), deoxyribonucleases (DNAses), phosphodiesterases, phytases, carbohydrases, galactanases, xanthanases, xyloglucanases, oxidoreductases, perhydrolases, aminopeptidases, asparaginases, carbohydrases, carboxypeptidases, catalases, chitinases, cyclodextrin glycosyltransferases, alpha-galactosidases, beta-galactosidases, glucoamylases, alpha-glucosidases, beta-glucosidases, invertases, transglutaminases, and dispersins, and combinations of at least two of the foregoing types. More preferably, the additional enzyme is selected from the group consisting of amylases, lipases, proteases other than the polypeptide having protease activity of the present invention, cellulases, mannanases, esterases, xylanases, DNAses, dispersins, pectinases, oxidoreductases, and cutinases, and combinations of at least two of the foregoing types. Further preferred, the additional enzyme is selected from the group consisting of amylases, lipases, proteases other than the polypeptide having protease activity of the present invention, cellulases, mannanases, and combinations of at least two of the foregoing types. Most preferably, the additional enzyme is an amylase, preferably, an alpha-amylase.

Particular preferred additional enzymes are disclosed elsewhere herein and that description is incorporated by reference also to this part of the description.

The composition of the present invention can comprise more than one enzyme of different types, e.g., an amylase and a protease, or more than one enzyme of the same type, e.g., two or more different proteases, or mixtures thereof, e.g., an amylase and two different proteases.

The detergent compositions may comprise water-soluble sources of calcium and/or magnesium ions. In one embodiment, the detergent composition comprises an enzyme stabilizing system as described herein. Preferably, in particular in the case of liquid detergent compositions, the detergent composition may comprise at least one protease inhibitor as described herein, preferably selected from boronic acid derivatives, preferably 4-FPBA, and peptide aldehyde, preferably Z-VAL-H or Z-GAY-H. Preferably, the detergent composition is boron-free.

In one embodiment, the invention relates to a method to provide a detergent composition, preferably a liquid detergent composition, more preferably a liquid laundering detergent composition, comprising the steps of mixing in one or more steps
(a) at least one polypeptide having protease activity of the present invention, preferably wherein the protease is provided within a protease formulation as described herein; and
(b) at least one detergent component, preferably selected from surfactant, builder, polymer, preservative, and additional enzyme different to the protease, present in amounts effective in cleaning performance and/or effective in maintaining the physical characteristics of the detergent.

In one embodiment, the present invention is directed to a detergent composition comprising
a) a polypeptide having protease activity of the present invention;
b) one or more surfactants, preferably, in a concentration of 0.2-65%, preferably 0.2-40%,
c) one or more builders, preferably, in a concentration of 0.01-25%, and
d) optionally one or more additional compounds selected from the group consisting of additional enzyme different from
the protease under a), defoamer, polymer, bleaching system (bleach), rheology modifier, hydrotrope, softening agent, desiccant, whitening agent, buffer, preservative, anti-corrosion additive, dyestuff and fragrance; preferably wherein detergent composition, is a liquid, powder, pouch, or capsule detergent composition.

Preferably the detergent composition, preferably powder detergent composition, of the present invention comprises in addition to the polypeptide having protease activity of the present invention one or more of the compounds selected from the group consisting of alcohol ethoxylate 7EO, Coco fatty acid C₁₂-₁₈, C₁₂-C₁₄-fatty alcohol ether sulfate (1 -3 EO, preferably 2 EO), Linear alkyl benzene sulphonic acid, AcetateNa, CitrateNa, Na Silicate, Na Carbonate, Na Phosphate, Na Hydrogencarbonate, Zeolite4A, HEDP, MGDA, Na Sulfate, Na Chloride, optical brightener, and polymers and optionally Bleach activator and Percarbonate.

Preferably the detergent composition, preferably powder detergent composition, of the present invention comprises in addition to the polypeptide having protease activity of the present invention
b) one or more surfactants selected from the group consisting of Alcohol ethoxylate 7EO, Coco fatty acid C₁₂-₁₈, C₁₂-C₁₄- fatty alcohol ether sulfate (1 -3 EO, preferably 2 EO), Linear alkyl benzene sulphonic acid, preferably, in a concentration of 0.2-65%,
c) one or more builders selected from the group consisting of HEDP, MGDA, GLDA, and DTPMP, preferably, in a concentration of 0.01-25%, and
d) one or more compounds selected from the group consisting of AcetateNa, CitrateNa, Na Silicate, Na Carbonate, Na Phosphate, Na Hydrogencarbonate, Zeolite 4A, Na Sulfate, Na Chloride, optical brightener, carboxymethylcellulose, and polymers, and optionally Bleach activator and Percarbonate.

Preferably the detergent composition, preferably liquid detergent composition, of the present invention comprises in addition to the polypeptide having protease activity of the present invention one or more of the compounds selected from the group consisting of alcohol ethoxylate 7EO, Coco fatty acid C₁₂-₁₈, C₁₂-C₁₄-fatty alcohol ether sulfate (1 -3 EO, preferably 2 EO), Linear alkyl benzene sulphonic acid, sulphonic acid, 1,2 Propandiol, Triethanolamine, Monoethanolamine, NaOH, Glycerol, Ethanol, Na citrate, and Polymer.

Preferably the detergent composition, preferably liquid detergent composition, of the present invention comprises in addition to the polypeptide having protease activity of the present invention
b) one or more surfactants selected from the group consisting of Alcohol ethoxylate 7EO, Coco fatty acid C₁₂-₁₈, C₁₂-C₁₄-fatty alcohol ether sulfate (1-3 EO, preferably 2 EO), Linear alkyl benzene sulphonic acid, preferably, in a concentration of 0.2-65%,
c) one or more builders selected from the group consisting of HEDP, MGDA, GLDA, and DTPMP, preferably, in a concentration of 0.01-25%, and
d) one or more compound selected from the group consisting of sulphonic acid, 1,2 Propandiol, Triethanolamine, Monoethanolamine, NaOH, Glycerol, Ethanol, Na citrate, and Polymer.

### Preferred formulations

In one embodiment, the polypeptide having protease activity of the present invention is included in a formulation comprising one or more, preferably all, compounds selected from the group consisting of (all percentages are w/w):
The polypeptide having protease activity of the present invention, from 0.01% to 0.5%;
Anionic detersive surfactant (such as alkyl benzene sulphonate, alkyl ethoxylated sulphate and mixtures), from 8% to 15%;
Non-ionic detersive surfactant (such as alkyl ethoxylated alcohol), from 0.5% to 4%;
Cationic detersive surfactant (such as quaternary ammonium compounds), from 0 to 4%;
Other detersive surfactant (such as zwitterionic detersive surfactants, amphoteric surfactants and mixtures thereof), from 0% to 4%;
Carboxylate polymer (such as co-polymers of maleic acid and acrylic acid), from 1% to 4%;
Polyethylene glycol polymer (such as a polyethylene glycol polymer comprising poly vinyl acetate side chains), from 0.5% to 4%;
Polyester soil release polymer (such as Repel-o-tex from and/or Texcare polymers), from 0.1 to 2%;
Cellulosic polymer (such as carboxymethyl cellulose, methyl cellulose and combinations thereof), from 0.5% to 2%;
Other polymer (such as amine polymers, dye transfer inhibitor polymers, hexamethylenediamine derivative polymers, and mixtures thereof), from 0% to 4%;
Zeolite builder and phosphate builder (such as zeolite 4A and/or sodium tripolyphosphate), from 0% to 4 wt%;
Other builder (such as sodium citrate and/or citric acid), from 0% to 3%;
Carbonate salt (such as sodium carbonate and/or sodium bicarbonate), from 15% to 30%;
Silicate salt (such as sodium silicate), from 0% to 10%;
Filler (such as sodium sulphate and/or bio-fillers), from 10% to 40%;
Source of available oxygen (such as sodium percarbonate), from 10% to 20%;
Bleach activator (such as tetraacetylethylene diamine (TAED) and/or nonanoyloxybenzenesulphonate (NOBS), from 2% to 8%;
Bleach catalyst (such as oxaziridinium-based bleach catalyst and/or transition metal bleach catalyst), from 0% to 0.1%; Other bleach (such as reducing bleach and/or pre- formed peracid), from 0% to 10%;
Chelant (such as ethylenediamine-N'N'-disuccinic acid (EDDS) and/or hydroxyethane diphosphonic acid (HEDP)), from 0.2% to 1 %;
Photobleach (such as zinc and/or aluminium sulphonated phthalocyanine), from 0% to 0.1%;
Hueing agent (such as direct violet 99, acid red 52, acid blue 80, direct violet 9, solvent violet 13 and any combination thereof), from 0% to 1%;
Brightener (such as brightener 15 and/or brightener 49), from 0.1% to 0.4%;
Fabric softener (such as montmorillonite clay and/or polydimethylsiloxane (PDMS)), from 0% to 4%;
Flocculant (such as polyethylene oxide), from 0% to 1%;
Suds suppressor (such as silicone and/or fatty acid), from 0% to 0.1 %;
Perfume (such as perfume microcapsule, spray-on perfume, starch encapsulated perfume accords, perfume loaded zeolite, and any combination thereof), from 0.1 % to 1 %; and
Aesthetics (such as coloured soap rings and/or coloured speckles/noodles), from 0% to 1%; and
Optionally an additional protease differing from the protease variant described herein (such as Savinase, Coronase, Ovozyme, Kannase, Liquanase, Polarzyme, Purafect, Purafast, Properase, Excellase, FN3, FN4, Effectenz P, Preferenz P, Progress Uno, Progress Excel, Blaze, Excellenz P), from about 0.05 wt% to about 0.2 wt%;
Optionally Amylase (such as Termamyl, Termamyl Ultra, Natalase, Optisize HT Plus, Purastar, Powerase, Stainzyme, Preferenz S, Effectenz S, Amplify, Amplify Prime, Excellenz S and any combination thereof), from about 0.05 wt% to about 0.2 wt%;
Optionally Cellulase (such as Carezyme, Celluclean, Biotouch, Whitezyme, Revitalenz, and combinations thereof), from 0.05% to 0.2%;
Optionally Lipase (such as Lipex, Lipolex, Lipoclean, Preferenz L, and any combination thereof), from 0.05% to 0.2%;
Optionally other enzyme (such as xyloglucanase, cutinase, pectate lyase (such as Xpect), Mannanase (such as Mannanway, Mannastar, Marvellenz, Effectenz M, Preferenz M, Preferenz F, and combinations thereof) bleaching enzyme, and combinations thereof), from 0.05% to 0.2%;
Miscellaneous balance.
In another embodiment, the polypeptide having protease activity of the present invention is included in a formulation comprising one or more, preferably all, compounds selected from the group consisting of (all percentages are w/w): The polypeptide having protease activity of the present invention, from 0.01% to 0.5%;
Carboxyl group-containing polymer (comprising from about 60% to about 70% by mass of an acrylic acid-based monomer (A); and from about 30% to about 40% by mass of a sulfonic acid group-containing monomer (B); and wherein the average molecular weight is from about 23,000 to about 50,000 preferably in the range of from about 25,000 to about 38,000 as described in WO2014032269), from about 0.5 wt% to ab out 1.5 wt%;
Anionic detersive surfactant (such as alkyl benzene sulphonate, alkyl ethoxylated sulphate and mixtures thereof), from about 8 wt% to about 15 wt%;
Non-ionic detersive surfactant (such as alkyl ethoxylated alcohol) from about 0.5 wt% to 4wt%;
Cationic detersive surfactant (such as quaternary ammonium compounds), from about 0 wt% to about 4 wt%;
Other detersive surfactant (such as zwitterionic detersive surfactants, amphoteric surfactants and mixtures thereof), from about 0 wt% to 4 wt%;
Carboxylate polymer (such as co-polymers of maleic acid and acrylic acid) from about 1 wt% to about 4 wt%; Polyethylene glycol polymer (such as a polyethylene glycol polymer comprising poly vinyl acetate side chains), from about 0 wt% to about 4 wt%;
Polyester soil release polymer (such as Repel-O-Tex and/or Texcare polymers), from about 0.1 wt% to about 2 wt%; Cellulosic polymer (such as carboxymethyl cellulose, methyl cellulose and combinations thereof) from about 0.5 wt% to about 2 wt%;
Other polymer (such as amine polymers, dye transfer inhibitor polymers, hexamethylenediamine derivative polymers, and mixtures thereof), from about 0 wt% to about 4 wt%;
Zeolite builder and phosphate builder (such as zeolite 4A and/or sodium tripolyphosphate), from about 0 wt% to about 4 wt%;
Other builder (such as sodium citrate and/or citric acid), from about 0 wt% to about 3 wt%;
Carbonate salt (such as sodium carbonate and/or sodium bicarbonate), from about 15 t% to about 30 wt%;
Silicate salt (such as sodium silicate), from about 0 wt% to about 10 wt%;
Filler (such as sodium sulphate and/or bio-fillers), from about 10 wt% to about 40 wt%;
Source of available oxygen (such as sodium percarbonate), from about 10 wt% to about 20wt%;
Bleach activator (such as tetraacetylethylene diamine (TAED) and/or nonanoyloxybenzenesulphonate (NOBS), from about 2 wt% to about 8 wt%;
Bleach catalyst (such as oxaziridinium-based bleach catalyst and/or transition metal bleach catalyst), from about 0 wt% to about 0.1 wt%;
Other bleach (such as reducing bleach and/or pre-formed peracid), from about 0 wt% to about 10 wt%;
Chelant (such as ethylenediamine-N'N'-disuccinic acid (EDDS) and/or hydroxyethane diphosphonic acid (HEDP)), from about 0.2 wt% to about 1 wt%;
Photobleach (such as zinc and/or aluminium sulphonated phthalocyanine), from about 0 wt% to about 0.1 wt%;
Hueing agent (such as direct violet 99, acid red 52, acid blue 80, direct violet 9, solvent violet 13 and any combination thereof), from about 0 wt% to about 0.5 wt%;
Brightener (such as brightener 15 and/or brightener 49), from about 0.1 wt% to about 0.4 wt%;
Fabric softener (such as montmorillonite clay and/or polydimethylsiloxane (PDMS)), from 0 wt% to 15 wt%; Flocculant (such as polyethylene oxide), from 0 wt% to 1 wt%;
Suds suppressor (such as silicone and/or fatty acid), from 0 wt% to 0.1 wt%;
Perfume (such as perfume microcapsule, spray-on perfume, starch encapsulated perfume accords, perfume loaded zeolite, and any combination thereof), from 0.1 wt% to 1 wt%; and
Aesthetics (such as colored soap rings and/or colored speckles/noodles), from 0 wt% to 1 wt%; and
Optionally an additional protease differing from the protease variant described herein (such as Savinase, Coronase, Ovozyme, Kannase, Liquanase, Polarzyme, Purafect, Purafast, Properase, Excellase, FN3, FN4, Effectenz P, Preferenz P, Progress Uno, Progress Excel, Blaze, Excellenz P), from about 0.05 wt% to about 0.2 wt%,
Optionally Amylase (such as Termamyl, Termamyl Ultra, Natalase, Optisize HT Plus, Purastar, Powerase, Stainzyme, Preferenz S, Effectenz S, Amplify, Amplify Prime, Achieve alpha, Excellenz S), from about 0.05 wt% to about 0.2 wt%, Optionally, Cellulase (such as Carezyme, Celluzyme, Celluclean, Biotouch, Whitezyme, Revitalenz, and combinations thereof, typically having an enzyme activity of about from 10 to 50mg active enzyme/ g), from about 0.05 wt% to 0.5 wt%
Optionally, Lipase (such as Lipex, Lipolex, Lipoclean, Preferenz L, and any combination thereof, typically having an enzyme activity of from about 10 mg to about 50 mg active enzyme/ g), from about 0.2 wt% to about 1 wt%
Optionally, other enzyme (such as xyloglucanase (e.g., Whitezyme), cutinase, pectate lyase (e.g., Xpect), mannanase, (e.g., Mannanway, Mannastar, Marvellenz, Effectenz M, Preferenz M, Preferenz F, and combinations thereof), bleaching enzyme, typically having an enzyme activity of from about 10 mg to about 50 mg active enzyme/g), from 0 wt% to 2 wt%,
Miscellaneous Balance.

Further preferred detergent formulations comprise the components listed below (all percentages are w/w):
- Aqua, Alcohol Ethoxy Sulfate, Alcohol Ethoxylate, Amino Oxide, Citrid Acid, C12-18 topped palm kernel fatty acid, Amylase, Glycosidase, Ethanol, 1,2 Propanediol, Sodium Formate, Calcium Chloride, Sodium hydroxide, Silicone Emulsion, Trans-sulphated EHDQ, a polypeptide having protease activity of the present invention;
- Linear sodium alkyl benzene sulfonate 8,8 %, Ethoxylated fatty alcohol C12-18 (7 EO) 4,7 %, Sodium soap 3,2 %, Anti foam DC2-4248S 3,9 %, Sodium aluminium silicate zeolite 4A 28,3 %, Sodium carbonate11,6 %, Sodium salt of a copolymer from acrylic and maleic acid (Sokalan CP5) 2,4 %, Sodium silicate 3,0 %, Carboxymethylcellulose 1,2 %, Dequest 2066 2,8 %, Optical whitener 0,2 %, Sodium sulfate 6,5 %, amylase 0,4 %, a protease variant as described herein;
- 12% LAS, 11% AEO Biosoft N25-7 (Nl), 7% AEOS (SLES), 6% MPG (monopropylene glycol), 3% ethanol, 3% TEA, 2.75% cocoa soap, 2. 75% soya soap, 2% glycerol, 2% sodium hydroxide, 2% sodium citrate, 1 % sodium formiate, 0.2% DTM PA and 0.2% PCA, a polypeptide having protease activity of the present invention;
- 5-15% Anionic surfactants; <5% Non-ionic surfactants, Phosphonates, Soap; Enzymes, Optical brighteners, Benzisothiazolinone, Methylisothiazolinone, Perfumes, Alpha-isomethyl ionone, Citronellol, Geraniol, Linalool, a polypeptide having protease activity of the present invention;
- Aqua, Sodium Dodecylbenzenesulfonate, C14-C15 Pareth-7, Sodium Citrate, Propylene Glycol, Sodium Palm Kernelate, Sodium Laureth Sulfate, MEA Dodecylbenzenesulfonage, Sulfated Ethoxylated Hexamethylenediamine Quaternized, Sodium Cumenesulfonate, Perfume, Co-polymer of PEG/Vinyl Acetate, Sodium formate, Hydrogenated Castor Oil, Sodium Diethylenetriamine Pentamethylene Phosphonate, PEG/PPG-10/2 Propylheptyl Ether, Butyophenyl Methylpropional, Polyvinylpyridine-N-Oxide, Sorbitol, Glycerin, Ethanolamine, Sodium Hydroxide, Alpha-Isomethyllonone, amylase, Calcium Chloride, Geraniol, Linalool, Citronelllol, Tripropylene Glycol, Glycosidase, Benzisothiazolinone, Dimethicone, Glycosidase, Sodium Acetate, Cellulase, Colorant, Glyceryl Stearate, Hydroxyethylcellulose, Silica, a polypeptide having protease activity of the present invention;
- Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, Perfume, Polyvinylpyridine-N-Oxide, Sorbitol, Calcium Chloride, amylase, Glycerin, Glucosidase, Glycosidase, Sodium Acetate, Colorant, Cellulase, a polypeptide having protease activity of the present invention;
- Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, Perfume, Sorbitol, Calcium Chloride, amylase, Glycerin, Glucosidase, Glycosidase, Sodium Acetate, Colorant, Cellulase, a polypeptide having protease activity of the present invention
- Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, Sorbitol, Calcium Chloride, amylase, Glycerin, Glycosidase, Sodium Acetate, Cellulase, Silica, a polypeptide having protease activity of the present invention;
- Aqua, Sodium Dodecylbenzenesulfonate, C₁₄-C₁₅ Pareth-7, Sodium Citrate, Propylene Glycol, Sodium Palm Kernelate, Sodium Laureth Sulfate, MEA Dodecylbenzenesulfonage, Sulfated Ethoxylated Hexamethylenediamine Quaternized, Sodium Cumenesulfonate, Perfume, Co-polymer of PEG/Vinyl Acetate, Sodium formate, C12-C14 Pareth-7, Hydrogenated Castor Oil, Sodium Diethylenetriamine Pentamethylene Phosphonate, PEG/PPG-10/2 Propylheptyl Ether, Butyophenyl Methylpropional, Fluorescent Brightener, Sorbitol, Glycerin, Ethanolamine, Sodium Hydroxide, Alpha-Isomethyl ionone, Amylase, Calcium Chloride, Geraniol, Linalool, Citronelllol, Tripropylene Glycol, Sodium Chloride, Glycosidase, Benzisothiazolinone, Dimethicone, Glycosidase, Sodium Acetate, Cellulase, Colorant, Glyceryl Stearate, Hydroxyethylcellulose, Silica, a polypeptide having protease activity of the present invention;
- 15-30% Anionic surfactants, Non-ionic surfacts, 5-15% Soap, < 5% Polycarboxylates, Perfume, Phosphates, Optical Brighteners, a protease variant as described herein;
- 15-30% Anionic Surfactants, 5-15% Non-ionic Surfactants, Soap, Benzisothiazolinone, Methylisothiazolinone, Perfumes, a polypeptide having protease activity of the present invention;
- 11 % LAS, 2% AS/AEOS, 2% soap, 3% AEO, 15.15% sodium carbonate, 3% sodium slilcate, 18.75% zeolite, 0.15% chelant, 2% sodium citrate, 1.65% AA/MA copolymer, 2.5% CMC and 0.5% SRP, a polypeptide having protease activity of the present invention;
- 16.5% LAS, 15% zeolite, 12% sodium disilicate, 20% sodium carbonate, 1 % sokalan, 35.5% sodium sulfate, a polypeptide having protease activity of the present invention;
- 15-30% Anionic surfactants, <5% Nonionic surfactants, Phosphonates, Polycarboxylates, Zeolites; Enzymes, Perfumes, Hexyl cinnamal, a polypeptide having protease activity of the present invention;
- 15 - 30 % of the following: anionic surfactants, oxygen-based bleaching agent and zeolites, less than 5 % of the following: non-ionic surfactants, phosphonates, polycarboxylates, soap, Further ingredients: Perfumes, Hexyl cinnamal, Benzyl salicylate, Linalool, optical brighteners, Enzymes and Citronellol, a polypeptide having protease activity of the present invention;
- Water, Alcohol Ethoxysulfate, Diethylene Glycol, Alcohol Ethoxylate, Ethanolamine, Linear Alkyl Benzene Sulfonate, Sodium Fatty Acids, Polyethyleneimine Ethoxylate, Citric Acid, Sodium Cumene Sulfonate, Propylene Glycol, DTPA, Disodium Diaminostilbene Disulfonate, Dipropylethyl Tetramine, Sodium Hydroxide, Sodium Formate, Calcium Formate, Dimethicone, Amylase, Liquitint^{™}, Hydrogenated Castor Oil, Fragrance, a polypeptide having protease activity of the present invention;
- Linear alkylbenzene sulfonate, propylene glycol, citric acid, sodium hydroxide, ethanolamine, ethanol, alcohol sulfate, polyethyleneimine ethoxylate, sodium fatty acids, diquaternium ethoxysulfate, amylase, diethylene glycol, laureth-9, alkyldimethylamine oxide, fragrance, disodium diaminostilbene disulfonate, DTPA, sodium formate, calcium formate, polyethylene glycol 4000, mannanase, Liquitint^{™} Blue, dimethicone, a polypeptide having protease activity of the present invention;
- Water, sodium alcoholethoxy sulfate, propylene glycol, ethanol, linear alkylbenzene sulfonate sodium, salt, polyethyleneimine ethoxylate, diethylene glycol, trans sulfated & ethoxylated hexamethylene diamine, alcohol ethoxylate, linear alkylbenzene sulfonate, MEA salt, sodium formate, sodium alkyl sulfate, DTPA, amine oxide, calcium formate, disodium diaminostilbene, disulfonate, amylase, dimethicone, benzisothiazolinone, a polypeptide having protease activity of the present invention;
- Water, alcoholethoxy sulfate, linear alkylbenzene sulfonate, diethylene glycol, propylene glycol, ethanolamine, citric acid, alcohol sulfate, sodium hydroxide, polyethyleneimine, ethoxylate, sodium fatty acids, ethanol, amylase, Laureth-9, diquatemium ethoxysulfate, lauramine oxide, sodium cumene, sulfonate, fragrance, DTPA, disodium, diaminostilbene, disulfonate, sodium formate, disodium distyrylbiphenyl, disulfonate, calcium formate, polyethylene glycol 4000, mannanase, pectinase, Liquitint^{™} Blue, dimethicone, a polypeptide having protease activity of the present invention;
- Water, alcoholethoxy sulfate, propylene glycol, sodium fatty acids, laurtrimonium chloride, ethanol, sodium hydroxide, sodium cumene sulfonate, citric acid, ethanolamine, diethylene glycol, silicone polyether, fragrance, polyethylene-imine ethoxylate, amylase, Laureth-9, DTPA, polyacrylamide quaternium chloride, disodium diaminostilbene disulfonate, sodium formate, Liquitint^{™} Orange, dipropylethyl tetraamine, dimethicone, cellulase, a polypeptide having protease activity of the present invention;
- Water, sodium alcoholethoxy sulfate, sodium alkyl sulfate, MEA citrate, linear alkylbenzene sul-fonate, MEA salt, propylene glycol, diethylene glycol, polyethyleneimine ethoxylate, ethanol, sodium fatty acids, ethanolamine, lauramine oxide, Laureth-9, DTPA, sodium cumene sulfonate, sodium formate, calcium formate, linear alkylbenzene sulfonate, sodium salt, alcohol sulfate, sodium hydroxide, diquaternium ethoxysulfate, fragrance, amylase, mannanase, pectinase, disodium diaminostilbene disulfonate, benzisothiazolinone, Liquitint^{™} Blue, dimethicone, dipropylethyl tetraamine, a polypeptide having protease activity of the present invention;
- Water, Sodium alcoholethoxy sulfate, MEA citrate, Sodium Alkyl Sulfate, alcohol ethoxylate, linear alkylbenzene sul-fonate, MEA salt, sodium fatty acids, polyethyleneimine ethoxylate, diethylene glycol, propylene glycol, diquaternium ethoxysulfate, polyethyleneimine, ethoxylate propoxylate, ethanol, sodium cumene sulfonate, fragrance, DTPA, disodium diaminostilbene disulfonate, Mannanase, cellulasesodium formate, calcium formate, Lauramine oxide, Liquitint^{™} Blue, Dimethicone / polydimethyl silicone, a polypeptide having protease activity of the present invention;
- Water, alcoholethoxy sulfate, linear alkylbenzene sulfonate, alcohol ethoxylate, citric acid, Ethanolamine, sodium fatty acids, diethylene glycol, propylene glycol, sodium hydroxide, polyethyleneimine ethoxylate, silicone polyether, ethanol, amylase, sodium cumene sulfonate, diquaternium ethoxysulfate, Laureth-9, fragrance, DTPA, disodium diaminostilbene disulfonate, disodium distyrylbiphenyl disulfonate, sodium formate, calcium formate, mannanase, Liquitint^{™} Orange, dimethicone, polyacrylamide quaternium chloride, cellulase, dipropylethyl tetraamine, a polypeptide having protease activity of the present invention;
- Water, alcoholethoxy sulfate, diethylene glycol, monoethanolamine citrate,
   sodium formate, propylene glycol, linear alkylbenzene sulfonates, ethanolamine, ethanol, polyethyleneimine ethoxylate, amylase, benzisothiazolin, calcium formate, citric acid, diethylenetriamine pentaacetate sodium, dimethicone, diquaternium ethoxysulfate, disodium dia-minostilbene disulfonate, Laureth-9, mannanase, sodium cumene sulfonate, sodium fatty acids, a polypeptide having protease activity of the present invention;
- Water, alcoholethoxy sulfate, MEA Citrate, alcohol sulfate, Alcohol ethoxylate, Linear alkylbenzene sulfonate MEA, sodium fatty acids, polyethyleneimine ethoxylate, diethylene glycol, propylene glycol, diquaternium ethoxysulfate, polyethyleneimine ethoxylate propoxylate, ethanol, sodium cumene sulfonate, fragrance, DTPA, disodium diaminostilbene disulfonate, mannanase, cellulase, amylase, sodium formate, calcium formate, lauramine oxide, Liquitint^{™} Blue, dimethicone, a polypeptide having protease activity of the present invention;
- Water, sodium alcoholethoxy sulfate, MEA Citrate, Linear alkylbenzene sulfonate: sodium salt, Alcohol ethoxylate, Linear alkylbenzene sulfonate: MEA salt, sodium fatty acids, polyethyleneimine ethoxylate, diethylene glycol, propylene glycol, diquatemium ethoxysulfate, amylase, polyethyleneimine ethoxylate propoxylate, ethanol, sodium cumene sulfonate, citric acid, DTPA, disodium diaminostilbene disulfonate, sodium formate, calcium formate, dimethicone, a polypeptide having protease activity of the present invention;
- Water, alcohol ethoxylate sulfate, linear alkylbenzene sulfonate Sodium/Mea salts, propylene glycol, diethylene glycol, sodium formate, ethanol, sodium fatty acids, fragrance, lauramine oxide, DTPA, Polyethylene amine ethoxylate, calcium formate, disodium diaminostilbene disulfonate, dimethicone, tetramine, Liquitint^{™} Blue, a polypeptide having protease activity of the present invention;
- Linear alkylbenzene sulfonates, C₁₂-₁₆ Pareth-9, propylene glycol, alcoholethoxy sulfate, water, polyethyleneimine ethoxylate, glycerine, fatty acid salts, PEG-136 polyvinyl acetate, ethylene Diamine disuccinic salt, monoethanolamine citrate, sodium bisulfite, diethylenetriamine pentaacetate sodium, disodium distyrylbiphenyl disulfonate, calcium formate, mannanase, exyloglucanase, sodium formate, hydrogenated castoroil, natalase, dyes, termamyl, subtilisin, benzisothiazolin, perfume, a polypeptide having protease activity of the present invention;
- Deionized water, Dipropylene Glycol Butyl Ether, Sodium Alkyl Sulfate, Hydrogen Peroxide, Ethanol, Magnesium Sulfate, Alkyl Dimethyl Amine Oxide, Citric Acid, Sodium Hydroxide, Trimethoxy Benzoic Acid, Fragrance, a polypeptide having protease activity of the present invention;
- Water, Alkyl Ethoxylate, Linear Alkylbenzenesulfonate, Hydrogen Peroxide, Diquaternium Ethoxysulfate, Ethanolamine, Disodium Distyrylbiphenyl Disulfonate, tetrabutyl Ethylidinebisphenol, F&DC Yellow 3, Fragrance, a polypeptide having protease activity of the present invention;
- Sodium percarbonate, sodium sulfate, sodium carbonate, sodium aluminosilicate, nonanoyloxy benzene sulfonate, sodium polyacrylate, water, sodium alkylbenzenesulfonate, DTPA, polyethylene glycol, sodium palmitate, amylase, modified starch, FD&C Blue 1, fragrance, a polypeptide having protease activity of the present invention;
- Water, Alkyl Ethoxylate, MEA Borate, Linear Alkylbenzenesulfonate, Propylene Glycol, Diquaternium Ethoxysulfate, Calcium Chlorideenzyme, Ethanolamine, Benzoisothiazolinone, amylase, Sodium Citrate, Sodium Hydroxide, Fragrance, a polypeptide having protease activity of the present invention;
- Water, Alkyl Amine Oxide, Dipropylene Glycol Phenyl Ether, Hydrogen Peroxide, Citric Acid, Ethylene Diamine Disuccinic Acid Sodium salt, Sodium Alkyl Sulfate, Fragrance, an amylase with at least 91% sequence identity to SEQ ID NO: 1, a polypeptide having protease activity of the present invention;
- Sodium bicarbonate, sodium carbonate, sodium percarbonate, alcohol ethoxylate, sodium chloride, maleic/acrylic copolymer, nonanoyloxy benzene sulfonate, sodium sulfate, colorant, diethylenetriamine pentaacetate sodium salt, hydrated aluminosilicate (zeolite), polyethylene glycol, sodium alkylbenzene sulfonate, sodium palmitate, starch, water, fragrance, a polypeptide having protease activity of the present invention;
- Polyvinyl Alcoholpouch film, wherein there is packed a liquid part and a powder part: Liquid Ingredients: Dipropylene Glycol, diquaternium Ethoxysulfate, Water, Glycerin, Liquitint^{™} Orange, a polypeptide having protease activity of the present invention;
- power ingredients: sodium percarbonate, nonanoyloxy benzene sulfonate, sodium carbonate, sodium sulfate, sodium aluminosilicate, sodium polyacrylate, sodium alkylbenzenesulfonate, maleic/acrylic copolymer, water, amylase, polyethylene glycol, sodium palmitate, modified starch, glycerine, DTPA, fragrance, a polypeptide having protease activity of the present invention;
- Water, sodium alcoholethoxy sulfate, linear alkyl benzene sulfonate, sodium/MEA salts, MEA citrate, propylene glycol, polyethyleneimine ethoxylate, ethanol, diethylene glycol, polyethyleneimine propoxyethoxylate, sodium fatty acids, sodium cumene sulfonate, DTPA, fragrance, amylase, disodium diaminostilbene disulfonate, calcium formate, sodium formate, gluoonase, dimethicone, Liquitint^{™} Blue, mannanase, a polypeptide having protease activity of the present invention;
- Sodium Carbonate, Sodium Aluminosilicate, Sodium Sulfate, Linear Alkylbenzene Sulfonate, Bentonite, Water, Sodium Percarbonate, Sodium Polyacrylate, Silicate, Alkyl Sulfate, Nonanoyloxybenzenesulfonate, DTPA, Polyethylene Glycol 4000, Silicone, Ethoxylate, fragrance, Polyethylene Oxide, Palmitic Acid, Disodium Diaminostilbene Disulfonate, Amylase, Liquitint^{™}Red, FD&C Blue 1, Cellulase, a polypeptide having protease activity of the present invention;
- Water, sodium alcoholethoxy sulfate, MEA citrate, linear alkyl benzene sulfonate: sodium/MEA salts, propylene glycol, polyethyleneimine ethoxylate, ethanol, diethylene glycol, polyethyleneimine, propoxyethoxylate, diquaternium ethoxysulfate, alcohol sulfate, dimethicone, fragrance, sodium fatty acids, DTPA, sodium bisulfite, disodium diaminostilbene disulfonate, amylase, gluconase, castor oil, calcium formate, MEA, styrene acrylate copolymer, sodium formate, Liquitint^{™} Blue, a polypeptide having protease activity of the present invention;
- Water, sodium alcoholethoxy sulfate, MEA citrate, linear alkyl benzene sulfonate: sodium/MEA salts, propylene glycol, ethanol, diethylene glycol, polyethyleneimine propoxyethoxylate, polyethyleneimine ethoxylate, alcohol sulfate, dimethicone, fragrance, sodium fatty acids, DTPA, amylase, sodium bisulfite, disodium diaminostilbene disulfonate, castor oil, calcium formate, MEA, styrene acrylate copolymer, propanaminium propanamide, gluconase, sodium formate, Liquitint^{™} Blue, a polypeptide having protease activity of the present invention;
- Water, sodium alcoholethoxy sulfate, MEA citrate, linear alkyl benzene sulfonate: sodium/MEA salts, propylene glycol, polyethyleneimine ethoxylate, ethanol, diethylene glycol, polyethyleneimin propoxyethoxylate, diquaternium ethoxysulfate, alcohol sulfate, dimethicone, fragrance, sodium fatty acids, DTPA, sodium bisulfite, disodium diaminostilbene disulfonate, amylase, gluconase, castor oil, calcium formate, MEA, styrene acrylate copolymer, propanaminium propanamide, sodium formate, Liquitint^{™} Blue, a polypeptide having protease activity of the present invention;
- Sodium Carbonate, Sodium Aluminosilicate, Alkyl Sulfate, Sodium Sulfate, Linear Alkylbenzene Sulfonate, Water, Sodium polyacrylate, Silicate, Ethoxylate, Sodium percarbonate, Polyethylene Glycol 4000, Amylase, Disodium Diaminostilbene Disulfonate, Silicone, Cellulase, a polypeptide having protease activity of the present invention;
- Sodium Carbonate, Sodium Aluminosilicate, Sodium Sulfate, Linear Alkylbenzene Sulfonate, Alkyl Sulfate, Sodium Percarbonate, Water, Sodium Polyacrylate, Silicate, Nonanoyloxybenzenesulfonate, Ethoxylate, Polyethylene Glycol 4000, Fragrance, DTPA, Disodium Diaminostilbene Disulfonate, Palmitic Acid, Amylase, Silicone, Cellulase, a polypeptide having protease activity of the present invention;
- Sodium Carbonate, Sodium Aluminosilicate, Sodium Sulfate, Linear Alkylbenzene Sulfonate, Water, Nonanoyloxybenzenesulfonate, Alkyl Sulfate, Sodium Polyacrylate, Silicate, Sodium Percarbonate, Ethoxylate, Polyethylene Glycol 4000, Fragrance, DTPA, Palmitic Acid, Disodium Diaminostilbene Disulfonate, Amylase, Silicone, Cellulase, a polypeptide having protease activity of the present invention;
- Sodium Carbonate, Sodium Aluminosilicate, Sodium Sulfate, Sodium Percarbonate, Alkyl Sulfate, Linear Alkylbenzene Sulfonate, Water, Nonanoyloxybenzenesulfonate, Sodium Polyacrylate, Silicate, Ethoxylate, Polyethylene Glycol 4000, DTPA, Fragrance, Natalase, Palmitic Acid, Amylase, Disodium, Diaminostilbene Disulfonate, FD&C Blue 1, Silicone, Cellulase, Alkyl Ether Sulfate, a polypeptide having protease activity of the present invention;
- Sodium Carbonate, Sodium Aluminosilicate, Sodium Sulfate, Linear Alkylbenzene Sulfonate, Sodium Percarbonate, Nonanoyloxybenzenesulfonate, Alkyl Sulfate, Water, Silicate, Sodium Polyacrylate, Ethoxylate, Polyethylene Glycol 4000, Fragrance, DTPA, Palmitic Acid, Amylase, Disodium Diaminostilbene Disulfonate, Silicone, FD&C Blue 1, Cellulase, Alkyl Ether Sulfate, a polypeptide having protease activity of the present invention;
- Sodium Carbonate, Sodium Aluminosilicate, Sodium Sulfate, Linear Alkylbenzene Sulfonate, Sodium Percarbonate, Alkyl Sulfate, Water, Sodium Polyacrylate, Silicate, Nonanoyloxybenzenesulfonate, Ethoxylate, Polyethylene Glycol 4000, DTPA, Fragrance, Cellulase, Amylase, Disodium Diaminostilbene Disulfonate, Silicone, FD&C Blue 1, a polypeptide having protease activity of the present invention;
- Water, Sodium alcoholethoxy sulfate, MEA citrate, linear alkylbenzene sulfonate, sodium salt, linear alkylbenzene sulfonate: MEA salt, alcohol ethoxylate, sodium fatty acids, propylene glycol, diethylene glycol, polyethyleneimine ethoxylate propoxylate, diquaternium ethoxysulfate, Ethanol, sodium cumene sulfonate, fragrance, DTPA, Sodium bisulfate, disodium diaminostilbene disulfonate, Mannanase, cellulase, amylase, sodium formate, calcium formate, Lauramine oxide, Liquitint^{™} Blue, Dimethicone / polydimethyl silicone, a polypeptide having protease activity of the present invention;
- Water, sodium alcoholethoxy sulfate, linear alkyl benzene sulfonate: sodium/MEA salts, MEA citrate, propylene glycol, polyethyleneimine ethoxylate, fragrance, ethanol, diethylene glycol, polyethyleneimine propoxyethoxylate, amylase, alcohol sulfate, sodium fatty acids, DTPA, disodium diaminostilbene disulfonate, MEA, mannanase, gluconase, sodium formate, dimethicone, Liquitint^{™} Blue, tetramine, a polypeptide having protease activity of the present invention;
- Water, Sodium alco- holethoxy sulfate, MEA citrate, linear alkylbenzene sulfonate, sodium salt, linear alkylbenzene sulfonate: MEA salt, alcohol ethoxylate, sodium fatty acids, propylene glycol, diethylene glycol, polyethyleneimine ethoxylate propoxylate, diquaternium ethoxysulfate, ethanol, sodium cumene sulfonate, fragrance, DTPA, Sodium bisulfate, disodium diaminostilbene disulfonate, Mannanase, cellulase, amylase, sodium formate, calcium formate, Lauramine oxide, Liquitint^{™} Blue, Dimethicone / polydimethyl silicone, a polypeptide having protease activity of the present invention;
- Sodium Carbonate, Sodium Aluminosilicate, Sodium Sulfate, Linear Alkylbenzene Sulfonate, Sodium Percarbonate, Nonanoyloxybenzenesulfonate, Alkyl Sulfate, Water, Silicate, Sodium Polyacrylate Ethoxylate, Polyethylene Glycol 4000, Fragrance, DTPA, Palmitic Acid, Amylase, Disodium Diaminostilbene Disulfonate, Silicone, FD&C Blue 1, Cellulase, Alkyl Ether Sulfate, a polypeptide having protease activity of the present invention; or
- Aqua, dodecylbenzenesulfonic acid, laureth-11, peg-75 lanolin, propylene glycol, alcohol denat., potassium soyate, potassium hydroxide, disodium cocoamphodiacetate, ethylendiamine triacetate cocosalkyl acetamide, parfum, zinc ricinoleate, sodium chloride, benzisothiazolinone, methylisothiazolinone, ci 16255, benzyl alcohol, a polypeptide having protease activity of the present invention.

Preferably, the formulations listed above including a protease further comprise 4-FPBA and/or a peptide aldhehyde protease inhibitor, most preferably Z-GAY or Z-VAL.

The polypeptide having protease activity of the present invention can be comprised in one of the following detergent compositions.

| Model detergent B | % wt/wt | |
|---|---|---|
| Maranil DBS/LC (LAS) | 9 % | |
| Texapon N 70 (SLES) | 5 % | |
| Dehydol LT 7 (AEO) | 2 % | |
| Coconut fatty acid | 0 % | |
| Sodium Citrate | 0.5 % | |
| Propylene glycol | 0.5 % | |
| Sodium Hydroxide | To adjust pH | |
| H2O | Up to 100 % | |
| pH | 8.0 | |

| ES1-C | | wt% in formulation |
|---|---|---|
| Maranil DBS/LC | linear alkylbenzene sulfonate, anionic surfactant | 5.5 |
| Edenor coco fatty acid | C₁₂-C₁₈ coco fatty acid | 2.4 |
| Lutensol AO7 | alkyl polyethyleneglycol ether, non-ionic surfactant | 5.5 |
| Texapon N70 | sodium laureth sulfate 2EO, anionic surfactant | 5.5 |
| 1,2 propylene glycol | | 6.0 |
| C₂H₅OH | | 2.0 |
| NaOH | | 2.2 |
| Citrate | | 3.0 |
| A formulation comprising the polypeptide having protease activity of the present invention and optionally one or more enzymes, preferably an amylase | | 0.01 - 0.5% |
| pH | | 8-8.5 |
| Water | | up to 100% |

| | % wt in formulation |
|---|---|
| Linear C₁₀C₁₃-alkyl benzene sulfonic acid | 10.0 |
| C₁₂-C₁₄-fatty alcohol ether sulfate (2 EO) | 10.0 |
| C₁₂-C₁₄-fatty alcohol ethoxylate (7EO) | 20.0 |
| C₁₂-C₁₈ coco fattv acid | 5.0 |
| Na-Citrate | 1.5 |
| NaOH 50% | |
| Propylene glycol | 10.0 |
| A formulation comprising the polypeptide having protease activity of the present invention and optionally one or more enzymes, preferably an amylase | 0.01 - 0.5% |
| Water | Ad 100% |
| | |

| | % wt in formulation |
|---|---|
| Linear C₁₀C₁₃-alkyl benzene sulfonic acid | 10.0 |
| C₁₂-C₁₄-fatty alcohol ether sulfate (2 EO) | 10.0 |
| C₁₂-C₁₄-fatty alcohol ethoxylate (7EO) | 20.0 |
| C₁₂-C₁₈ coco fattv acid | 5.0 |
| MGDA | 1.5 |
| NaOH 50% | |
| Propylene glycol | 10.0 |
| A formulation comprising the polypeptide having protease activity of the present invention and optionally one or more enzymes, preferably an amylase | 0.01 - 0.5% |
| Water | Ad 100% |

| | % wt in formulation |
|---|---|
| Linear C₁₀C₁₃-alkyl benzene sulfonic acid | 10.0 |
| C₁₂-C₁₄- fatty alcohol ether sulfate (2 EO) | 10.0 |
| C₁₂-C₁₄-fatty alcohol ethoxylate (7EO) | 20.0 |
| C₁₂-C₁₈ coco fatty acid | 5.0 |
| GLDA | 1.5 |
| NaOH 50% | |
| Propylene glycol | 10.0 |
| A formulation comprising the polypeptide having protease activity of the present invention and optionally one or more enzymes, preferably an amylase | 0.01 - 0.5% |
| Water | Ad 100% |

| | % wt in formulation |
|---|---|
| Linear C₁₀C₁₃-alkyl benzene sulfonic acid | 10.0 |
| C₁₂-C₁₄- fatty alcohol ether sulfate (2 EO) | 10.0 |
| C₁₂-C₁₄-fatty alcohol ethoxylate (7EO) | 20.0 |
| C₁₂-C₁₈ coco fatty acid | 5.0 |
| Phosphonate (e.g., HEDP or DTPMP) | 1.5 |
| NaOH 50% | |
| Propylene glycol | 10.0 |
| A formulation comprising the polypeptide having protease activity of the present invention and optionally one or more enzymes, preferably an amvlas | 0.01 - 0.5% |
| Water | Ad 100% |

| **% wt in formulation** | | | | | | | | | | | | |
|---|---|---|---|---|---|---|---|---|---|---|---|---|
| **Alcohol ethoxylate 7EO** | 0.6 | 0 | 1 | 0 | 0 | 5.2 | 1.2 | 5 | 4 | 0.5 | 0.5 | 0 |
| **Coco fatty acid C₁₂-₁₈** | 1.2 | 0 | 0 | 0 | 0 | 0 | 0 | 0 | 0 | 0.3 | 0 | 0.6 |
| **C₁₂-C₁₄-fatty alcohol ether sulfate (2 EO)** | 1.5 | 0 | 0 | 0 | 0 | 6 | 0 | 3.9 | 4.4 | 1.6 | 0 | 0 |
| **Linear alkyl benzene sulphonic acid** | 12.1 | 11.2 | 13.6 | 21.9 | 18.7 | 12.7 | 7.6 | 12.1 | 11.5 | 12.2 | 6.5 | 10.4 |
| **Bleach activator** | 0 | 0 | 0 | 0 | 0 | 0 | 0.2 | 9.5 | 9.5 | 0.5 | 0.8 | 2.2 |
| **Percarbonate** | 0 | 0 | 0 | 0 | 0 | 0 | 3.6 | 19.4 | 16.6 | 2.2 | 11.5 | 5.8 |
| **AcetateNa** | 0 | 0 | 0 | 0.1 | 0 | 0.1 | 0 | 6.7 | 7.1 | 0.3 | 1 | 0.7 |
| **CitrateNa** | 0 | 0 | 0 | 0 | 0 | 14 | 0 | 1.6 | 8.2 | 0.3 | 0.9 | 1.7 |
| **Na Silicate** | 27.9 | 5.8 | 6.6 | 2 | 15 | 20.3 | 3.6 | 11.3 | 16.4 | 10.2 | 9.1 | 16.5 |
| **Na Carbonate** | 17.2 | 35 | 37.3 | 30.1 | 37 | 1 | 21.6 | 8.7 | 1.4 | 8 | 22.9 | 14.8 |
| **Na Phosphate** | 0 | 0 | 0 | 14 | 0.3 | 0 | 0 | 0 | 0 | 0 | 0 | 0 |
| **Na Hydrogencarbonate** | 0.7 | 0.9 | 0.5 | 2.7 | 0.4 | 10.5 | 0.2 | 2.8 | 1.6 | 0.8 | 0.5 | 0.5 |
| **Zeolite4A** | 4.2 | 0.1 | 5.1 | 10.2 | 1.8 | 11.6 | 1.6 | 1.4 | 2.4 | 1.6 | 1.8 | 2.3 |
| **HEDP** | 0 | 0 | 0 | 0 | 0 | 0.13 | 0 | 0.27 | 0.16 | 0 | 0 | 0.17 |
| **MGDA** | 0 | 1.1 | 0 | 0 | 0 | 0 | 0 | 0 | 0 | 0 | 0 | 0 |
| **Cellulase** | 0 - 0.5 | 0 - 0.5 | 0 - 0.5 | 0 - 0.5 | 0 - 0.5 | 0 - 0.5 | 0 - 0.5 | 0 - 0.5 | 0 - 0.5 | 0 - 0.5 | 0 - 0.5 | 0 - 0.5 |
| **Lipase** | 0 - 0.4 | 0 - 0.4 | 0 - 0.4 | 0 - 0.4 | 0 - 0.4 | 0 - 0.4 | 0 - 0.4 | 0 - 0.4 | 0 - 0.4 | 0 - 0.4 | 0 - 0.4 | 0 - 0.4 |
| **Mannanase** | 0 - 0.4 | 0 - 0.4 | 0 - 0.4 | 0 - 0.4 | 0 - 0.4 | 0 - 0.4 | 0 - 0.4 | 0 - 0.4 | 0 - 0.4 | 0 - 0.4 | 0 - 0.4 | 0 - 0.4 |
| **Protease *** | 0 - 1.5 | 0 - 1.5 | 0 - 1.5 | 0 - 1.5 | 0 - 1.5 | 0 - 1.5 | 0 - 1.5 | 0 - 1.5 | 0 - 1.5 | 0 - 1.5 | 0 - 1.5 | 0 - 1.5 |
| **Amylase** | 0 - 0.5 | 0 - 0.5 | 0 - 0.5 | 0 - 0.5 | 0 - 0.5 | 0 - 0.5 | 0 - 0.5 | 0 - 0.5 | 0 - 0.5 | 0 - 0.5 | 0 - 0.5 | 0 - 0.5 |
| **Na Sulfate** | 30.8 | 1.3 | 33 | 11 | 22 | 3 | 51 | 4 | 6 | 57 | 38 | 37 |
| **Na Chloride** | 0.2 | 43 | 0.1 | 0 | 0.1 | 0.1 | 1 | 1 | 0.5 | 1.2 | 0.2 | 1 |
| **optical brightener** | 0.02 | 0 | | 0.1 | 0.06 | | | 0.29 | 0.1 | 0.23 | 0.13 | 0.19 |
| **Polymers** | 1 | 0 | 0.2 | 2 | 0.5 | 3 | 2.2 | 9.2 | 2.2 | 0.7 | | 0.4 |

| | | | | | | | | | | | | |
|---|---|---|---|---|---|---|---|---|---|---|---|---|
| *) a formulation comprising the polypeptide having protease activity of the present invention; the formulation can further comprise a protease inhibitor, preferably selected from phenylboronic acid (preferably 4-FPBA) or a peptide aldehyde or a bisulfite adduct or acetal thereof (preferably a tripeptide aldehyde, preferably, Z-GAY-H or Z-VAL-H). | | | | | | | | | | | | |

| **% wt in formulation** | | | | | | | | | | | | | | |
|---|---|---|---|---|---|---|---|---|---|---|---|---|---|---|
| **alcohol ethoxylate 7EO** | 5,40 | 10,80 | 12,40 | 7,30 | 1,60 | 7,60 | 3,80 | 0,30 | 13,30 | 8,00 | 5,70 | 20,00 | 9,20 | 29,00 |
| **Coco fatty acid C₁₂-₁₈** | 2,40 | 3,10 | 3,20 | 3,20 | 3,50 | 6,40 | 2,80 | 3,00 | 1,70 | 1,80 | 2,50 | 5,00 | 8,60 | 10,40 |
| **C₁₂-C₁₄-fatty alcohol ether sulfate (2 EO)** | 5,40 | 8,80 | 7,10 | 7,10 | 5,40 | 14,00 | 2,80 | 4,50 | 3,90 | 4,10 | | 10,00 | 22,20 | |
| **Linear alkyl benzene sulphonic acid** | 5,50 | 0,00 | 14,50 | 15,50 | 10,70 | 0,00 | 6,30 | 5,43 | 11,45 | 5,90 | 10,10 | 10,00 | 28,00 | 27,00 |
| **1,2 Propanediol** | 6,00 | 3,50 | 8,70 | 8,70 | 1,10 | 7,80 | 0,50 | | 2,50 | 0,40 | 6,00 | 10,00 | 7,00 | 7,00 |
| **Triethanolamine** | | | | | | | | | | 8,10 | | | | |
| **Monoethanolamine** | | | 4,00 | 4,30 | 0,30 | | 0,40 | 1,80 | | | | | 8,00 | 7,00 |
| **NaOH** | 2,20 | 1,10 | | | | 1,00 | | | 2,20 | | 3,30 | 1,50 | | |
| **Glycerol** | | 0,80 | 3,00 | 2,80 | | | | 0,60 | 0,20 | 1,90 | | | 7,00 | 10,00 |
| **Ethanol** | 2,00 | | | | 0,38 | 0,39 | | | 1,84 | | | | | |
| **Na citrate** | 3,00 | 2,80 | 3,40 | 2,10 | 7,40 | 5,40 | 4,60 | 3,30 | 3,30 | 1,40 | | 1,50 | | |
| **Polymer** | 0 - 5% | 0 - 5 % | 0 - 5 % | 0 - 5 % | 0 - 5 % | 0 - 5 % | 0 - 5 % | 0 - 5 % | 0 - 5 % | 0 - 5 % | 0 - 5 % | 0 - 5 % | 0 - 5 % | 0 - 5 % |
| **Protease *** | 0 - 1 % | 0 - 1 % | 0 - 1 % | 0 - 1 % | 0 - 1 % | 0 - 1 % | 0 - 1 % | 0 - 1 % | 0 - 1 % | 0 - 1 % | 0 - 1 % | 0 - 3 % | 0 - 3 % | 0 - 3 % |
| **Amylase** | 0 - 0,5 % | 0 - 0,5 % | 0 - 0,5 % | 0 - 0,5 % | 0 - 0,5 % | 0 - 0,5 % | 0 - 0,5 % | 0 - 0,5 % | 0 - 0,5 % | 0 - 0,5 % | 0 - 0,5 % | 0 - 0,5 % | 0 - 0, 5 % | 0 - 0,5 % |
| **Cellulase** | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % |
| **Lipase** | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % |
| **Mannanase** | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % | 0 - 0,2 % |
| **Pectate Lyase** | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % | 0 - 0,3 % |
| **water** | **to 100** | **to 100** | **to 100** | **to 100** | **to 100** | **to 100** | **to 100** | **to 100** | **to 100** | **to 100** | **to 100** | **to 100** | **to 100** | **to 100** |

| | | | | | | | | | | | | | | |
|---|---|---|---|---|---|---|---|---|---|---|---|---|---|---|
| *) a formulation comprising the polypeptide having protease activity of the present invention, preferably the formulation comprises a protease inhibitor, preferably selected from phenylboronic acid (preferably 4-FPBA) or a peptide aldehyde or a bisulfite adduct or acetal thereof (preferably a tripeptide aldehyde, preferably, Z-GAY-H or Z-VAL-H). | | | | | | | | | | | | | | |

### Compositions with antimicrobial agents

An antimicrobial agent is a chemical compound that kills microorganisms or inhibits their growth or reproduction. Microorganisms can be bacteria, yeasts or molds. A preservative is an antimicrobial agent which may be added to aqueous products and compositions to maintain the original performance, characteristics and integrity of the products and compositions by killing contaminating microorganisms or inhibiting their growth.

The composition/formulation comprising the polypeptide having protease activity of the present invention may contain one or more antimicrobial agents and/or preservatives as listed in patent WO 2021/115912 A1 ("Formulations comprising a hydrophobically modified polyethyleneimine and one or more enzymes") on pages 35 to 39.

Especially of interest for the cleaning compositions and fabric and home care products and specifically in the laundry formulations are any of the following antimicrobial agents and/or preservatives:
4,4'-dichloro 2-hydroxydiphenyl ether (further names: 5-chloro-2-(4-chlorophenoxy) phenol, Diclosan, DCPP), Tinosan^{®} HP 100 (30wt.% of DCPP in in 1,2-propylene glycol); 2-Phenoxyethanol (further names: Phenoxyethanol, Methylphenylglycol, Phenoxetol, ethylene glycol phenyl ether, Ethylene glycol monophenyl ether, 2-(phenoxy) ethanol, 2-phenoxy-1-ethanol); 2-bromo-2-nitropropane-1,3-diol (further names: 2-bromo-2-nitro-1,3-propanediol); Glutaraldehyde (further names: 1-5-pentandial, pentane-1,5-dial, glutaral, glutardialdehyde); Glyoxal (further names: ethandial, oxylaldehyde, 1,2-ethandial); 5-bromo-5-nitro-1,3-dioxane (further names: 5-bromo-5-nitro-m-dioxane);Phenoxypropanol (further names: propylene glycol phenyl ether, phenoxyisopropanol 1-phenoxy-2-propanol, 2-phenoxy-1-propanol); Glucoprotamine (chemical description: reaction product of glutamic acid and alkylpropylenediamine, further names: Glucoprotamine 50); Cyclohexyl hydroxyl diazenium-1-oxide, potassium salt (further names: N-cyclohexyl-diazenium dioxide, Potassium HDO, Xyligene);Formic acid (further names: methanoic acid) and its salts, e.g. sodium formiate);Tetrahydro-3,5-dimethyl-1,3,5-thiadia-zine-2-thione (further names: 3,5-dimethyl-1,3-5-thiadiazinane-2-thi-one, Dazomet; 2,4-dichlorobenzyl alcohol (further names: dichlorobenzyl alcohol, 2,4-dichloro-benzenemethanol, (2,4-dichloro-phenyl)-methanol, DCBA); 1-propanol (further names: n-propanol, propan-1-ol, n-propyl alcohol; 1,3,5-Tris-(2-hydroxyethyl)-hexahydro-1,3,5-triazin (further names: Hexayhydrotriazine, Tris(hydroethyl)-hexyhydrotriazin, hexyhydro-1,3-5-tris(2-hydroxyethyl)-s-triazine, 2,2',2"-(hexahydro-1,3,5-triazine-1,3,5- triyl)triethanol; 2-butyl-benzo[d]isothiazol-3-one ("BBIT"); 2-methyl-2H-isothiazol-3-one ("MIT""); 2-octyl-2H-isothiazol-3-one ("OIT"); 5-Chloro-2-methyl-2H-isothiazol-3-one ("CIT" or "CMIT"); Mixture of 5-chloro-2-methyl-2H- isothiazol-3-one ("CMIT") and 2-methyl-2H-isothiazol-3-one ("MIT") (Mixture of CMIT/MIT); 1,2-benzisothiazol-3(2H)-one ("BIT"); Hexa-2,4-dienoic acid (trivial name "sorbic acid") and its salts, e.g., calcium sorbate, sodium sorbate; potassium (E,E)-hexa-2,4-dienoate (Potassium Sorbate); Lactic acid and its salts; L-(+)-lactic acid; especially sodium lactate; Benzoic acid and salts of benzoic acid, e.g., sodium benzoate, ammonium benzo-ate, calcium benzoate, magnesium benzoate, MEA-benzoate, potassium benzoate; Salicylic acid and its salts, e.g., calcium salicylate, magnesium salicylate, MEA salicylate, sodium salicylate, potassium salicylate, TEA salicylate; Benzalkonium chloride, benzalkonium bromide, benzalkonium sac-charinate; Didecyldimethylammonium chloride ("DDAC"); N-(3-aminopropyl)-N-dodecylpropane-1,3-diamine ("Diamine"); Peracetic acid; Hydrogen peroxide.

At least one antimicrobial agent or preservative may be added to the inventive composition in a concentration of 0.001 to 10% relative to the total weight of the composition.

Preferably, the composition contains 2-phenoxyethanol in a concentration of 0.1 to 2% or 4,4'-dichloro 2-hydroxydiphenyl ether (DCPP) in a concentration of 0.005 to 0.6%.

The invention also encompasses a method of preserving an aqueous composition according to the invention against microbial contamination or growth, which method comprises addition of at least one antimicrobial agent or preservative, preferably 2-phenoxyethanol.

The invention also encompasses a method of providing an antimicrobial effect on textiles after treatment with a solid laundry detergent (e.g. powders, granulates, capsules, tablets, bars etc.), a liquid laundry detergent, a softener or an after-rinse containing 4,4'-dichloro 2-hydroxydiphenyl ether (DCPP).

### Methods of use

The polypeptide having protease activity of the present invention can be used in various applications.

The polypeptide having protease activity of the present invention or a composition comprising said polypeptide having protease activity of the present invention may be used in cleaning, food processing, animal feed, pulp and paper processing, baking, mining and oil well service, textile processing, leather processing, water treatment, brewery, ethanol production, circular economy, waste treatment, or recycling.

The present invention is also directed to the use of a polypeptide having protease activity of the present invention in a cleaning process such as laundry or hard surface cleaning, preferably for home care or I&I cleaning. The polypeptide having protease activity of the present invention or a composition comprising said polypeptide may be used in various industrial and institutional cleaning applications, including commercial laundry such as on premise laundry or tun-nelwashing, mechanical ware wash such as in a hood machine or in a tunnelwasher, manual dish wash cleaning, carpet cleaning, open plant cleaning (outside pipe cleaning), cleaning in place (inside pipe cleaning), membrane cleaning such as in dairy, food, beverage or water treatment, vehicle care such as pad cleaning, microorganism removal, virus removal, insect removal or malodor removal or veterinary cleaning. The polypeptide having protease activity of the present invention or a composition comprising said polypeptide may also be used in textile processing, leather processing or water treatment.

The polypeptide having protease activity of the present invention or a composition comprising said polypeptide may be used in the bioenergy industry, in particular in bioethanol production, oil and gas recovery, in particular liquefaction for improved oil recovery and food processing, in particular beverage production and/or processing.

The present invention also refers to the use of a polypeptide having protease activity of the present invention for providing a detergent composition with improved protease stability and/or for providing a detergent composition with improved wash performance, preferably on protease sensitive stains.

Thus, the present invention therefore also refers to a method for cleaning, preferably laundry or hard surface cleaning, comprising the step of contacting a subject, preferably a textile or a hard surface, with a composition comprising a polypeptide having protease activity of the present invention, preferably wherein the composition comprises at least one additional detergent component, preferably a surfactant and/or a builder.

Further, the present invention also refers to a method for improving protease stability in a detergent composition and/or for improving wash performance of a detergent composition, preferably on protease-sensitive stains comprising the step of formulating a p polypeptide having protease activity of the present invention in a detergent composition.

The present invention also refers to a method of laundering fabric or of cleaning hard surfaces, which method comprises treating a fabric or a hard surface with a composition comprising the polypeptide having protease activity of the present invention and further comprising 4,4'-dichloro 2-hydroxydiphenylether.

In one embodiment, the polypeptide having protease activity of the present invention is used to improve the sustainability profile of a composition or a method and/or are used in circular economy. With respect to the use in circular economy, the polypeptide having protease activity of the present invention can be used in waste treatment or recycling. In one embodiment the present invention is directed to a process for waste treatment, preferably for solubilizing waste, comprising the step of contacting waste with a polypeptide having protease activity of the present invention and preferably with one or more enzymes selected from the group consisting of lipase, glucanase, amylase, pectate lyase, and mannanase, under conditions supporting enzymatic solubilizations of the waste. In one embodiment, the waste is municipal solid waste. In one embodiment, the liquified waste can be used as substrate for microbial fermentation.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims. The detailed description is merely exemplary in nature and is not intended to limit application and uses. The following examples further illustrate the present invention without, however, limiting the scope of the invention thereto. Various changes and modifications can be made by those skilled in the art on the basis of the description of the invention, and such changes and modifications are also included in the present invention.

### Examples

### Material and Methods

### 1. Library Generation

Genes encoding proteases were cloned by restriction-ligation based standard protocols into a gram-positive expression vector comprising a promoter sequence, a sequence coding for a secretion signal peptide, and a ribosome binding site. Post reaction, the plasmid assembly mixtures were transformed into *B*. *subtilis* PY79 by established natural-competency transformation methods. Successful transformations were selected by plating on LB agar plates supplemented with 20 µg/ml kanamycin sulfate and incubating overnight at 37 °C. After overnight selection, individual colonies were grown in TB medium with 20 µg/ml kanamycin sulfate overnight at 35 °C with shaking at 1000 rpm with a throw of 3 mm. Cells were then pelleted by centrifugation, and plasmid DNA was isolated by alkaline lysis method with a Qiagen QlAprep Spin Miniprep Kit. The isolated DNA was transformed into electrocompetent *Bacillus licheniformis* cells. As an example, the cells were prepared by growing the *B*. *licheniformis* strain in an osmolyte-dense rich medium (LB broth with 2 M sucrose) and harvesting the cells in early exponential growth phase. Cells were harvested by chilling on ice and pelleting by centrifugation. Following the harvest, cells were washed to remove salts with EP washing buffer (1 mM MgCl₂, 7mM KH₂PO₄, 500 mM trehalose dihydrate, and 272 mM sucrose) by 3 cycles of suspension and pelleting by centrifugation. Finally, cells were concentrated by resuspending in the washing buffer at 1 to 10% of the original culture volume. Once prepared, plasmid DNA was added to the *B*. *licheniformis* electrocompetent cells in a 0.2 cm Bio-Rad electroporation cuvette. The cells were electroporated with a Bio-Rad Gene Pulser Xcell per manufacturer's instructions. Cells were immediately rescued after the shock by addition of 1 ml of the osmolyte-dense rich medium. After two hours of recovering at 37 °C, the successful transformants were then selected by plating on LB agar plates supplemented with 20 µg/ml kanamycin sulfate and incubating overnight at 37 °C.

### 2. Preparation and expression of variants

Single colonies of the expression strains were picked into 600 µL of rich medium (TB broth) supplemented with 20 µg/mL kanamycin sulfate in a 96-well plate. The cultures were grown at 30 °C with shaking at 1000 rpm for 16 hours, then 6 µL of culture was used to inoculate 600 µL of defined glucose-mineral media as defined in the Tables below with 20 µg/mL kanamycin sulfate in a 96 well plate.

**Table 2: Composition of initial fermentation medium.**

| **Compound** | **Formula** | **Concentration [g/L]** |
|---|---|---|
| Citric acid | C₆H₈O₇ | 3.0 |
| Calcium carbonate | CaCO₃ | 0.04 |
| Monopotassium phosphate | KH₂PO₄ | 4 |
| Ammonium sulfate | (NH4)₂SO₄ | 15 |
| Trace element solution | (Table 3) | 1 |
| Magnesium sulfate | MgSO₄ | 1 |
| Sodium hydroxide | NaOH | 1.0 |
| MOPS | C₇H₁₅NO₄S | 84 |
| Glucose | C₆H₁₂O₆ | 5 |
| Raffinose | C₁₈H₃₂O₁₆ | 20 |

**Table 3: Trace element composition of the trace element solution comprising 40 g/L citric acid**

| **Trace element** | **Symbol** | **Concentration [mM]** |
|---|---|---|
| Manganese | Mn | 24 |
| Zinc | Zn | 17 |
| Copper | Cu | 32 |
| Cobalt | Co | 1 |
| Nickel | Ni | 2 |
| Molybdenum | Mo | 0.2 |
| Iron | Fe | 38 |

The cultures were grown at 30 °C with shaking at 1000 rpm with a throw of 3 mm for 48 hours, after which the supernatant was harvested by pelleting the cells by centrifugation and removing the residual culture liquid. The harvested supernatant is referred to as the enzyme solution and had a protease concentration in the range of 0.5-1.5 g/L.

### 3. Protease Activity Assays

Proteolytic activity was determined by using a Suc-AAPF-AMC substrate (Bachem, Product Number: 4012873). Suc-AAPF-AMC is an abbreviation for N-Succinyl-Alanine-Alanine-Proline-Phenylalanine-7-amino-4-methylcoumarin, a blocked peptide which can be cleaved by endo-proteases. Following proteolytic cleavage, free AMC molecules are liberated and measured by fluorescence spectrophotometry at an excitation wavelength of 360 nm and emission wavelength of 448 nm. The slope of the time-dependent fluorescent signal increase (Vₘₐₓ) is proportional to the amount of protease in the solution and the specific activity of the protease in question (activity per mg enzyme) under the given set of conditions. Protease samples were diluted in assay buffer (100 mM Tris-HCl, 0.1% Brij-35, pH 8.6) or in a detergent challenge solution prior to activity assessment, for example reaching a pre-assay dilution between 400-800 fold. The assay was conducted by transferring 10 µL of diluted enzyme samples to a 384 well microtiter plate containing 40 µL substrate working solution or by transferring 20 uL of diluted enzyme sample to a 96 well microtiter plate containing 80 µL substrate working solution. The solution was mixed at room temperature and the fluorescence signal was measured every minute over 15 minutes with an excitation wavelength of 360nm and emission wavelength of 448 nm using a fluorescence-capable standard plate reader (BioTek Synergy Neo^{®} or Neo2^{®}) The substrate conversion rates (Vₘₐₓ) were calculated for each sample and the residual activity was calculated by dividing the activity after storage time with the activity of the sample at time point zero.

### Example 1: Storage stability assessment of protease variants in model detergent

Novel protease variants were generated by introduction of mutations into SEQ ID NO: 43 using a combinatorial approach and the library was expressed in 96DWP as described in Materials and Methods. To assess the detergent stability, the expressed protease supernatants were diluted into model B liquid detergent (see Table 5) under the condition described in Table 4 and subjected to thorough mixing. The solution was equilibrated for 1 hour, and then measured for activity with the Suc-AAPF-AMC assay as described in the Materials and Methods section. This was considered time zero (unstressed sample). The sample-containing detergent plate was then sealed and placed at elevated temperatures (45°C) for the storage stability assessment. After incubation for one day (18 hours), the protease activity of the stressed sample was measured and compared to the unstressed control to compute the residual activity, which is defined as the activity after storage at 45°C divided by the activity at time point zero. The residual activity given in Table 6 below is the residual activity in detergent.SEQ ID NO: 41 was included as a reference control. The combinatorial library based on SEQ ID NO: 43 generated variants containing 19-26 mutations compared to SEQ ID NO: 41 and 8-15 mutations compared to SEQ ID NO: 43 at various selected positions. All mutants had significantly better stability compared to their parent sequences (SEQ ID NO: 41 and 43) as demonstrated in Table 6.

**Table 4: Challenge condition for stability studies**

| **Condition** | **Component** | **Percent** |
|---|---|---|
| Condition 1 | Detergent | 90% (v/v) |
| | Calcium Ions | 0.000055% (wt/v) |
| | Enzyme Solution | 10% (v/v) |

**Table 5: Model B detergent for stability studies**

| **Component** | **Percent wt/wt** |
|---|---|
| Maranil DBS/LC (LAS) | 9 % |
| Texapon N 70 (SLES) | 5 % |
| Dehydol LT 7 (AEO) | 2 % |
| Coconut fatty acid | 0 % |
| Sodium Citrate | 0.5 % |
| Propylene glycol | 0.5 % |
| Sodium Hydroxide | To adjust pH |
| H2O | Up to 100 % |
| pH | 8.0 |

**Table 6: Storage stability of novel variants in Model B detergent**

| **SEQ ID** | **Residual Activity** |
|---|---|
| SEQ ID NO: 41 | 0.013 |
| SEQ ID NO: 43 | 0.078 |
| SEQ ID NO: 1 | 0.871 |
| SEQ ID NO: 2 | 0.849 |
| SEQ ID NO: 3 | 0.887 |
| SEQ ID NO: 4 | 1.049 |
| SEQ ID NO: 5 | 0.873 |
| SEQ ID NO: 6 | 0.754 |
| SEQ ID NO: 7 | 0.832 |
| SEQ ID NO: 8 | 0.892 |
| SEQ ID NO: 9 | 0.835 |
| SEQ ID NO: 10 | 0.752 |
| SEQ ID NO: 11 | 0.810 |
| SEQ ID NO: 12 | 0.869 |
| SEQ ID NO: 13 | 0.783 |
| SEQ ID NO: 14 | 0.821 |
| SEQ ID NO: 15 | 0.369 |
| SEQ ID NO: 16 | 0.914 |
| SEQ ID NO: 17 | 0.776 |
| SEQ ID NO: 18 | 0.835 |
| SEQ ID NO: 19 | 0.835 |
| SEQ ID NO: 20 | 0.920 |
| SEQ ID NO: 21 | 0.934 |
| SEQ ID NO: 22 | 0.806 |
| SEQ ID NO: 23 | 0.871 |
| SEQ ID NO: 24 | 0.848 |
| SEQ ID NO: 25 | 0.834 |
| SEQ ID NO: 26 | 0.910 |
| SEQ ID NO: 27 | 0.811 |
| SEQ ID NO: 28 | 0.785 |
| SEQ ID NO: 29 | 0.878 |
| SEQ ID NO: 30 | 0.951 |
| SEQ ID NO: 31 | 0.890 |
| SEQ ID NO: 32 | 0.888 |
| SEQ ID NO: 33 | 0.918 |
| SEQ ID NO: 34 | 0.892 |
| SEQ ID NO: 35 | 0.936 |
| SEQ ID NO: 36 | 0.921 |
| SEQ ID NO: 37 | 0.838 |
| SEQ ID NO: 38 | 0.853 |
| SEQ ID NO: 39 | 0.785 |
| SEQ ID NO: 40 | 0.680 |
| SEQ ID NO: 46 | 0.794 |
| SEQ ID NO: 47 | 0.428 |
| SEQ ID NO: 48 | 0.776 |
| SEQ ID NO: 49 | 0.421 |
| SEQ ID NO: 50 | 0.680 |
| SEQ ID NO: 51 | 0.689 |
| SEQ ID NO: 52 | 0.861 |
| SEQ ID NO: 53 | 0.455 |
| SEQ ID NO: 54 | 0.562 |
| SEQ ID NO: 55 | 0.542 |
| SEQ ID NO: 56 | 0.775 |
| SEQ ID NO: 57 | 0.615 |

### Example 3: Stable detergent formulation

Liquid laundry detergent formulations are prepared with or without (comparative) 0.5% by weight of the variant polypeptide of the present invention and either 0.2 % of the biocide Tinosan^{®} HP 100 (from BASF SE) or 1% 2-phenoxyethanol (Protectol^{®} PE, BASF SE). The formulations are prepared by first preparing a premix, containing the AEO und AES surfactants, the solvents 1,2-propanediol and ethanol and, where relevant, Tinosan^{®} HP 100 or 2-phenoxyethanol. This premix is stirred at room temperature to form a homogeneous mixture. Then, LAS, fatty acid and citric acid, as shown in Table 7, and water up to 90% are added. Subsequently, the pH is adjusted to pH=8.5 using NaOH. Then the final formulations are prepared by stirring at room temperature: 90% of this obtained mixture, 0.5% of the polypeptide of the present invention and water up 100%.

Compositions and results are shown in Table 7.

**Table 7**

| **Ingredient** | **Formulation A (comparative)** | **Formulation B (comparative)** | **Formulation C (inventive)** | **Formulation D (inventive)** |
|---|---|---|---|---|
| AEO | 5.4% | 5.4% | 5.4% | 5.4% |
| AES | 7.7% | 7.7% | 7.7% | 7.7% |
| LAS | 5.5% | 5.5% | 5.5% | 5.5% |
| Coco-fatty acid | 2.4% | 2.4% | 2.4% | 2.4% |
| citric acid | 3% | 3% | 3% | 3% |
| 1,2-propanediol | 6% | 6% | 6% | 6% |
| ethanol | 2% | 2% | 2% | 2% |
| Variant polypeptide of the present invention | - | - | 0.5% | 0.5% |
| Tinosan^{®} HP 100 | 0.2% | | 0.2% | |
| 2-Phenoxyethanol | - | 1% | | 1% |
| NaOH | Up to pH=8.5 | Up to pH=8.5 | Up to pH=8.5 | Up to pH=8.5 |
| Water | Up to 100% | Up to 100% | Up to 100% | Up to 100% |
| **Observations / measurements** | **Observations / measurements** | **Observations / measurements** | **Observations / measurements** | **Observations / measurements** |
| Appearance | Homogeneous slightly yellow, viscous liquid | Homogeneous slightly yellow, viscous liquid | Homogeneous slightly yellow, viscous liquid | Homogeneous slightly yellow, viscous liquid |
| Solubility / dispersibility in water at 4g/L deionized water | Foaming, homogeneous aqueous mixture | Foaming, homogeneous aqueous mixture | Foaming, homogeneous aqueous mixture | Foaming, homogeneous aqueous mixture |

| | | | | |
|---|---|---|---|---|
| AEO: C₁₃/C₁₅ Oxo-alcohol (7EO) Lutensol^{®} AO7 (BASF SE) (CAS 68002-97-1) AES: C₁₂/C₁₄-Fatty alcohol ethersulfate (2EO), sodium salt: Texapon^{®} N 70 (BASF SE) (CAS 68891-38-3) LAS: Linear alkylbenzene sulfonic acid Maranil^{®} DBS/LC (BASF SE) (CAS 85536-14-7) Coco fatty acid: Edenor^{®} K12-18 (Emery Oleochemicals) (CAS 90990-15-1) 1,2-propanediol: racemic mixture (CAS 57-55-6) Tinosan^{®} HP 100 is a commercial product from BASF SE, containing 30% of the antimicrobial active 4,4'-dichloro-2-hydroxydiphenylether (CAS 3380-30-1) in 1,2-propyleneglycol (CAS 122-99-6). 2-phenoxyethanol is available from BASF SE as Protectol^{®} PE | | | | |

In the table above the concentrations of the surfactant trade products are given.

It is clear from the above table, that the polypeptide of the present invention and Tinosan HP 100 or 2-phenoxyethanol can be combined in a liquid laundry formulation.

## Claims

1. A polypeptide having protease activity or a functional fragment of said polypeptide having protease activity, wherein the polypeptide has an amino acid sequence which is at least 95% identical to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56 and SEQ ID NO: 57 wherein the functional fragment comprises 100 to 259 consecutive amino acids of the full-length polypeptide.

2. The polypeptide of claim 1, comprising at least one amino acid substitution at a position selected from the group consisting of: 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

3. The polypeptide of claim 1 or 2, comprising an amino acid substitution at positions 18, 38, 78, 183, 204 and 218 compared to the amino acid sequence as set forth in SEQ ID NO: 41 and referring to the numbering of SEQ ID NO: 42.

4. The polypeptide of any one of the preceding claims, comprising an amino acid substitution at positions 18, 21, 24, 38, 56, 78, 109, 144, 160, 182, 183, 204, 210, 218, 228 and 248 compared to the amino acid sequence as set forth in SEQ ID NO: 41 and referring to the numbering of SEQ ID NO: 42.

5. The polypeptide of claim 2 or 4, wherein:
(a) the amino acid substitution at amino acid residue 18 is X18Q/E/D; and/or
(b) the amino acid substitution at amino acid residue 21 is X21I/V/F/W/Y, preferably is X21I/V; and/or
(c) the amino acid substitution at amino acid residue 24 is X24K; and/or
(d) the amino acid substitution at amino acid residue 38 is X38R/K/H/W; and/or
(e) the amino acid substitution at amino acid residue 56 is X56D; and/or
(f) the amino acid substitution at amino acid residue 78 is X78N/D/R/W/F/H/K/E/L/Y/M/C/Q, preferably is X78N/D; and/or
(g) the amino acid substitution at amino acid residue 109 is X109K/A; and/or
(h) the amino acid substitution at amino acid residue 144 is X144N/R; and/or
(i) the amino acid substitution at amino acid residue 160 is X160H/S/D/E/P, preferably is X160H; and/or
(j) the amino acid substitution at amino acid residue 182 is X182K/R/E; and/or
(k) the amino acid substitution at amino acid residue 183 is X183D/E/C/Q/A/M, preferably is X183D/E; and/or
(l) the amino acid substitution at amino acid residue 204 is X204D/E/C/G, preferably is X204D/E; and/or
(m) the amino acid substitution at amino acid residue 210 is X210I/V, preferably is X210I; and/or
(n) the amino acid substitution at amino acid residue 218 is X218T/S/D, preferably is X218S/T; and/or
(o) the amino acid substitution at amino acid residue 228 is X228S; and/or
(p) the amino acid substitution at amino acid residue 248 is X248Q/R.

6. The polypeptide of claim 3, wherein:
(a) the amino acid substitution at amino acid residue 18 is X18Q/E/D; and/or
(b) the amino acid substitution at amino acid residue 38 is X38R/K/H/W; and/or
(c) the amino acid substitution at amino acid residue 78 is X78N/D/R/W/F/H/K/E/L/Y/M/C/Q, preferably is X78N/D; and/or
(d) the amino acid substitution at amino acid residue 183 is X183D/E/C/Q/A/M, preferably is X183D/E; and/or
(e) the amino acid substitution at amino acid residue 204 is X204D/E/C/G, preferably is X204D/E; and/or
(f) the amino acid substitution at amino acid residue 218 is X218T/S/D, preferably is X218S/T.

7. The polypeptide of any one of the preceding claims, wherein said polypeptide comprises amino acid residue D or E at position 101, preferably E at position 101, referring to the numbering of SEQ ID NO: 42.

8. The polypeptide of any one of the preceding claims, wherein the polypeptide exhibits one or more improved properties compared to the protease according to SEQ ID NO: 41 or SEQ ID NO: 43, preferably compared to the protease according to SEQ ID NO: 43, apreferably wherein the improved properties are selected from:
(i) increase in stability,
(ii) increase in storage stability, and
(iii) increase in storage stability in a detergent composition.

9. A polynucleotide encoding the variant polypeptide of any one of the preceding claims.

10. A composition comprising the polypeptide of any one of claims 1 to 8 and at least one additional component.

11. The composition of claim 10, wherein the composition comprises an enzyme stabilizing system.

12. The composition of claim 11, wherein the enzyme stabilizing system comprises at least one compound selected from the group consisting of polyols (preferably, 1,3-propanediol, ethylene glycol, glycerol, 1,2-propanediol, or sorbitol), inorganic salts (preferably, CaCl2, MgCl2, or NaCl), short chain (preferably, C1-C3) carboxylic acids or salts thereof (preferably, formic acid, formate (preferably, sodium formate), acetic acid, acetate, or lactate), borate, boric acid, boronic acids (preferably, 4-formyl phenylboronic acid (4-FPBA)), peptide aldehydes (preferably, Z-VAL-H or Z-GAY-H), peptide acetals, and peptide aldehyde hydrosulfite adducts, preferably peptide aldehydes (preferably, Z-VAL-H or Z-GAY-H).

13. The composition of any one of claims 10 to 12, wherein the composition comprises one or more additional enzymes different from the variant polypeptide referred to in any of the preceding claims, preferably one or more additional enzymes selected from the group consisting of amylases, additional proteases, lipases, cellulases, hemicellulases, mannanases, xylanases, DNases, dispersins, pectinases, oxidoreductases, and cutinases, preferably selected from amylases, mannanases, cellulase, and lipases, most preferably amylases.

14. The composition of any one of claims 10 to 13, wherein the composition is a detergent composition, preferably a laundry detergent composition or a hard surface cleaning detergent composition, preferably a biodegradable detergent composition, preferably wherein the composition comprises one or more surfactants and/or one or more builder, preferably strong sequestering builder.

15. The composition of any one of claims 10 to 14, wherein the composition further comprises 2-phenoxyethanol and/or 4,4'-dichloro 2-hydroxydiphenylether, preferably comprising phenoxyethanol in an amount ranging from 2 ppm to 5% by weight of the composition; more preferably comprising 0.1 to 2% of phenoxyethanol by weight of the composition and/or preferably comprising 4,4'-dichloro 2-hydroxydiphenylether in a concentration from 0.001 to 3%, preferably 0.002 to 1%, more preferably 0.01 to 0.6%, each by weight of the composition.
